(19) Europäisches Patentamt
European Patent Office
Office européen des brevets

(11) **EP 3 858 350 A1**

(12) **EUROPEAN PATENT APPLICATION**

(43) Date of publication:
**04.08.2021 Bulletin 2021/31**

(21) Application number: **20213908.5**

(22) Date of filing: **30.10.2013**

(51) Int Cl.:
*A61K 31/4439* (2006.01) *A61K 31/12* (2006.01)
*A61K 31/20* (2006.01) *A61K 39/395* (2006.01)
*A61P 35/00* (2006.01) *C12Q 1/68* (2018.01)
*G01N 33/574* (2006.01) *A61K 31/28* (2006.01)
*A61K 31/496* (2006.01) *A61K 31/7105* (2006.01)
*A61K 31/713* (2006.01) *C07K 16/40* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2012 US 201261720218 P**
**27.08.2013 US 201361870418 P**
**27.08.2013 US 201361870435 P**

(62) Document number(s) of the earlier application(s) in accordance with Art. 76 EPC:
**13852049.9 / 2 914 261**

(71) Applicant: **Pacylex Pharmaceuticals Inc.**
**Calgary, AB T2P 4H2 (CA)**

(72) Inventors:
- **BERTHIAUME, Luc, G.**
  **Edmonton, Alberta T6E 4X2 (CA)**
- **PERINPANAYAGAM, Conganige, Maneka, Anne**
  **Calgary, Alberta T3H 1B7 (CA)**
- **YAP, Chuiyee**
  **Edmonton, Alberta T5Y 0C1 (CA)**
- **BEAUCHAMP, Erwan**
  **Edmonton, Alberta T6E 2S7 (CA)**

(74) Representative: **Dehns**
**St. Bride's House**
**10 Salisbury Square**
**London EC4Y 8JD (GB)**

Remarks:
•This application was filed on 14.12.2020 as a divisional application to the application mentioned under INID code 62.
•Claims filed after the date of receipt of the divisional application (Rule 68(4) EPC).

(54) **SYNTHETIC LETHALITY AND THE TREATMENT OF CANCER**

(57) The invention provides a product comprising the NMT1 inhibitor DDD86481 or DDD85646, and a chemotherapeutic treatment, for combined use in the treatment of a cancer deficient in NMT2 in a subject. In an embodiment, the product comprises the NMT1 inhibitor DDD86481 and doxorubicin, for combined use in the treatment of a cancer deficient in NMT2 in a subject, wherein the cancer is B-cell lymphoma.

Figure 5

**Effectiveness of NMT inhibitors on Burkitt's Lymphoma Cell Line Ramos in comparison to immotalized normal B lymphocytic cell line (IM9) after 48 Hours at different concentrations**

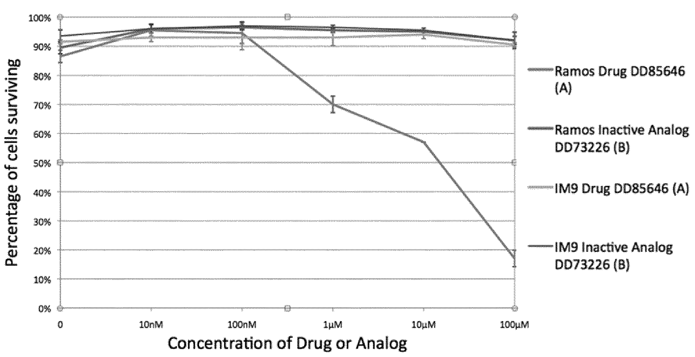


EP 3 858 350 A1

## Description

### CROSS RELATED APPLICATIONS

This application claim priority to US 61/720,218, filed October 30, 2012, US 61/870,418, filed August 27, 2013, US 61/870,435, filed August 27, 2013, the entire contents all of which are hereby incorporate by reference.

### FIELD OF THE INVENTION

The field of the invention generally relates to compounds, compositions and methods for treatment of cancer.

### BACKGROUND OF THE INVENTION

Cancer is a leading cause of death in Canada. The Canadian Cancer Society estimate there will be approximately 170000 new cases of cancer in 2011, and approximately 75000 deaths as a result of cancer.

An emerging approach for the treatment of cancer relates to the concept of synthetic lethality. Two genes (or two gene products) are synthetic lethal if mutation of either alone is compatible with viability but mutation of both leads to death. Put another way, "synthetic lethality" describe situations where a mutation and a drug (for example) together cause a cancer cell's death - either the mutation or the drug would not result in cell death. Targeting a gene (or gene product) that is synthetic lethal to a cancer-relevant mutation should kill only cancer cells and spare normal cells. Synthetic lethality therefore provides a framework for the development of anti-cancer specific agents.

The approach of synthetic lethality to the treatment of cancer is emerging, is not yet a routine approach largely due to the absence identification of synthetic lethal genes (and gene products).

*N*-myristoylation of proteins is a modification in which myristate (a 14-carbon saturated fatty acid) is covalently attached to the $NH_2$ terminal glycine of a variety of cellular, viral, and onco-proteins (e.g., oncogenic Src-related tyrosine kinases, heterotrimeric G alpha subunits, etc.).

Cellular myristoylated proteins have diverse biological functions in signal transduction and oncogenesis. Modification of proteins by myristoylation is required for the subcellular targeting, protein conformation and biological activity of many important proteins in eukaryotic cells, including those required for signal transduction and regulatory functions important in cell growth. Tyrosine kinases of the Src family (proto-oncogenes) are among the most extensively studied myristoylated proteins.

Myristoylation of proteins is catalyzed by *N*-myristoyltransferase (NMT). NMT is responsible for this activity in eukaryotic cells and works by modifying its polypeptide substrate after the removal of the initiator methionine residue by methionyl aminopeptidase. This modification occurs primarily as a cotranslational process, although myristoylation can also occur post-translationally after proteolytic cleavage of proteins, typically during apoptosis. Two isozymes of the mammalian NMT enzymes have been cloned and are designated NMT1 and NMT2. NMTs play a pro-survival role in cells. The two NMTs are present in all normal cells.

There remains a need for compounds, composition and method for the treatment of cancer.

This background information is provided for the purpose of making known information believed by the applicant to be of possible relevance to the present invention. No admission is necessarily intended, nor should it be construed, that any of the preceding information constitutes prior art against the present invention.

### SUMMARY OF THE INVENTION

In accordance with one aspect of the present invention there is provided compounds and compositions for the treatment of a subject with cancer. There are also provided methods for identifying subject with cancer that are suitable for treatment with the compounds, composition and methods are described herein.

In accordance with one aspect, there is provided a method of treating a subject having a cancer deficient in NMT2, comprising: administering to said subject an NMT inhibitor.

In a specific aspect, said NMT inhibitor comprises an NMT1 inhibitor.

In a specific aspect, said NMT1 inhibitor comprises a small molecule, an antibody, a peptide fragment, a nucleic acid, or combinations thereof.

In a specific aspect, said small molecule comprises Tris-DBA, HMA, or DDD85646, DDD86481, or a derivative thereof.

In a specific aspect,said antibody is a monoclonal antibody or a polyclonal antibody.

In a specific aspect,said nucleic acid comprises a dsRNA molecule, a RNAi molecule, a miRNA molecule, a ribozyme, a shRNA molecule, or a siRNA molecule.

In a specific aspect, said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

In a specific aspect, said subject is a human subject.

In accordance with another aspect, there is provided a method for treating a subject with a cancer or suspect of having a cancer, comprising: requesting a test providing the results of an analysis to determine whether a sample from the subject expresses NMT2, and administering an NMT1 inhibitor to the subject if the sample is deficient in NMT2.

In accordance with another aspect, there is provided a method, comprising: obtaining a sample from a subject with a cancer or suspected of having a cancer; processing said sample; performing a binding assay comprising contacting the processed sample with an antibody to NMT2 to form a complex between the antibody and NMT2 protein present in the processed sample, said binding assay generating at least one assay result indicative of said complex; wherein administering an NMT1 inhibitor to said subject is indicated when the amount of NMT2 protein said sample is low or absent, optionally as compared to a control.

In accordance with another aspect, there is provided a method, comprising: obtaining a sample from a subject having a cancer or suspect of having a cancer; processing said sample; performing a binding assay comprising contacting the processed sample with an antibody to NMT2 protein to form a complex between the antibody and NMT2 protein present in the processed sample, said binding assay generating at least one assay result indicative of said complex; and administering an NMT1 inhibitor to said subject when the amount to NMT2 protein in said sample is low or absent, optionally as compared to a control.

In a specific aspect, said analysis to determine whether said sample from the subject expresses NMT2, comprises performing a binding assay comprising contacting the processed sample with an antibody to NMT2 to form a complex between the antibody and NMT2 present in the processed sample, said binding assay generating at least one assay result indicative of said complex.

In a specific aspect, said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

In a specific aspect, instrumentation having a detector set to detect the complex formed between said antibody and said NMT2 in said sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

In a specific aspect, said subject is human.

In another aspect, there is provided a method, comprising: obtaining a sample from a subject having a cancer or suspected of having a cancer; processing said sample; performing a binding assay comprising contacting the processed sample with a detectable label which binds to NMT2 nucleic acid to form a complex between the detectable label and NMT2 nucleic acid present in the sample, said binding assay generating at least one assay result indicative of said complex; wherein administering an NMT1 inhibitor to said subject is indicated when the amount to NMT2 nucleic acid in said sample is low or absent, optionally as compared to a control.

In another aspect, there is provided a method, comprising: obtaining a sample from a subject having a cancer or suspected of having a cancer; processing said sample; performing a binding assay comprising contacting the processed sample with a detectable label which binds to NMT2 nucleic acid to form a complex between the detectable label and NMT2 nucleic acid present in the sample, said binding assay generating at least one assay result indicative of said

complex; and administering an NMT1 inhibitor to said subject when the amount to NMT2 nucleic acid in said sample is low or absent, optionally as compared to a control.

In a specific aspect, said analysis to determine whether said sample from the subject expresses NMT2, comprises performing a binding assay comprising contacting the processed sample with a detectable label which binds to NMT2 nucleic acid to form a complex between the detectable label and NMT2 nucleic acid present in the sample, said binding assay generating at least one assay result indicative of said complex.

In a specific aspect, said binding assay comprises, a hybridization assay using detectably labeled DNA or RNA probes.

In a specific aspect, said hybridization assay is quantitative or semi-quantitative.

In a specific aspect, said hybridization assay is RT-PCR, in situ hybridization, RNA protection assay ("RPA"), cDNA and oligonucleotide microarray, representation difference analysis ("RDA"), differential display, EST sequence analysis, serial analysis of gene expression ("SAGE"), and multiplex ligation-mediated amplification with the Luminex FlexMAP ("LMF").

In a specific aspect, instrumentation having a detector set to detect the complex between the detectable label and NMT2 nucleic acid present in the sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

In a specific aspect, said subject is a human.

In another aspect, there is provided a method, comprising: obtaining a sample from a subject having a cancer or suspected of having a cancer; processing said sample; performing a binding assay comprising contacting the processed sample with a an antibody to which binds to myristoylated protein, or azido-biotin labeled myristoylated proteins, within the sample to form a complex between the detectable label and myristoylated protein present in the sample, said binding assay generating at least one myristoylation profile indicative of said complex; wherein administering an NMT1 inhibitor to said subject is indicated when said myristoylation profile indicates said cancer is deficient in NMT2, optionally as compared to a control

In another aspect, there is provided a method, comprising: obtaining a sample from a subject having a cancer or suspected of having a cancer; processing said sample; performing a binding assay comprising contacting the processed sample with a an antibody to which binds to myristoylated protein, or azido-biotin labeled myristoylated proteins, within the sample to form a complex between the detectable label and myristoylated protein present in the sample, said binding assay generating at least one myristoylation profile indicative of said complex; and administering an NMT1 inhibitor to said subject is when said myristoylation profile indicates said cancer is deficient in NMT2, optionally as compared to a control

In a specific aspect, said processing comprises treating said sample with alyknyl-myristate and desthiobiotin azido-PEG biotin .

In a specific aspect, said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

In a specific aspect, instrumentation having a detector set to detect the complex formed between said antibody and said NMT2 in said sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell

Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

In a specific aspect, said subject is human.

In another aspect there is provided a use of an NMT inhibitor for treating a subject having a cancer deficient in NMT2.

In a specific aspect, said NMT inhibitor comprises an NMT1 inhibitor.

In a specific aspect, said NMT1 inhibitor comprises a small molecule, an antibody, a peptide fragment, a nucleic acid, or combinations thereof.

In a specific aspect, said small molecule comprises Tris-DBA, HMA, or DDD85646, DDD86481, or a derivative thereof.

In a specific aspect, said small molecule comprises DDD86481.

In a specific aspect, said antibody is a monoclonal antibody or a polyclonal antibody.

In a specific aspect, said nucleic acid comprises a dsRNA molecule, a RNAi molecule, a miRNA molecule, a ribozyme, a shRNA molecule, or a siRNA molecule.

In a specific aspect, said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

In a specific aspect, said subject is a human subject.

In another aspect, there is provided a use of an NMT1 inhibitor for treating a subject with a cancer, or suspected of having a cancer, wherein said an NMT1 inhibitor is indicated of use when the amount of NMT2 protein in a sample from said subject is low or absent, optionally as compared to a control, wherein a binding assay comprising contacting a processed sample from said subject with an antibody to NMT2 to form a complex between the antibody and NMT2 protein present in the processed sample generates at least one assay result indicative of said complex; wherein said assay result is indicative of said amount of NMT2 protein in said sample.

In a specific aspect, said analysis to determine whether said sample from the subject expresses NMT2, comprises performing a binding assay comprising contacting the processed sample with an antibody to NMT2 to form a complex between the antibody and NMT2 present in the processed sample, said binding assay generating at least one assay result indicative of said complex.

In a specific aspect, said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

In a specific aspect, instrumentation having a detector set to detect the complex formed between said antibody and said NMT2 in said sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

In a specific aspect, said subject is human.

In another aspect, there is provided a use of an NMT1 inhibitor for treating a subject with a cancer, or suspected of having a cancer, wherein said an NMT1 inhibitor is indicated of use when the amount of NMT2 nucleic acid in a sample

from said subject is low or absent, optionally as compared to a control, wherein a binding assay comprising contacting a processed sample from said subject with a detectable label which binds to NMT2 nucleic acid to form a complex between the detectable label and NMT2 nucleic acid in said sample, said binding assay generating at least one assay result indicative of said complex.

In a specific aspect, said analysis to determine whether said sample from the subject expresses NMT2, comprises performing a binding assay comprising contacting the processed sample with a detectable label which binds to NMT2 nucleic acid to form a complex between the detectable label and NMT2 nucleic acid present in the sample, said binding assay generating at least one assay result indicative of said complex.

In a specific aspect, said binding assay comprises, a hybridization assay using detectably labeled DNA or RNA probes.

In a specific aspect, said hybridization assay is quantitative or semi-quantitative.

In a specific aspect, said hybridization assay is RT-PCR, in situ hybridization, RNA protection assay ("RPA"), cDNA and oligonucleotide microarray, representation difference analysis ("RDA"), differential display, EST sequence analysis, serial analysis of gene expression ("SAGE"), and multiplex ligation-mediated amplification with the Luminex FlexMAP ("LMF").

In a specific aspect, instrumentation having a detector set to detect the complex between the detectable label and NMT2 nucleic acid present in the sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

In a specific aspect, said subject is a human.

In one aspect there is provided a use of an NMT1 inhibitor for treating a subject with a cancer, or suspected of having a cancer, wherein said use of said NMT1 inhibitor is indicated when a myristoylation profile from a sample from said subject indicates said cancer is deficient in NMT2, optionally as compared to a control, wherein performing a binding assay comprising contacting a processed sample with an antibody which binds to myristoylated protein, or azido-biotin labeled myristoylated proteins, within the sample to form a complex between the detectable label and myristoylated protein present in the sample, said binding assay generating at least one myristoylation profile indicative of said complex

In a specific aspect, said processing comprises treating said sample with alyknyl-myristate and desthiobiotin azido-PEG biotin .

In a specific aspect, said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

In a specific aspect, instrumentation having a detector set to detect the complex formed between said antibody and said NMT2 in said sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

In a specific aspect, said subject is human.

In another aspect there is provided a method for identifying a subject suitable for treatment with an NMT1

inhibitor, comprising: obtaining a sample from said subject with a cancer or suspected of having a cancer; processing said sample; performing a binding assay comprising contacting the processed sample with an antibody to NMT2 to form a complex between the antibody and NMT2 protein present in the processed sample, said binding assay generating at least one assay result indicative of said complex; wherein treatment with said NMT1 inhibitor is indicated when the amount of NMT2 protein said sample is low or absent, optionally as compared to a control.

In a specific aspect, said analysis to determine whether said sample from the subject expresses NMT2, comprises performing a binding assay comprising contacting the processed sample with an antibody to NMT2 to form a complex between the antibody and NMT2 present in the processed sample, said binding assay generating at least one assay result indicative of said complex.

In a specific aspect, said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

In a specific aspect, instrumentation having a detector set to detect the complex formed between said antibody and said NMT2 in said sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

In a specific aspect, said subject is human.

In another aspect there is provided, a method for identifying a subject suitable for treatment with an NMT1 inhibitor, comprising: obtaining a sample from a subject having a cancer or suspected of having a cancer; processing said sample; performing a binding assay comprising contacting the processed sample with a detectable label which binds to NMT2 nucleic acid to form a complex between the detectable label and NMT2 nucleic acid present in the sample, said binding assay generating at least one assay result indicative of said complex; wherein administering an NMT1 inhibitor to said subject is indicated when the amount to NMT2 nucleic acid in said sample is low or absent, optionally as compared to a control.

In a specific aspect, said analysis to determine whether said sample from the subject expresses NMT2, comprises performing a binding assay comprising contacting the processed sample with a detectable label which binds to NMT2 nucleic acid to form a complex between the detectable label and NMT2 nucleic acid present in the sample, said binding assay generating at least one assay result indicative of said complex.

In a specific aspect, said binding assay comprises, a hybridization assay using detectably labeled DNA or RNA probes.

In a specific aspect, said hybridization assay is quantitative or semi-quantitative.

In a specific aspect, said hybridization assay is RT-PCR, in situ hybridization, RNA protection assay ("RPA"), cDNA and oligonucleotide microarray, representation difference analysis ("RDA"), differential display, EST sequence analysis, serial analysis of gene expression ("SAGE"), and multiplex ligation-mediated amplification with the Luminex FlexMAP ("LMF").

In a specific aspect, wherein instrumentation having a detector set to detect the complex between the detectable label and NMT2 nucleic acid present in the sample is used to determine an amount of complex in said sample.

In a specific aspect, wherein said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma,

ovarian carcinoma, non-small cell lung carcinoma, osteosarcoma, melanoma, gastric adenocarcinoma, endometrial adenocarcinoma, esophageal squamous carcinoma.

In a specific aspect, said subject is a human.

In another aspect there is provided a method for identifying a subject suitable for treatment with an NMT1 inhibitor, comprising: obtaining a sample from a subject having a cancer or suspected of having a cancer; processing said sample; performing a binding assay comprising contacting the processed sample with a an antibody to which binds to myristoylated protein, or azido-biotin labeled myristoylated proteins, within the sample to form a complex between the detectable label and myristoylated protein present in the sample, said binding assay generating at least one myristoylation profile indicative of said complex; wherein administering an NMT1 inhibitor to said subject is indicated when said myristoylation profile indicates said cancer is deficient in NMT2, optionally as compared to a control

In a specific aspect, said processing comprises treating said sample with alyknyl-myristate and desthiobiotin azido-PEG biotin.

In a specific aspect, said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

In a specific aspect, instrumentation having a detector set to detect the complex formed between said antibody and said NMT2 in said sample is used to determine an amount of complex in said sample.

In a specific aspect, said instrumentation is a spectrophotometer, spectrofluorometer, optical device, or electrochemical device.

In a specific aspect, said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

In a specific aspect, wherein said subject is human.

In another aspect there is provided a kit for identifying a subject suitable for treatment with an NMT1 inhibitor, comprising: an antibody to NMT2; instructions for identifying the subject according to the method of any one of claims 68-74.

In a specific aspect the kit further comprising a control.

In another aspect there is provided a kit for identifying a subject suitable for treatment with an NMT1 inhibitor, comprising: a nucleic acid for binding to NMT2; instructions for identifying the subject according to the method of any one of claims 75-83.

In a specific aspect, the kit further comprising a control.

The kit of claim 93, wherein said nucleic acid is RNA or DNA.

In another aspect there is provided a kit for identifying a subject suitable for treatment with an NMT1 inhibitor, comprising: NeutrAvidinTM-HRP; and instructions for identifying said subject according to any one of claims 84-90.

In a specific aspect the kit further comprising a control.

In a specific aspect the kit further comprising alyknyl-myristate or desthiobiotin azido-PEG biotin.

## BRIEF DESCRIPTION OF THE DRAWINGS

Embodiments of the present invention will now be described, by way of example only, with reference to the attached Figures, wherein:

Figure 1 depicts immunoblot analysis of NMT1 and NMT2 expression in one type of normal B cells (L0) and various B cell lymphomas and T cell leukemias;

Figure 2 is a graph illustrating sensitivity of various normal cells and various B cell lymphomas and T cell leukemias to the NMT inhibitors tris-dibenzylideneacetone-dipalladium (Tris-DBA);

Figure 3 is a bar graph illustrating inhibition of N-myristoyltransferase (NMT) by tris-dibenzylideneacetone-dipalladium (Tris-DBA); and

Figure 4 are immunoblotts depicting lymphoma cell lines probed with antibodies against NMT1 and NMT2.

Figure 5 is a line graph showing the sensitivity of NMT inhibitors on a Burkitt's Lymphoma cell line in comparison

to an immortalized normal B lymphocytic cell line;

Figure 6 depicts the results of transfection of Ramos B lymphoma cells with pcDNA3.1-V5-NMT2 showing increased survival to TrisDBA (5 ug/ml) 2.5 fold vs control cells transfected with empty plasmid vector (Panel A) showing cell viability, and (Panel B) an immunoblott;

Figure 7 depicts differences in the NMT2 protein levels present in various lymphocytic cell lines and solid lymphoma tumors;

Figure 8 A and B depicts differences in the NMT2 protein levels present in various solid lymphoma tumors analyzed by immuno-histochemistry: NMT Immunohistochemical staining of normal lymph nodes, Burkitt's lymphoma (BL) and diffuse large B cell lymphoma (DLBCL), in Panel C the log2(micro-array fluorescence intensity NMT) for NMT1 and NMT2 is plotted for all the cell lines of the CCLE database, in Panel D the log2(micro-array fluorescence intensity NMT) for NMT1 and NMT2 is plotted for the 100 cell lines of the CCLE database with the lowest NMT2 expression level, (panel E) the expression of NMT2 in Burkitt's, Diffuse Large B cell and follicular lymphoma was analyzed by immune-histochemistry and the peroxidase staining was quantified using Image J. Normal lymph node content in NMT2 is 0.392 +/- 0.3 (relative unit, data not shown).;

Figure 9 depicts residual viability of various B lymphocytic cell lines treated with DDD85646 (panel A), DDD86481 (Panel B), and DDD73226 (Panel C) for 72 hours, Panel D depicts confirmation of the inhibition of myristoylation by DDD86481 in IM9 (i) and BL2 (ii) lymphocytes, Panel E depicts the minimal dose of DDD86481 required to inhibit myristoylation in IM-9, BL2 and Ramos cell lines;

Figure 10 (Panel A) depicts sensitivity of various immortalized normal L0 B lymphocytes, malignant B lymphoma cells (Ramos and BL2), and, T cell leukemia (CEM) to the NMT inhibitor TrisDBA for 24 hours, (Panel B) depicts transfection of Ramos B lymphoma cells with pcDNA3.1-V5-NMT2 increased survival to TrisDBA (5 ug/ml) 2.5 fold vs control cells transfected with empty plasmid vector.

Figure 11 depicts NMT expression levels in the 967 cancer cell lines encyclopedia (CCLE) database (panel A), NMT2 expression in select cancer using a box and whisker plot (panel B), the 50 CCLE cell lines with the lowest NMT2 expression are listed and sorted by cancer type;

Figure 12 depicts that NMTs are cleaved during apoptosis but remain active;

Figure 13 depicts purification of recombinant GST- and His6-tagged hNMT1 and hNMT2;

Figure 14 depicts comparison of enzyme activities between purified recombinant full-length His6-NMT1 and recombinant "caspase-truncated" ct-His6-NMT1;

Figure 15 depicts comparison of the EC50 and IC50 of various NMT inhibitors and different cell lines;

Figure 16 depicts DDD86481 induction of apoptosis;

Figure 17 depicts (Panel A) an immunoblot with the indicated lymphoid cell lines probed for the presence of NMT2, and (Panel B) the expression level of NMT2 mRNA shown as $log_2$(micro-array fluorescence intensity NMT2) for the indicated cell lines of the CCLE data;

Figure 18 depicts the use of a desthiobiotin-PEG-azide probe to pull-down post-translationally ω-alkynyl-myristoylated proteins in leukemic Jurkat T cells using streptavidin-sepharose beads;

Figure 19 depicts depicts scaled-up use of a desthiobiotin-PEG-azide probe to pull-down post-translationally ω-alkynyl-myristoylated proteins in leukemic Jurkat T cells using streptavidin-magnetic beads ;

Figure 20 depicts myristoylation profiles of "normal" immortalized B cells (IM9) and BL cells (BL2 and Ramos) labeled with alkynyl-myristate;

Figure 21 depict time and dose dependent cytotoxicity graphs from the combination of DDD86481 and doxorubixin;

Figure 22 depicts immunoblotting conducted with cells incubated with DMSO, Staurosporine, α FAS, or carrier alone;

Figure 23 depicts that that caspase truncated NMT2 is 3-4 times more active than full length NMT2;

Figure 24 depicts immunoblotts of "Normal" B cells (IM9) and malignant BL cells (Ramos, BL2) treated with 1 μM SAHA (HDAC class I/II inhibitor) for 24 h;

Figure 25 depicts that NMT2 protein levels are reduced in various BL cell lines;

Figure 26 depicts that proteosomal degradation is not the cause of NMT2 depletion in BL cells;Figure 27 depicts NMT1 is cleaved by caspase-8, but not NMT2;

Figure 28 depicts both NMT1 and NMT2 were cleaved by caspase-3;

Figure 29 depicts caspase cleavage sites of NMT1 and NMT2 as identified by Edman degradation shown in bold font and the positively charged lysine (K) box is highlighted on the NMT1 and NMT2 amino acid sequences (amino acids 1 to 80);

Figure 30 depicts confirmation of NMT cleavage sites by site-directed mutagenesis;

Figure 31 depicts caspase cleavage sites of NMT1 and NMT2 as identified by Edman degradation shown in bold font and the positively charged lysine (K) box is highlighted on the NMT1 and NMT2 amino acid sequences (amino acids 1 to 80);

Figure 32 depicts changes to NMT levels as cells undergo apoptosis;

Figure 33 depicts initial NMT activity in the lysates of transiently transfected COS7 cells;

Figure 34 depicts initial NMT activity in the lysates of transiently transfected COS7 cells.
Figure 35 depicts NMT activity in COS7 cells transiently expressing V5- NMT1 and V5-NMT2 incubated with staurosporine (2.5 μM) and cycloheximide (5 μg/mL);
Figure 36 depicts purification of recombinant hexahistidine(His)-tagged full-length and caspase-cleaved hNMT1;
Figure 37 depicts Purification of recombinant hexahistidine(His)-tagged full-length and caspase-cleaved hNMT2;
Figure 38 depicts NMT activity of purified full length and caspase-cleaved hexahistidine(His)-NMTs assayed using a peptide myristoylation assay;
Figure 39 depicts subcellular fractionation of endogenous NMTs in HeLa cells during apoptosis;
Figure 40 depicts quantification of amount of NMT in different fractions after the subcellular fractionation of endogenous NMTs in HeLa cells during apoptosis; and
Figure 41 depicts sub-cellular fractionation of HeLa cells undergoing apoptosis labelled with alkynyl-myristate; and
Figure 42 depicts effect of 2-hydroxymyristic acid (HMA) on the induction of apoptosis. Jurkat T cells were treated with or without HMA (1 mM) and apoptosis was induced with anti-Fas (150 ng/ml) and cycloheximide (5 μg/ml).

In the Detailed Description that follows, the numbers in bold face type serve to identify the component parts that are described and referred to in relation to the drawings depicting various embodiments of the invention. It should be noted that in describing various embodiments of the present invention, the same reference numerals have been used to identify the same of similar elements. Moreover, for the sake of simplicity, parts have been omitted from some figures of the drawings.

## DETAILED DESCRIPTION

As will be described in more detail below, there is described herein compounds, composition and methods for the treatment of a subject with cancer. There are also described here methods for identifying subject with cancer that are suitable for treatment with the compounds, composition and methods are described herein. There are also described here methods for identifying subject with cancer.

The present application provides methods and compositions for the treatment of NMT deficient cancers in a subject. NMT-deficient cancers include cancers deficient in NMT2 or NMT1. In a specific example, the NMT deficient cancer is a NMT2 deficient cancer.

The term "cancer", as used herein, refers to a variety of conditions caused by the abnormal, uncontrolled growth of cells. Cells capable of causing cancer, referred to as "cancer cells", possess characteristic properties such as uncontrolled proliferation, immortality, metastatic potential, rapid growth and proliferation rate, and/or certain typical morphological features. Cancer cells may be in the form of a tumour, but such cells may also exist alone within a subject, or may be a non-tumorigenic cancer cell. A cancer can be detected in any of a number of ways, including, but not limited to, detecting the presence of a tumor or tumors (e.g., by clinical or radiological means), examining cells within a tumor or from another biological sample (e.g., from a tissue biopsy), measuring blood markers indicative of cancer, and detecting a genotype indicative of a cancer. However, a negative result in one or more of the above detection methods does not necessarily indicate the absence of cancer, e.g., a patient who has exhibited a complete response to a cancer treatment may still have a cancer, as evidenced by a subsequent relapse.

In a specific example of the present disclosure, the cancer is lymphoma.

The term "lymphoma" as used herein refers to a malignant growth of B or T cells in the lymphatic system. "Lymphoma" includes numerous types of malignant growths, including Hodgkin's Lymphoma and non-Hodgkin's lymphoma. The term "non-Hodgkin's Lymphoma" as used herein, refers to a malignant growth of B or T cells in the lymphatic system that is not a Hodgkin's Lymphoma (which is characterized, e.g., by the presence of Reed-Sternberg cells in the cancerous area). Non-Hodgkin's lymphomas encompass over 29 types of lymphoma, the distinctions between which are based on the type of cancer cells.

In a more specific example of the present disclosure, the cancer is a B-lymphoma.

Thus, in one embodiment, the compounds, compositions and methods of the disclosure are suitable for the treatment of a subject with B cell lymphoma.

Examples of B-cell lymphomas include, but are not limited to, for example, follicular lymphoma, diffuse large B-cell lymphoma, mantle cell lymphoma, B-CLL/SLL, immunocytoma/Waldenstrom's, and MALT-type/monocytoid B cell lymphoma. Also contemplated are the treatment of pediatric lymphomas such as Burkitt's lymphoma, diffuse large B-cell lymphoma, follicular lymphoma, precursor B-LBL, precursor T-LBL, and anaplastic large cell lymphoma.

In other embodiment, the cancer is is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal

Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, or esophageal squamous carcinoma.

The term "subject", as used herein, refers to an animal, and can include, for example, domesticated animals, such as cats, dogs, etc., livestock (e.g., cattle, horses, pigs, sheep, goats, etc.), laboratory animals (e.g., mouse, rabbit, rat, guinea pig, etc.), mammals, non-human mammals, primates, non-human primates, rodents, birds, reptiles, amphibians, fish, and any other animal. In a specific example, the subject is a human.

The term "treatment" or "treat" as used herein, refers to obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, diminishment of the reoccurrence of disease, and remission (whether partial or total), whether detectable or undetectable. "Treating" and "Treatment" can also mean prolonging survival as compared to expected survival if not receiving treatment. "Treating" and "treatment" as used herein also include prophylactic treatment. For example, a subject with early cancer, for example an early stage lymphoma, can be treated to prevent progression or alternatively a subject in remission can be treated with a compound or composition described herein to prevent recurrence.

It is shown herein that B cell lymphoma cells express NMT1, but not NMT2. This is in contrast to the leukemic and other cells tested which express both NMT1 and NMT2. (As shown in Figures 1 and 4)

It is further shown herein that B lymphoma cells are sensitive to inhibition of cell viability by NMT inhibitors.

In one example, the NMT inhibitor is tris-dibenzylideneacetone-dipalladium (Tris-DBA) (Figure 2)

In other examples, the NMT inhibitor 2-hydroxymyristae (HMA) is used to inhibit B lymphoma cells.

In yet another example, the pyrazole sulphonamide inhibitor of *T. brucie* NMT [J.A.Frearson et al (2010) Nature. 464.728-723)] (DDD85646) is used to inhibit B lymphoma cells. (Figure 5).

In another example, the inhibitor is DDD86481.

In a specific example, treatment of a subject with B lymphoma comprises administering said subject with an NMT inhibitor.

NMT inhibitor compounds or derivatives may be used in the present invention for the treatment of NMT2 deficient cancer.

There term "deficient" as used herein refers broadly to inhibition, reduction or elimination of (as compared to wild type or control samples), for example, NMT synthesis, levels, activity, or function, as well as inhibition of the induction or stimulation of synthesis, levels, activity, or function of the protein of NMT (for example NMT 1 or NMT2). The term also refers to any metabolic or regulatory pathway, which can regulate the synthesis, levels, activity, or function of NMT. The term includes also includes inhibition, reduction or elimination resulting form binding with other molecules and complex formation. Therefore, the term "NMT deficient" refers to that which results in the inhibition, reduction, or elimination of protein function or protein pathway function. However, the term does not imply that each and every one of these functions must be inhibited at the same time.

In some examples, a cancer may be identified as being deficient in NMT by determining the presence of a mutation in a NMT gene. Such methods of nucleic acid detection and amplification are well known to the skilled worker.

For example the nucleic acid to be amplified may be from a biological sample. Various methods (such as phenol and chloroform extraction) of extraction are suitable for isolating the DNA or RNA. Nucleic acid extracted from a sample can be amplified using nucleic acid amplification techniques well known in the art. Non limiting examples include chain reaction (PCR), reverse transcriptase polymerase chain reaction (RT-PCR), nested PCR, ligase chain reaction, amplifiable RNA reporters, Q-beta replication, transcription-based amplification, boomerang DNA amplification, strand displacement activation, cycling probe technology, isothermal nucleic acid sequence based amplification (NASBA), or other sequence replication assays or signal amplification assays may also be used.

Methods of amplification are well known in the art. Some methods employ reverse transcription of RNA to cDNA.

In one example, PCR is used to amplify a target sequence of interest, e.g., a NMT2 sequence.

Nucleic acids may be amplified prior to detection or may be detected directly during an amplification step, e.g., "real-time" methods. In some embodiments, the target sequence is amplified using a labeled primer such that the resulting amplicon is detectably labeled. In some embodiments, the primer is fluorescently labeled. In some embodiments, the target sequence is amplified and the resulting amplicon is detected by electrophoresis.

The level of gene expression can be determined by assessing the amount of NMT2 mRNA in a sample. Methods of measuring mRNA in samples are known in the art. To measure mRNA levels, the cells in the samples can be lysed and the levels of mRNA in the lysates or in RNA purified or semi-purified from lysates can be measured by any variety of methods familiar to those in the art. Such methods include, without limitation, hybridization assays using detectably labeled DNA or RNA probes, e.g., northern blotting, or quantitative or semi-quantitative RT-PCR methodologies using

appropriate oligonucleotide primers. Alternatively, quantitative or semi-quantitative in situ hybridization assays can be carried out using, for example, tissue sections, or unlysed cell suspensions, and detectably labeled, e.g., fluorescent, or enzyme-labeled, DNA or RNA probes. Additional methods for quantifying mRNA include RNA protection assay ("RPA"), cDNA and oligonucleotide microarrays, representation difference analysis ("RDA"), differential display, EST sequence analysis, serial analysis of gene expression ("SAGE"), and multiplex ligation-mediated amplification with the Luminex FlexMAP ("LMF").

Amplification can also be monitored using "real-time" methods. Real time PCR allows for the detection and quantitation of a nucleic acid target. Typically, this approach to quantitative PCR utilizes a fluorescent dye, which may be a double-strand specific dye, such as SYBR Green.RTM. I. Alternatively, other fluorescent dyes, e.g., FAM or HEX, may be conjugated to an oligonucleotide probe or a primer. Various instruments capable of performing real time PCR are known in the art. The fluorescent signal generated at each cycle of PCR is proportional to the amount of PCR product. A plot of fluorescence versus cycle number is used to describe the kinetics of amplification and a fluorescence threshold level is used to define a fractional cycle number related to initial template concentration. When amplification is performed and detected on an instrument capable of reading fluorescence during thermal cycling, the intended PCR product from non-specific PCR products can be differentiated using melting analysis. By measuring the change in fluorescence while gradually increasing the temperature of the reaction subsequent to amplification and signal generation it may be possible to determine the ($\Delta$ct) of the intended product(s) as well as that of the nonspecific product.

The methods may include amplifying multiple nucleic acids in sample, also known as "multiplex detection" or "multiplexing." As used herein, the term "multiplex PCR" refers to PCR, which involves adding more than one set of PCR primers to the reaction in order to detect and quantify multiple nucleic acids, including nucleic acids from one or more target gene markers. Furthermore, multiplexing with an internal control, e.g., 18s rRNA, GADPH, or .beta.-actin) provides a control for the PCR without reaction.

In some examples, a cancer may be identified as being deficient in NMT by determining epigenetic inactivation a NMT gene.

In some examples, a cancer may be identified as being deficient in NMT by determining the activity of NMT (including NMT1 or NMT2) in a sample of cells from a subject. Activity may be determined relative to a control, for example in the case of defects in cancer cells, relative to non-cancerous cells, preferably from the same tissue. Thus, a cancer deficient in NMT may have reduced or eliminated NMT activity and/or expression. The activity of NMT may be determined by using techniques well known in the art, and/or as described herein. In these examples, a cancer deficient in NMT has a reduced or eliminated activity.

In some examples, a cancer may be identified as NMT (e.g., NMT1, NMT2, or both) deficient by determining the amount, concentration and/or levels of NMT protein(s).

In some examples, a cancer may be identified as NMT deficient by determining the amount of myristoylated proteins in a biological sample from a subject with cancer, or suspected of having cancer. In this example, the presence, absence or amount of myristoylated protein can be determined, for example, using click chemistry using appropriate fatty acid analogs. Non-limiting methods are described herein. Alternate methods of determining the presence, absence, or amount of myristoylated proteins will be known to the skilled worker. A sample which has a reduced amount myristoylated protein in a sample (optionally as compared to a control) is indicative of an NMT deficient sample, or NMT deficient cancer. In some examples, a sample which has a reduced amount of myristoylated protein in a sample is indicative of an NMT2 deficient sample, or an NMT2 deficient cancer.

In some examples, a cancer may be identified as NMT deficient by determining the amount of the amount of acylation of proteins in a biological sample from a subject with cancer, or suspect of having cancer. In this example, the presence, absence or amount of acylation of proteins can be determined. Such methods would be known to the skilled worker. A sample which has a reduced amount of acylation of proteins in a sample (optionally as compared to a control) is indicative of an NMT deficient sample, or NMT deficient cancer. In some examples, a sample which has a reduced amount a of acylation of proteins in a sample is indicative of an NMT2 deficient sample, or an NMT2 deficient cancer.

In some examples, a cancer may be identified as a NMT deficient by determining the presence of one or more sequence variations such as mutations and polymorphisms may include a deletion, insertion or substitution of one or more nucleotides, relative to the wild-type nucleotide sequence. The one or more variations may be in a coding or non-coding region of the nucleic acid sequence and, may reduce or abolish the expression or function of NMT. Thus, the variant nucleic acid may encode a variant polypeptide which has reduced or abolished activity or may encode a wild-type polypeptide which has little or no expression within the cell, for example through the altered activity of a regulatory element.

In some example, a cancer may be identified as NMT deficient by determining the gene(s) that effect or negatively regulate the expression of NMT.

A variety of methods may be used for determining the presence or absence of a particular nucleic acid sequence in a sample obtained from a subject.

In some examples, a cancer may be identified as NMT-deficient by assessing the level of expression or activity

of a positive or negative regulator of NMT of a component of the NMT pathway. Expression levels may be determined, for example, by immunoassays, such as immoblotts and ELISA, and nucleic acid detection methods, such as RT-PCR, nanostring technology, RNA-seq, nucleic acid hybridisation or karyotypic analysis.

In some examples, a cancer may be identified as being deficient in NMT1 and/or NMT2 by determining the presence in a cell sample from the individual of one or more variations, for example, polymorphisms or mutations in NMT1 and/or NMT2.

Mutations and polymorphisms associated with cancer may also be detected at the protein level by detecting the presence of a variant (i.e. a mutant or allelic variant) polypeptide.

In another example, there is provided a method a treating a subject with cancer, wherein said cancer comprises cancer cells which are deficient in NMT2, comprising administering to said subject an NMT inhibitor and/or an NMT1 inhibitor.

The term "inhibit" or "inhibitor" as used herein, refers to any method or technique which inhibits protein synthesis, levels, activity, or function, as well as methods of inhibiting the induction or stimulation of synthesis, levels, activity, or function of the protein of interest, for example NMT2. The term also refers to any metabolic or regulatory pathway, which can regulate the synthesis, levels, activity, or function of the protein of interest. The term includes binding with other molecules and complex formation. Therefore, the term "inhibitor" refers to any agent or compound, the application of which results in the inhibition of protein function or protein pathway function. However, the term does not imply that each and every one of these functions must be inhibited at the same time.

In another example, there is provided a method of treating a subject with cancer, wherein said cancer comprises cancer cells deficient in NMT1, comprising administering to said subject an NMT inhibitor and/or an NMT2 inhibitor.

In some examples, treatment methods comprise administering to a subject a therapeutically effective amount of a compound described herein and optionally consists of a single administration or application, or alternatively comprises a series of administrations or applications. In a specific example, said compound is a NMT inhibitor, an NMT1 inhibitor and/or an NMT2 inhibitor.

In a more specific example, the NMT inhibitor is Tris-DBA, HMA, DDD85646, DDD86481, or derivatives thereof.

In other examples, the compounds and/or compositions are provided in a pharmaceutically effect amount suitable for administration to a subject.

The term "pharmaceutically effective amount" as used herein refers to the amount of a drug or pharmaceutical agent that will elicit the biological or medical response of a tissue, system, animal or human that is being sought by a researcher or clinician. This amount can be a therapeutically effective amount.

The compounds and compositions are provided in a pharmaceutically acceptable form.

The term "pharmaceutically acceptable" as used herein includes compounds, materials, compositions, and/or dosage forms (such as unit dosages) which are suitable for use in contact with the tissues of a subject without excessive toxicity, irritation, allergic response, or other problem or complication, commensurate with a reasonable benefit/risk ratio. Each carrier, excipient, etc. is also be "acceptable" in the sense of being compatible with the other ingredients of the formulation.

The actual amount administered, and rate and time-course of administration, will depend on the nature and severity of what is being treated. Prescription of treatment, e.g. decisions on dosage etc., is within the responsibility of general practitioners and other medical doctors, and typically takes account of the disorder to be treated, the condition of the individual patient, the site of delivery, the method of administration and other factors known to practitioners.

A compound or composition may be administered alone or in combination with other treatments, either simultaneously or sequentially, dependent upon the condition to be treated.

The formulations may conveniently be presented in unit dosage form and may be prepared by any methods well known in the art of pharmacy. Such methods include the step of bringing the active compound into association with a carrier, which may constitute one or more accessory ingredients. In general, the formulations are prepared by uniformly and intimately bringing into association the active compound with liquid carriers or finely divided solid carriers or both, and then if necessary shaping the product.

The compounds and compositions may be administered to a subject by any convenient route of administration, whether systemically/peripherally or at the site of desired action, including but not limited to, oral (e.g. by ingestion); topical (including e.g. transdermal, intranasal, ocular, buccal, and sublingual); pulmonary (e.g. by inhalation or insufflation therapy using, e.g. an aerosol, e.g. through mouth or nose); rectal; vaginal; parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot / for example, subcutaneously or intramuscularly.

Formulations suitable for oral administration (e.g., by ingestion) may be presented as discrete units such as capsules, cachets or tablets, each containing a predetermined amount of the active compound; as a powder or granules; as a solution or suspension in an aqueous or non-aqueous liquid; or as an oil-in- water liquid emulsion or a water- in-oil liquid emulsion; as a bolus; as an electuary; or as a paste.

Formulations suitable for parenteral administration (e.g., by injection, including cutaneous, subcutaneous, intramuscular, intravenous and intradermal), include aqueous and non-aqueous isotonic, pyrogen-free, sterile injection solutions which may contain anti-oxidants, buffers, preservatives, stabilisers, bacteriostats, and solutes which render the formulation isotonic with the blood of the intended recipient; and aqueous and non- aqueous sterile suspensions which may include suspending agents and thickening agents, and liposomes or other microparticulate systems which are designed to target the compound to blood components or one or more organs. Examples of suitable isotonic vehicles for use in such formulations include Sodium Chloride Injection, Ringer's Solution, or Lactated Ringer's Injection.

The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets. Formulations may be in the form of liposomes or other microparticulate systems which are designed to target the active compound to blood components or one or more organs.

Compositions comprising compounds disclosed herein may be used in the methods described herein in combination with standard chemotherapeutic regimes or in conjunction with radiotherapy.

In the case of lymphoma in a patient, known treatments are dependent upon the subject being treated, the type of disease, and its stage. Existing treatment modalities for lymphoma are known to the skilled worker. Accordingly, known treatments may be used together with the NMT inhibitors disclosed herein.

Common drug combinations for use in treating lymphomas include, but are not limited, to CHOP (i.e., cyclophosphamide, doxorubicin, vincristine, and prednisone), GAP-BOP (i.e., cyclophosphamide, doxorubicin, procarbazine, bleomycin, vincristine, and prednisone), m-BACOD (i.e., methotrexate, bleomycin, doxorubicin, cyclophosphamide, vincristine, dexamethasone, and leucovorin), ProMACE-MOPP (i.e., prednisone, methotrexate, doxorubicin, cyclophosphamide, etoposide, leucovorin with standard MOPP), ProMACE-CytaBOM (prednisone, doxorubicin, cyclophosphamide, etoposide, cytarabine, bleomycin, vincristine, methotrexate, and leucovorin), and MACOP-B (methotrexate, doxorubicin, cyclophosphamide, vincristine, prednisone, bleomycin, and leucovorin). For relapsed aggressive non-Hodgkin's lymphoma the following chemotherapy drug combinations may be used with the compounds and compositions described herein: IMVP-16 (i.e., ifosfamide, methotrexate, and etoposide), MIME (i.e., methyl-gag, ifosfamide, methotrexate, and etoposide), DHAP (i.e., dexamethasone, - 16 high dose cytarabine, and cisplatin), ESHAP (i. e., etoposide, methylprednisone, high dosage cytarabine, and cisplatin), CEFF(B) (i.e., cyclophosphamide, etoposide, procarbazine, prednisone, and bleomycin), and CAMP (i.e., lomustine, mitoxantrone, cytarabine, and prednisone).

Treatment for salvage chemotherapy used for certain lymphomas such as for relapsed, resistant Hodgkin's Disease include but are not limited to VABCD (i.e., vinblastine, doxorubicin, dacarbazine, lomustine and bleomycin), ABDIC (i.e., doxorubicin, bleomycin, dacarbazine, lomustine, and prednisone), CBVD (i.e., lomustine, bleomycin, vinblastine, dexamethasone), PCVP (i.e., vinblastine, procarbazine, cyclophosphamide, and prednisone), CEP (i.e., lomustine, etoposide, and prednimustine), EVA (i.e., etoposide, vinblastine, and doxorubicin), MOPLACE (i.e., cyclophosphamide, etoposide, prednisone, methotrexate, cytaravine, and vincristine), MIME (i.e., methyl-gag, ifosfamide, methotrexate, and etoposide), MINE (i.e., mitoquazone, ifosfamide, vinorelbine, and etoposide), MTX-CHOP (i.e., methotrexate and CHOP), CEM (i.e., lomustine, etoposide, and methotrexate), CEVD (i.e., lomustine, etoposide, vindesine, and dexamethasone), CAVP (i.e., lomustine, melphalan, etoposide, and prednisone), EVAP (i.e., etoposide, vinblastine, cytarabine, and cisplatin), and EPOCH (i.e., etoposide, vincristine, ; doxorubicin, cyclophosphamide, and prednisone).

It will be appreciated that alternate methods to inhibit NMT1 or NMT2 may be used in a synthetic lethal strategy for the treatment of cancer, and in particular the treatment of B cell lymphoma. For example, expression of NMT1 or NMT2 may be inhibited using anti-sense or RNAi technology. The use of these approaches to down-regulate gene expression and/or protein activity is known to the skilled worker.

In another embodiment of the present disclosure there is provided a method for determining the benefit of NMT2-inhibitor and/or NMT1-inhibitor treatment of a patient.

In one example, a method of the present disclosure comprises qualitatively or quantitatively determining, analyzing or measuring a sample from a subject with cancer, or suspected of having cancer, for the presence or absence, or amount or concentration, of NMT1 and/or NMT2.

In another example, a method of the present disclosure comprises qualitatively or quantitatively determining, analyzing or measuring a sample from a subject with cancer, or suspected of having cancer, for the presence or absence, or amount or concentration, of myristolayted proteins.

In another example, a method of the present disclosure comprises qualitatively or quantitatively determining, analyzing or measuring a sample from a subject with cancer, or suspect of having cancer, for the presence or absence, or amount of concentration of acylated proteins.

The term "sample" as used herein refers to any sample from a subject, including but not limited to a fluid, cell or tissue sample that comprises cancer cells, or which is suspected of containing cancer cells, which can be assayed for gene expression levels, proteins levels, enzymatic activity levels, and the like. The sample may include, for example, a blood sample, a fractionated blood sample, a bone marrow sample, a biopsy, a frozen tissue sample, a fresh tissue

specimen, a cell sample, and/or a paraffin embedded section, material from which RNA can be extracted in sufficient quantities and with adequate quality to permit measurement of relative mRNA levels, or material from which polypeptides can be extracted in sufficient quantities and with adequate quality to permit measurement of relative polypeptide levels.

The determination, analysis or measurement of NMT1 or NMT2, or the presence or absence of NMT1 and/or NMT2 can be correlated with the benefit of NMT 1-inhibtor or NMT2-inhibitor treatment of cancer in the patient.

The determination, analysis or measurement of myristolyated proteins, or the presence or absence of myristolyated proteins can be correlated with the benefit of NMT1-inhibtor or NMT2-inhibitor treatment of cancer in the patient.

The determination, analysis or measurement of acylated proteins, or the presence or absence of myristolyated proteins can be correlated with the benefit of NMT 1-inhibtor or NMT2-inhibitor treatment of cancer in the patient.

In a specific example, antibodies of the present invention are immunoreactive or immunospecific for, and therefore specifically and selectively bind to a protein of interest, for example the protein NMT1 or NMT2. In one example, antibodies which are immunoreactive and immunospecific for human NMT1 or NMT2 can be used. Antibodies for human NMT1 or NMT2 are preferably immunospecific. The term "antibody" and "antibodies" includes, but is not limited to, monoclonal and polyclonal antibodies.

In another example, antibodies of the present invention are immunoreactive or immunospecific for, and therefore specifically and selectively bind to both NMT1 or NMT2 protein. In this example, antibodies which are immunoreactive and immunospecific for both human NMT1 or NMT2 can be used. Antibodies for human NMT1 or NMT2 are preferably immunospecific. In this example, and owing to the different molecular mass of NMT1 and NMT2, it is possible identify the presence or absence of both proteins using a single antibody, using, for example SDS-PAGE and immunoblotting. The term "antibody" and "antibodies" includes, but is not limited to, monoclonal and polyclonal antibodies.

The term "binds specifically" refers to high avidity and/or high affinity binding of an antibody to a specific polypeptide e.g., an epitope of NMT1 or NMT2. Antibody binding to its epitope on this specific polypeptide is stronger than binding of the same antibody to any other epitope, particularly those which may be present in molecules in association with, or in the same sample, as the specific polypeptide of interest. Antibodies which bind specifically to a polypeptide of interest may be capable of binding other polypeptides at weak, yet detectable, level. Such weak binding, or background binding, is readily discernable from the specific antibody binding to the compound or polypeptide of interest, e.g., by use of appropriate controls, as would be known to the worker skilled in the art.

In one example, a sample containing cancerous cells or suspected as containing cancerous cells is obtained from a subject with cancer. Collection of such a sample is well known to the skilled worker. In a specific example, the sample is a blood sample. Methods of obtaining a sample sample, processing and/or storage of such a sample are also well known to the skilled worker.

In a specific example, the detection, analysis or measurement of NMT1 or NMT2 protein within a sample is carried out using immunohistochemistry. In a more specific example, the detection, analysis, or measurement of NMT 2 within a sample is carried out using immunohistochemistry. It will be clear to the skilled worker that other immunoassays, both qualitative or quantitative, may be used in the present invention.

In additional examples, immunohistochemistry (IHC) may be accomplished using any suitable method or system of immunohistochemistry. Non limiting examples include automated systems, quantitative IHC, semi-quantitative IHC, and manual methods.

The term "quantitative" immunohistochemistry refers to an automated method of scanning and scoring samples that have undergone immunohistochemistry, to identify and quantitate the presence of a specified biomarker, such as an antigen or other protein. For example, to quantitate NMT1 and/or NMT2. The score given to the sample is a numerical representation of the intensity of the immunohistochemical staining of the sample, and represents the amount of target biomarker (such as NMT1 or NMT2) present in the sample. As used herein, Optical Density (OD) is a numerical score that represents intensity of staining as well as the percentage of cells that are stained. As used herein, semi-quantitative immunohistochemistry refers to scoring of immunohistochemical results by human eye, where a trained operator ranks results numerically (e.g., as 0 [weak or absent staining], 1 or 2 [strong staining]).

Automated sample processing, scanning and analysis systems suitable for use with immunohistochemistry are known in the art, and may be used with the present invention. Such systems may include automated staining and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples.

In practice, in the example in which a patient sample is determined to have low (e.g., weak or absent) NMT2 tumour staining, the patient is considered a good candidate for NMT1 inhibitor treatment. In another specific example, a patient determined to have high (e.g., strong) NMT2 tumour staining is considered a poor candidate for NMT1 inhibitor treatment.

It will be appreciated that the cut point for IHC negative vs positive determination is a semi-quantitative determination, and made by an experienced pathologist using semi-quantitiative methods and light microscopy.

For example, IHC can be scored in any of the following ways: 1. any staining vs no staining; 2. Strong vs weak staining; 3. None vs weak vs strong; 4. An H Score comprised of the formula (% none x 0) + (% weak x 100) ; + (% moderate x 200) + (% strong x 300); 5. Computerized image analysis software for assisted quantitative scoring.

The cut points are initially defined by the variable drug sensitivity *in vitro*. Once drugs hit clinical trials, the sensitive vs not sensitive cut point will be further refined and validated.

In one example, in determining whether there is high (e.g., strong) or low (e.g., weak or absent) NMT2 tumour staining, the patient sample may be compared to one or more control samples. In one example, a control sample has had known and/or established level of NMT2 tumour staining. In one example, a control sample is a patient sample that has known and/or established levels of NMT2 tumour staining and/or known clinical outcome. In one example, a control is a cell line that has a known amount of NMT2 staining.

Continued treatment options for patients who are considered to be a poor candidate for NMT1 inhibitor treatment are known to the skilled worker.

It will be appreciated that in some circumstances, a patient who initially responds to NTM1 inhibitor treatment may relapse. Such a relapse can manifest is several ways, including but not limited to, reoccurrence of the primary tumour and development of metastasis. In addition to, or alternatively, an additional distinct tumour can arise.

In accordance with one aspect of the present invention, there is provided a method comprising: a) obtaining a sample from a subject with, or suspected as having, cancer; b) contacting the sample with an antibody to NMT2 to form a complex between the antibody and NMT2 present in the sample; c) measuring the complex formed to determine an amount or a concentration of NMT2 in the sample; and d) determining the benefit of NMT1 inhibitor treatment of said cancer in in said subject, wherein the determination of benefit of NMT1 inhibitor treatment is determined by the level of NMT2 in said sample.

In a specific aspect, administering an NMT1 inhibitor to said subject is indicated when the amount to NMT2 in said sample is low or absent, optionally as compared to a control.

In accordance with one aspect of the present invention, there is provided a method, comprising: obtaining a sample from a subject; processing said sample; performing a binding assay comprising contacting the processed sample with an antibody to NMT2 to form a complex between the antibody and NMT2 present in the sample, said binding assay generating at least one assay result indicative of said complex; and administering an NMT1 inhibitor to said subject when the amount to NMT2 in said sample is low or absent, optionally as compared to a control.

In some aspects, instrumentation having a detector set to detect the complex formed between the antibody and NMT2 in said sample is used to determine the amount of complex in the sample. In some examples, the instrumentation is a spectrophotometer, spectrofluorometer, optical device, or electrochemical device. In some examples, the antibody to NMT2 is a monoclonal antibody, or a polyclonal antibody.

Other examples that may be used in the detection, analysis or measurement of NMT1 or NMT2 include, but are not limited to, immunoblotting, ELISA, indirect immuno-fluorescence, multiplexing bead technology, immunoprecipitation and mass spectrometry from sample obtain from the subject. In practice, in the example in which a patient sample is determined to have low or absent NMT2 staining, the subject is considered a good candidate for NMT-inhibitor therapy.

In one example the sample is analyzed by light mircorcopy by direct examination or by image capture and analysis, or by fluorescent microscopy using direct examination of by image capture and analysis.

In another example, a method of the present disclosure comprises qualitatively or quantitatively determining, analyzing or measuring the activity of NMT1 and/or NMT2 protein activity in biological sample from a subject with cancer patient for the presence or absence or amount of NMT1 and/or NMT2 activity. In this example, the uses of substrates (natural or synthetic) of NMT1 or NMT2 are used to identify a sample in which NMT1 or NMT2 activity is present, absent, or the amount thereof.

In practice, in the example in which a subject's sample is determined to be NMT2 deficient, he subject is considered a good candidate for administration on an NMT1 inhibitor.

In practice, in the example in which a subject's sample is determined to have low or absent NMT2 protein levels, the subject is considered a good candidate for administration of an NMT1 inhibitor.

In practice, in the example in which a subject's sample is determined to have low or absent NMT2 activity, the subject is considered a good candidate for administration on an NMT1 inhibitor.

In practice, in the example in which a subject's sample is determined to have low or absent amount of myristoylated protein, the subject is considered a good candidate for administration on an NMT1 inhibitor.

In practice, in the example in which a subject's sample is determined to have a low or absent amount of acylated protein, the subject is considered a good candidate for administration on an NMT2 inhibitor.

In another example, a method of the present disclosure comprises identifying a mutation, deletion, or the like, in the NMT1 or NMT2 gene in a sample from a subject with cancer or suspect of having cancer. Wherein, said mutation, deletion, or the like, in NMT1 or NMT2 gene results in a loss of diminishment of NMT1 or NMT2 protein activity in cancer cells within said sample. Methods of identifying such mutations, deletions, or the like, in NMT1 or NMT2 are known to

the skilled worker, and include, but are not limited to, RFLP, RT-PCT, microarray analysis, and/or any suitable type of DNA sequencing. In practice, in the example in which a patient sample is determined to have a mutation, deletion, or the like, in NMT2 which results in a low or absent NMT2 protein activity, the subject is considered a good candidate for NMT-inhibitor therapy.

In another example, a method of the present disclosure comprises identifying a mutation, deletion, or the like, in the NMT1 or NMT2 mRNA in a sample from a subject with cancer or suspect of having cancer. Wherein, said mutation, deletion, or the like, in NMT1 or NMT2 mRNA results in a loss of diminishment of NMT1 or NMT2 protein activity in cancer cells within said sample. Methods of identifying such mutations, deletions, or the like, in NMT1 or NMT2 mRNA are known to the skilled worker, and include, but are not limited to, Northern blotting, RT-PCR, microarray analysis, and/or any suitable type of mRNA sequencing. In practice, in the example in which a patient sample is determined to have a mutation, deletion, or the like, in NMT2 mRNA which results in a low or absent NMT2 protein activity, the subject is considered a good candidate for NMT-inhibitor therapy.

In another example, a method of the present disclosure, there is provided a method for the treatment of a subject suffering from cancer, associated with a defect in NMT1 or NMT2, comprising administering to said subject an inhibitor of NMT. In a specific example, the cancer is associated with a defect in NMT2, and the inhibitor is an NMT1 inhibitor.

In another example, there is provided a use of an NMT1 inhibitor for treatment a cancer deficient in NMT2.

In another example, there is provided a use of an NMT1 inhibitor for treatment of a cancer, wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, or oresophageal squamous carcinoma. In a more specific example, said cancer is determined as being a cancer deficient in NMT2.

Examples of inhibitors include, but are not limited to, small molecules, antibodies, peptide fragments, and/or nucleic acid molecules.

Specific examples of small molecules include Tris-DBA, HMA, DDD85646, DDD86481, and their derivatives. The term "derivatives" as used herein includes, but is not limited to, salts, coordination complexes, esters such as in vivo hydrolysable esters, free acids or bases, hydrates, prodrugs or lipids, coupling partners.

Peptide fragments may be prepared wholly or partly by chemical synthesis that active site of NMT1. Peptide fragments can be prepared according to established, standard liquid or solid-phase peptide synthesis methods, which will be known to the skilled worker.

Nucleic acid inhibitors, or the complements thereof, inhibit activity or function by down-regulating production of active polypeptide. This can be monitored using conventional methods well known in the art, for example by screening using real time PCR as described in the examples.

Examples of nucleic acid inhibitors include anti-sense or RNAi technology, the use of which is to down-regulate gene expression is well established in the art. Anti-sense oligonucleotides may be designed to hybridize to the complementary sequence of nucleic acid, pre-mRNA or mature mRNA, interfering with the production of the base excision repair pathway component so that its expression is reduced or completely or substantially completely prevented. In addition to targeting coding sequence, anti- sense techniques may be used to target control sequences of a gene, e.g. in the 5' flanking sequence, whereby the anti-sense oligonucleotides can interfere with expression control sequences.

An alternative to anti-sense is to use a copy of all or part of the target gene inserted in sense, that is the same, orientation as the target gene, to achieve reduction in expression of the target gene by co-suppression.

Additionally, double stranded RNA (dsRNA) silencing may be used. dsRNA mediated silencing is gene specific and is often termed RNA interference (RNAi).

In another example, nucleic acid is used which on transcription produces a ribozyme, able to cut nucleic acid at a specific site and therefore also useful in influencing NMT.

In yet another example, small RNA molecules may be employed to regulate gene expression. These include targeted degradation of mRNAs by small interfering RNAs (siRNAs), post transcriptional gene silencing (PTGs), developmentally regulated sequence-specific translational repression of mRNA by micro-RNAs (miRNAs) and targeted transcriptional gene silencing.

In yet another example, the expression of a short hairpin RNA molecule (shRNA) in the cell may be used. A shRNA consists of short inverted repeats separated by a small loop sequence. One inverted repeat is complimentary to the gene target. In the cell the shRNA is processed by DICER into a siRNA which degrades the target NMT gene

mRNA and suppresses expression. In a preferred embodiment the shRNA is produced endogenously (within a cell) by transcription from a vector.

A defect in NMT1 or NMT2, is a NMT1 or NMT2 deficient, respectively, phenotype which may be deficient in a component of a NMT1 or NMT2 mediated pathway i.e., expression of activity of a component of the pathway may be reduced or abolished in the cancer cell relative to control cells. In some embodiments, the cancer cell may be deficient in NMT1 or NMT2 i.e., expression of activity of NMT1 or NMT2 may be reduced or abolished in the cancer cell relative to control cells.

Accordingly, there is provided the use of NMT2 as a marker for one or more of diagnosis, prognosis, classifying, or monitoring of cancer in a subject. In some examples, NMT2 is measured using an assay selected from immunoassays or nucleic acid detection, or protein activity.

The term "prognosis" as used herein refers to the prediction of the likelihood of cancer-attributable death or progression, including recurrence, metastatic spread, and drug resistance, of a neoplastic disease, such as breast cancer.

The term "prognostic marker" as used herein refers to a marker that informs about the outcome of a patient in the absence of systemic therapy or portends an outcome different from that of the patients without the marker, despite empiric (not targeted to the marker) systemic therapy.

The term "predictive marker" as used herein refers to a marker that predicts that differential efficacy (benefit) of a particular therapy based on marker status.

The term "diagnosis" as used herein, refers to the identification of a molecular and/or pathological state, disease or condition, such as the identification of breast cancer, or other type of cancer.

There is also provided the use of protein myristoylation as a marker for one or more of diagnosis, prognosis, classifying or monitoring cancer in a subject.

There is also provided the use of protein acylation as a marker for one or more of diagnosis, prognosis, classifying or monitoring cancer in a subject.

In some examples, said cancer is lymphoma. In more specific examples, said lymphoma is B cell lymphoma. In more specific examples, said B cell lymphoma is follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, or anaplastic large cell lymphoma.

In some examples, the cancer is acute myeloid leukemia, B Cell lymphoma, Blast Phase Chronic Myeloid Leukaemia, Burkitt's Lymphoma, Diffuse Large B Cell Lymphoma, Plasma Cell Myeloma, Intestinal Adenocarcinoma, Lung mixed Adenosquamous Carcinoma, Lunch Small Cell Carcinoma, Lung, Oesophagus Squamous Cell Carcinoma, Bone, Breast Ductal Carcinoma, Stomach Diffuse Adenocarcinoma, Thyroid Medullary Carcinoma, Urinary Tract Transitional Cell Carcinoma.

In some examples, the cancer is B cell lymphoma, Burkitt's lymphoma, Diffuse Large Cell Lymphoma, Acute Myeloid Leukemia, Myeloma, Ovarian clear cell carcinoma, Transition cell carcinoma (ureter and bladder cancer), chronic myelogenous leukemia (CML), lymphoma-CLL, small cell lung carcinoma, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

Methods of the invention are conveniently practiced by providing the compounds and/or compositions used in such method in the form of a kit. Such a kit preferably contains the composition. Such a kit preferably contains instructions for the use thereof.

The invention is described further in the clauses below.

1. A method of treating a subject having a cancer deficient in NMT2, comprising: administering to said subject an NMT inhibitor.

2. The method of item 1, wherein said NMT inhibitor comprises an NMT1 inhibitor.

3. The method of item 2, wherein said NMT1 inhibitor comprises a small molecule, an antibody, a peptide fragment, a nucleic acid, or combinations thereof.

4. The method of item 3, wherein said small molecule comprises Tris-DBA, HMA, or DDD85646, DDD86481, or a derivative thereof.

5. The method of item 3, wherein said antibody is a monoclonal antibody or a polyclonal antibody.

6. The method of item 3, wherein said nucleic acid comprises a dsRNA molecule, a RNAi molecule, a miRNA molecule, a ribozyme, a shRNA molecule, or a siRNA molecule.

7. The method of any one of item 1 to 6, wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

8. The method of any one of items 1 to 7, wherein said subject is a human subj ect.

9. A method for treating a subject with a cancer or suspect of having a cancer, comprising: requesting a test providing the results of an analysis to determine whether a sample from the subject expresses NMT2, and administering an NMT1 inhibitor to the subject if the sample is deficient in NMT2.

10. A method, comprising: obtaining a sample from a subject with a cancer or suspected of having a cancer; processing said sample; performing a binding assay comprising contacting the processed sample with an antibody to NMT2 to form a complex between the antibody and NMT2 protein present in the processed sample, said binding assay generating at least one assay result indicative of said complex; wherein administering an NMT1 inhibitor to said subject is indicated when the amount of NMT2 protein said sample is low or absent, optionally as compared to a control.

11. A method, comprising: obtaining a sample from a subject having a cancer or suspect of having a cancer; processing said sample; performing a binding assay comprising contacting the processed sample with an antibody to NMT2 protein to form a complex between the antibody and NMT2 protein present in the processed sample, said binding assay generating at least one assay result indicative of said complex; and administering an NMT1 inhibitor to said subject when the amount to NMT2 protein in said sample is low or absent, optionally as compared to a control.

12. The method of item 9, wherein said analysis to determine whether said sample from the subject expresses NMT2, comprises performing a binding assay comprising contacting the processed sample with an antibody to NMT2 to form a complex between the antibody and NMT2 present in the processed sample, said binding assay generating at least one assay result indicative of said complex.

13. The method of any one of itemss item 11 - 13, wherein said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

14. The method of any one of item 9 - 13, wherein instrumentation having a detector set to detect the complex formed between said antibody and said NMT2 in said sample is used to determine an amount of complex in said sample.

15. The method of item 14, wherein said instrumentation is a spectrophotometer, spectrofluorometer, optical device, or electrochemical device.

16. The method of anyone of items 9 to 15, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, esophageal squamous carcinoma.

17. The method of any one of items 9 - 16, wherein said subject is human.

18. A method, comprising: obtaining a sample from a subject having a cancer or suspected of having a cancer; processing said sample; performing a binding assay comprising contacting the processed sample with a detectable label which binds to NMT2 nucleic acid to form a complex between the detectable label and NMT2 nucleic acid present in the sample, said binding assay generating at least one assay result indicative of said complex; wherein administering an NMT1 inhibitor to said subject is indicated when the amount to NMT2 nucleic acid in said sample is low or absent, optionally as compared to a control.

19. A method, comprising: obtaining a sample from a subject having a cancer or suspected of having a cancer; processing said sample; performing a binding assay comprising contacting the processed sample with a detectable label which binds to NMT2 nucleic acid to form a complex between the detectable label and NMT2 nucleic acid present in the sample, said binding assay generating at least one assay result indicative of said complex; and administering an NMT1 inhibitor to said subject when the amount to NMT2 nucleic acid in said sample is low or absent, optionally as compared to a control.

20. The method of item 9, wherein said analysis to determine whether said sample from the subject expresses NMT2, comprises performing a binding assay comprising contacting the processed sample with a detectable label which binds to NMT2 nucleic acid to form a complex between the detectable label and NMT2 nucleic acid present in the sample, said binding assay generating at least one assay result indicative of said complex.

21. The method of item 17, 18, 19, or 20, wherein said binding assay comprises, a hybridization assay using detectably labeled DNA or RNA probes.

22. The method of item 21, wherein said hybridization assay is quantitative or semi-quantitative.

23. The method of item 22, wherein said hybridization assay is RT-PCR, in situ hybridization, RNA protection assay ("RPA"), cDNA and oligonucleotide microarray, representation difference analysis ("RDA"), differential display, EST sequence analysis, serial analysis of gene expression ("SAGE"), and multiplex ligation-mediated amplification with the Luminex FlexMAP ("LMF").

24. The method of item 23, wherein instrumentation having a detector set to detect the complex between the detectable label and NMT2 nucleic acid present in the sample is used to determine an amount of complex in said sample.

25. The method of item 24, wherein said instrumentation is a spectrophotometer, spectrofluorometer, optical device, or electrochemical device.

26. The method of anyone of items 17 - 25, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

27. The method of any one of items 18 to 26, wherein said subject is a human.

28. A method, comprising: obtaining a sample from a subject having a cancer or suspected of having a cancer; processing said sample; performing a binding assay comprising contacting the processed sample with a an antibody to which binds to myristoylated protein, or azido-biotin labeled myristoylated proteins, within the sample to form a complex between the detectable label and myristoylated protein present in the sample, said binding assay generating at least one myristoylation profile indicative of said complex; wherein administering an NMT1 inhibitor to said subject is indicated when said myristoylation profile indicates said cancer is deficient in NMT2, optionally as compared to

a control

29. A method, comprising: obtaining a sample from a subject having a cancer or suspected of having a cancer; processing said sample; performing a binding assay comprising contacting the processed sample with a an antibody to which binds to myristoylated protein, or azido-biotin labeled myristoylated proteins, within the sample to form a complex between the detectable label and myristoylated protein present in the sample, said binding assay generating at least one myristoylation profile indicative of said complex; and administering an NMT1 inhibitor to said subject is when said myristoylation profile indicates said cancer is deficient in NMT2, optionally as compared to a control

30. The method of item 28 or 29, wherein said processing comprises treating said sample with ω-alkynyl-myristate and desthiobiotin azido-PEG biotin .

31. The method of items 28 or 30, wherein said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

32. The method of item 31, wherein instrumentation having a detector set to detect the complex formed between said antibody and said NMT2 in said sample is used to determine an amount of complex in said sample.

33. The method of item 32, wherein said instrumentation is a spectrophotometer, spectrofluorometer, optical device, or electrochemical device.

34. The method of anyone of items 28 to 34, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

35. The method of any one of items 31 - 35, wherein said subject is human.

36. Use of an NMT inhibitor for treating a subject having a cancer deficient in NMT2.

37. The use of item 36, wherein said NMT inhibitor comprises an NMT1 inhibitor.

38. The use of item 36, wherein said NMT1 inhibitor comprises a small molecule, an antibody, a peptide fragment, a nucleic acid, or combinations thereof.

39. The use of item 38, wherein said small molecule comprises Tris-DBA, HMA, or DDD85646, DDD86481, or a derivative thereof.

40. The use of item 38 or 39, wherein said small molecule comprises DDD86481.

41. The use of item 38, wherein said antibody is a monoclonal antibody or a polyclonal antibody.

42. The use of item 38, wherein said nucleic acid comprises a dsRNA molecule, a RNAi molecule, a miRNA molecule, a ribozyme, a shRNA molecule, or a siRNA molecule.

43. The use of any one of item 38 to 42, wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal

Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

44. The use of any one of items 36 to 43, wherein said subject is a human subject.

45. Use of an NMT1 inhibitor for treating a subject with a cancer, or suspected of having a cancer, wherein said an NMT1 inhibitor is indicated of use when the amount of NMT2 protein in a sample from said subject is low or absent, optionally as compared to a control, wherein a binding assay comprising contacting a processed sample from said subject with an antibody to NMT2 to form a complex between the antibody and NMT2 protein present in the processed sample generates at least one assay result indicative of said complex; wherein said assay result is indicative of said amount of NMT2 protein in said sample.

46. The use of item 45, wherein said analysis to determine whether said sample from the subject expresses NMT2, comprises performing a binding assay comprising contacting the processed sample with an antibody to NMT2 to form a complex between the antibody and NMT2 present in the processed sample, said binding assay generating at least one assay result indicative of said complex.

47. The method of any one of items 45 - 47, wherein said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

48. The use of any one of item 36 - 47, wherein instrumentation having a detector set to detect the complex formed between said antibody and said NMT2 in said sample is used to determine an amount of complex in said sample.

49. The use of item 48, wherein said instrumentation is a spectrophotometer, spectrofluorometer, optical device, or electrochemical device.

50. The use of anyone of items 36 to 49, wherein said wherein said cancer is lymphoma, B cell lymphoma, follicular lymphoma, diffuse large B-cell lymphoma, mantle cell lymphoma, B-CLL/SLL, immunocytoma/Waldenstrom’s, MALT-type/monocytoid B cell lymphoma, Burkitt’s lymphoma, a pediatric lymphoma, anaplastic large cell lymphoma, acute myeloid leukemia, Blast Phase Chronic Myeloid Leukaemia, Burkitt’s Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

51. The use of any one of items 36 - 50, wherein said subject is human.

52. Use of an NMT1 inhibitor for treating a subject with a cancer, or suspected of having a cancer, wherein said an NMT1 inhibitor is indicated of use when the amount of NMT2 nucleic acid in a sample from said subject is low or absent, optionally as compared to a control, wherein a binding assay comprising contacting a processed sample from said subject with a detectable label which binds to NMT2 nucleic acid to form a complex between the detectable label and NMT2 nucleic acid in said sample, said binding assay generating at least one assay result indicative of said complex.

53. The use of item 52, wherein said analysis to determine whether said sample from the subject expresses NMT2, comprises performing a binding assay comprising contacting the processed sample with a detectable label which binds to NMT2 nucleic acid to form a complex between the detectable label and NMT2 nucleic acid present in the sample, said binding assay generating at least one assay result indicative of said complex.

54. The use of item 52 or 53, wherein said binding assay comprises, a hybridization assay using detectably labeled

DNA or RNA probes.

55. The method of item 54, wherein said hybridization assay is quantitative or semi-quantitative.

56. The use of item 55, wherein said hybridization assay is RT-PCR, in situ hybridization, RNA protection assay ("RPA"), cDNA and oligonucleotide microarray, representation difference analysis ("RDA"), differential display, EST sequence analysis, serial analysis of gene expression ("SAGE"), and multiplex ligation-mediated amplification with the Luminex FlexMAP ("LMF").

57. The use of item 55, wherein instrumentation having a detector set to detect the complex between the detectable label and NMT2 nucleic acid present in the sample is used to determine an amount of complex in said sample.

58. The use of item 55, wherein said instrumentation is a spectrophotometer, spectrofluorometer, optical device, or electrochemical device.

59. The use of anyone of items 53 - 58, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

60. The use of any one of items 53 to 59, wherein said subject is a human.

61. Use of an NMT1 inhibitor for treating a subject with a cancer, or suspected of having a cancer, wherein said use of said NMT1 inhibitor is indicated when a myristoylation profile from a sample from said subject indicates said cancer is deficient in NMT2, optionally as compared to a control, wherein performing a binding assay comprising contacting a processed sample with an antibody which binds to myristoylated protein, or azido-biotin labeled myristoylated proteins, within the sample to form a complex between the detectable label and myristoylated protein present in the sample, said binding assay generating at least one myristoylation profile indicative of said complex

62. The use of item 61, wherein said processing comprises treating said sample with alyknyl-myristate and desthiobiotin azido-PEG biotin .

63. The use of items 61 or 62, wherein said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

64. The use of item 63, wherein instrumentation having a detector set to detect the complex formed between said antibody and said NMT2 in said sample is used to determine an amount of complex in said sample.

65. The use of item 36, wherein said instrumentation is a spectrophotometer, spectrofluorometer, optical device, or electrochemical device.

66. The use of anyone of items 61 to 65, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

67. The use of any one of items 62 - 66, wherein said subject is human.

68. A method for identifying a subject suitable for treatment with an NMT1 inhibitor, comprising: obtaining a sample from said subject with a cancer or suspected of having a cancer; processing said sample; performing a binding assay comprising contacting the processed sample with an antibody to NMT2 to form a complex between the antibody and NMT2 protein present in the processed sample, said binding assay generating at least one assay result indicative of said complex; wherein treatment with said NMT1 inhibitor is indicated when the amount of NMT2 protein said sample is low or absent, optionally as compared to a control.

69. The method of item 68, wherein said analysis to determine whether said sample from the subject expresses NMT2, comprises performing a binding assay comprising contacting the processed sample with an antibody to NMT2 to form a complex between the antibody and NMT2 present in the processed sample, said binding assay generating at least one assay result indicative of said complex.

70. The method of items 68 or 69, wherein said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

71. The method of any one of item 68 - 70, wherein instrumentation having a detector set to detect the complex formed between said antibody and said NMT2 in said sample is used to determine an amount of complex in said sample.

72. The method of item 71, wherein said instrumentation is a spectrophotometer, spectrofluorometer, optical device, or electrochemical device.

73. The method of anyone of items 68 to 72, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

74. The method of any one of items 68 - 73, wherein said subject is human.

75. A method for identifying a subject suitable for treatment with an NMT1 inhibitor, comprising: obtaining a sample from a subject having a cancer or suspected of having a cancer; processing said sample; performing a binding assay comprising contacting the processed sample with a detectable label which binds to NMT2 nucleic acid to form a complex between the detectable label and NMT2 nucleic acid present in the sample, said binding assay generating at least one assay result indicative of said complex; wherein administering an NMT1 inhibitor to said subject is indicated when the amount to NMT2 nucleic acid in said sample is low or absent, optionally as compared to a control.

76. The method of item 75, wherein said analysis to determine whether said sample from the subject expresses NMT2, comprises performing a binding assay comprising contacting the processed sample with a detectable label which binds to NMT2 nucleic acid to form a complex between the detectable label and NMT2 nucleic acid present in the sample, said binding assay generating at least one assay result indicative of said complex.

77. The method of item 76, wherein said binding assay comprises, a hybridization assay using detectably labeled DNA or RNA probes.

78. The method of item 77, wherein said hybridization assay is quantitative or semi-quantitative.

79. The method of item 78, wherein said hybridization assay is RT-PCR, in situ hybridization, RNA protection assay ("RPA"), cDNA and oligonucleotide microarray, representation difference analysis ("RDA"), differential display, EST sequence analysis, serial analysis of gene expression ("SAGE"), and multiplex ligation-mediated amplification with the Luminex FlexMAP ("LMF").

80. The method of item 79, wherein instrumentation having a detector set to detect the complex between the detectable label and NMT2 nucleic acid present in the sample is used to determine an amount of complex in said sample.

81. The method of item 80, wherein said instrumentation is a spectrophotometer, spectrofluorometer, optical device, or electrochemical device.

82. The method of anyone of items 75 - 81, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

83. The method of any one of items 75 to 82, wherein said subject is a human.

84. A method for identifying a subject suitable for treatment with an NMT1 inhibitor, comprising: obtaining a sample from a subject having a cancer or suspected of having a cancer; processing said sample; performing a binding assay comprising contacting the processed sample with a an antibody to which binds to myristoylated protein, or azido-biotin labeled myristoylated proteins, within the sample to form a complex between the detectable label and myristoylated protein present in the sample, said binding assay generating at least one myristoylation profile indicative of said complex; wherein administering an NMT1 inhibitor to said subject is indicated when said myristoylation profile indicates said cancer is deficient in NMT2, optionally as compared to a control

85. The method of item 84, wherein said processing comprises treating said sample with alyknyl-myristate and desthiobiotin azido-PEG biotin.

86. The method of items 84 or 85, wherein said binding assay comprises fluorescence activated cell sorting, enzyme linked immunosorbent assay, immunohistochemistry, quantitative immunohistochemistry, fluorescence resonance energy transfer, Forster resonance energy transfer, biomolecular fluorescence complementation, mass spectrometry, immunoblot assay or coimmunoprecipitation assay.

87. The method of item 86, wherein instrumentation having a detector set to detect the complex formed between said antibody and said NMT2 in said sample is used to determine an amount of complex in said sample.

88. The method of item 87, wherein said instrumentation is a spectrophotometer, spectrofluorometer, optical device, or electrochemical device.

89. The method of anyone of items 84 to 88, wherein said wherein said cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Burkitt's Lymphoma, Plasma Cell Myeloma, Intestinal Adenocarcinoma, Lung mixed Adenosquamous Carcinoma, Lung Small Cell Carcinoma, Lung, Oesophagus Squamous Cell Carcinoma, Bone, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast

carcinoma, colorectal adenocarcinoma, pancreas adenocarcinoma, ovarian carcinoma, non-small cell lung carcinoma, osteosarcoma, melanoma, gastric adenocarcinoma, endometrial adenocarcinoma, esophageal squamous carcinoma.

90. The method of any one of items 84 - 89, wherein said subject is human.

91. A kit for identifying a subject suitable for treatment with an NMT1 inhibitor, comprising: an antibody to NMT2; instructions for identifying the subject according to the method of any one of items 68-74.

92. The kit of item 91, further comprising a control.

93. A kit for identifying a subject suitable for treatment with an NMT1 inhibitor, comprising: a nucleic acid for binding to NMT2; instructions for identifying the subject according to the method of any one of items 75-83.

94. The kit of item 93, further comprising a control.

95. The kit of item 93, wherein said nucleic acid is RNA or DNA.

96. A kit for identifying a subject suitable for treatment with an NMT1 inhibitor, comprising: NeutrAvidinTM-HRP; and instructions for identifying said subject according to any one of items 84-90.

97. The kit of item 96, further comprising a control.

98. The kit of item 97, further comprising $\omega$-alkynyl-myristate or desthiobiotin azido-PEG biotin.

To gain a better understanding of the invention described herein, the following examples are set forth. It should be understood that these example are for illustrative purposes only. Therefore, they should not limit the scope of this invention in any way.

## EXAMPLES

In the following examples, standard methodologies were employed, as would be appreciated by the skilled worker.

## MATERIALS AND METHODS

### Antibodies and reagents.

Tris dibutylbenzinylidene acetone paladium (TrisDBA) was a kind gift of Dr. Arbiser (U. Alabama). DDD85646 was synthesized as described [J.A.Frearson et al (2010) Nature. 464.728-723)] and/or DDD86481 was obtained from Dr. David Gray and Paul Wyatt, Dundee University)

Mouse anti-NMT1 (clone 14; 1:1000) and mouse anti-NMT2 (clone 30; 1:2000) antibodies were from BD Biosciences, San Jose, CA, USA. Rabbit anti-NMT1 (polyclonal, 1:3000) was purchased from Proteintech, Chicago, IL, USA. Rabbit anti-GFP (1:20,000), anti-PARP-1 (1:5000), anti-GAPDH (1:5000) and antic-terminal PAK2 (1:2000) antibodies were from Eusera (www.eusera.com), Edmonton, AB, Canada. Mouse anti-$\alpha$-tubulin (1:15,000) and rabbit-anti-V5 (1:10,000) antibodies were purchased from Sigma Aldrich, St. Louis, MO, USA. Mouse anti-His (1:2000) was from Qiagen, Germany. Rabbit anti-cleaved caspase-8 (1:1000) and anti-cleaved caspase-3 (1:1000) were both from Cell Signaling, Danvers, MA, USA. Enhanced chemiluminesce (ECL) Plus and ECL Prime western blotting detection kits were purchased from GE Healthcare, Pittsburgh, PA, USA. Unless stated otherwise, all chemicals used were purchased from Sigma-Aldrich (St. Louis, MO, USA) and were of the highest purity available.

**DNA constructs. Engineering of V5- and His-tagged NMT1 and NMT2 constructs.** NMT1 and NMT2 entry vectors which are compatible with the Gateway cloning system (Life Technologies, Grand Island, N.Y., USA) were purchased from Genecopoeia (Rockville, MD, USA). The NMT1 and NMT2 genes were incorporated into the destination vector pcDNA3.1/nV5 DEST (Life Technologies) using the LR clonase enzyme (Life Technologies) according to the manufacturer's instructions to generate the plasmids N-terminally-tagged NMTs (His-NMT1, His-NMT2, V5-NMT1 and V5-NMT2). V5-tagged NMT constructs were used for mammalian cell expression, whereas His-NMT constructs were used for bacterial expression. The cloning products were confirmed by DNA sequencing (Eurofins MWG Operon, Huntsville, AL, USA).

**Cell culture.** Origin of the B cells were a gift from Dr. Jim Stone or were obtained from ATCC. All reagents from cell culture were purchased from Invitrogen. B cells were cultured at 37°C and 5% $CO_2$ in a humidified incubator and maintained in RPMI media supplemented with 10% fetal bovine serum, 100 U/ml penicillin and 0.1 mg/ml streptomycin.

**Cell lysis.** Cells were washed in cold PBS, lysed in 0.1% SDS-RIPA buffer [50 mM Tris, 150 mM NaCl, 1% Igepal CA-630, 0.5% NaDC, 2 mM $MgCl_2$, and 1x complete protease inhibitor (Roche Diagnostics); pH 8.0] and rocked for 15 min at 4°C. Cell lysates supernatant were obtained after a 16,000g centrifugation for 15 min at 4°C.

**Induction of apoptosis.** Unless mentioned otherwise, apoptosis was induced using 2.5 $\mu$M staurosporine (STS) (Sigma Aldrich, St. Louise, MO, USA) and 5 $\mu$g/mL cycloheximide (ICN Biochemicals Inc. Aurora, OH, USA) in order to inhibit protein translation and enhance apoptosis induction.

**Incubation with NMT inhibitors.** Tris dibutylbenzinylidene acetone paladium (TrisDBA) was a kind gift of Dr. Arbiser. Cells were incubated at increasing concentrations for 24 hours with TrisDBA (or DMSO for control) or for 24 and 48 hours with DDD85646.

**B cell transfection.** B cells were transfected using the Neon® transfection system (Life technologies) following manufacturer's instructions and optimized protocol for Ramos B cells transfection (pulse voltage 1,300V; pulse width 20 ms, 2 pulses and $7.7.10^6$ cells/mL) adapted for 100 $\mu$L tips. Classically, two transfections were pulled to obtained enough living cells to perform a viability assay.

**Cell viability assay.** B and T cell viability was measured using the trypan blue exclusion method. Cells were grown in confluency conditions (2 x $10^6$ cells/mL maximum) assuring the minimum basal apoptosis. After incubation with NMT inhibitors, about 20 000 cells (10 $\mu$L) were incubated with 10 $\mu$L of TC10™ Trypan Blue Dye (Biorad) for 15 min. Cell viability was quantified using the TC10™ automated cell counter (Biorad).

***In vitro* NMT activity assay.** N-myristoyltransferase activity assay protocol was adapted from Raju, R. V., and Sharma, R. K. (1999) Preparation and assay of myristoyl-CoA:protein N-myristoyltransferase. Methods Mol Biol 116, 193-211. [$^3$H] myristoyl-CoA was freshly synthesized for each experiment, as previously described by Towler, D., and Glaser, L. (1986) Protein fatty acid acylation: enzymatic synthesis of an N-myristoylglycyl peptide. Proc Natl Acad Sci U S A 83, 2812-2816. Briefly, cells were resuspended in 0.25 M sucrose buffer (50 mM $NaH_2PO_4$, pH 7.4) and subjected to 2 rounds of sonication at level 6.0 on a Branson Sonicator. Reaction mixture is composed of 10 $\mu$L of cell extract (about 20 $\mu$g of proteins) incubated in NMT activity buffer (0.26M Tris-HCl, 3.25 mM EGTA, 2.92 mM EDTA and 29.25 mM 2-mercaptoethanol, 1% Triton X-100, pH 7.4) and myristoylable or non-myristoylable decapeptide corresponding to the N-terminal sequence of truncated-Bid (0.1 mM dissolved in water). Reaction was started by the addition of 7.4 $\mu$L ($\approx$ 10pMol) of freshly synthesized [$^3$H] myristoyl-CoA (final mixture volume = 25 $\mu$L) and incubated for 15 min at 30°C. The reaction is stopped by spotting 15 $\mu$L of the reaction mixture on a P81 phosphocellulose paper disc (Whatman, Kent, UK) and dried for 30 seconds. Discs were washed (washing buffer: 25 mM Tris buffer, pH 7.4) to remove the residual radioactivity ([$^3$H]-myristate and [$^3$H]-myristoyl-CoA) while [$^3$H]-myristoylpeptide is retained on the phosphocellulose paper. Radioactivity was quantified by liquid scintillation counting and converted into pMol of myristoylated peptide (Raju, R. V., and Sharma, R. K. (1999) Preparation and assay of myristoyl-CoA:protein N-myristoyltransferase. Methods Mol Biol 116, 193-211

**RT-PCR.** qRT-PCR was performed with Taqman NMT1 and NMT2 probes using an 18S probe as an internal control. The difference in the number of cycle times ($\Delta$ct) was calculated by subtracting the cycle time (ct) at which we see an exponential increase in the expression of the 18S internal control for each cell type from the NMT cycle time, again at a point where exponential increase of the signal is seen.

**Mutation of caspase cleavage sites in V5-NMT by site-directed mutagenesis**

The NMT1 and NMT2 caspase cleavage sites identified by Edman degradation sequencing (Alphalyse) were mutated by site-directed mutagenesis. Therefore, we used previously cloned V5-NMT1 and V5-NMT2 gateway vectors to mutate the identified caspase cleavage sites. Hence, the Asp-72 residue of V5-NMT1 and Asp-25, Asp-67 and both Asp-25,67 (double mutant) residues of V5-NMT2 were mutated using the Quickchange® site-directed mutagenesis kit (Agilent Technologies) according to manufacturer's instructions.

Briefly, site-directed mutagenesis was performed using 5 to 50 ng of dsDNA (vector), 5 $\mu$L of 10x reaction buffer, 125 ng of primers dissolved in nuclease-free water and was brought up to a final reaction volume of 50 $\mu$L with nuclease-free water. 2.5 U of PfuTurbo DNA polymerase (Agilent Technologies) was added to the mix prior to starting the reaction, which was performed for 18 cycles in the Eppendorf Mastercycler 1 thermocycler. Subsequently, the parental dsDNA was digested by adding 10 U of Dpn I restriction enzyme to each reaction and incubating for 1 h at 37°C. Additionally, the primers for site-directed mutagenesis were designed using the PrimerX program (http://www.bioinformatics.org/primerx/) (listed in table 2.6). Importantly, the aspartate (D) residues of the caspase cleavage sites were mutated into glutamate (E) residues to generate the following vectors; V5-NMT1 (D72E), V5-NMT2 (D25E), V5-NMT2 (D67E) and V5-NMT2 (D25,67E). The mutations were confirmed by automated DNA sequencing (Eurofins MWG Operon).

**Generation of His-tagged caspase-cleaved, truncated NMT vectors**

The following caspase cleaved, truncated NMTs were generated by molecular cloning; His-73NMT1, His-26NMT2 and His-68NMT2. The truncated DNA sequences were cloned into the pET-19b (Novagen®) vector, which has an N-terminal hexa histidine (His)-tag sequence.

Previously cloned gateway vectors, GFP-NMT1 and GFP-NMT2 were used as templates for these PCR reactions. PCR reactions were performed using Platinum Pfx® DNA polymerase (Life Technologies) and PCR reactions were set up as per manufacturer's guidelines. Each PCR reaction was prepared as follows; 200ng DNA template, 5 μL 10X Pfx amplification buffer, 5 μL 10X PCR enhancer solution, 1 mM MgSO4, 0.3 mM dNTP mixture, 0.5 μM each of forward and reverse primers (listed in table 2.6), 1 U Platinum Pfx® DNA polymerase and nuclease free water upto 50 μL. The reaction was set up in the Eppendorf Mastercycler 1 Thermocycler as per guidelines provided in the Platinum Pfx® DNA polymerase kit and the reactions were performed in 30 cycles.

**Inhibition of caspases**

Cells were pre-treated with 10 μM of established caspase inhibitors purchased from EMD Chemicals, 1 hour prior to the induction of apoptosis. The caspase inhibitors used were general caspase inhibitor (z-VAD-FMK), caspase-3 (z-DEVD-FMK), caspase-8 (z-IETD-FMK), caspase-9 (z-LEHD-FMK) and negative control (z-FA-FMK). The control cells were treated with DMSO (1:1000).

**Treatment of cells with MG-132**

IM9, KMH2, BL2 and Ramos cells were plated (3x106 cells per well in a 6-well dish) and treated with 10 μM MG-132 for 5 hours. Equal amount of DMSO was added to the control samples. Cells were then lysed and subjected to SDS-PAGE/Western blot analyses.

**Treatment of cells with suberoylanilide hydroxamic acid (SAHA)**

IM9, BL2 and Ramos cells were plated at 3x106 cells per well in a 6-well dish and treated with 1 μM suberoylanilide hydroxamic acid (SAHA), which is a reversible inhibitor of class I and class II histone deacetylases (HDACs), for 24 hours. Equal amount of DMSO was added to cells as a control. Cells were lysed and subjected to SDS-PAGE/Western blot analyses.

**Apoptosis Induction**

150 or 300 ng/mL mouse anti-Fas antibody was used to stimulate apoptosis through the extrinsic pathway and 2.5 μM staurosporine (STS) was used to stimulate apoptosis through the intrinsic pathway. Where indicated, cycloheximide (5 μg/mL) was used to inhibit protein translation and to further promote cell death.

**Treatment of cells with NMT inhibitor, 2-hydroxymyristic acid** (HMA)

HMA was saponified and conjugated to bovine serum albumin (BSA) prior to addition to cells to facilitate its cellular uptake as described previously (Yap et al., 2010). Briefly, HMA was incubated with a 20% molar excess of potassium hydroxide (KOH) at 65°C for 15 min. Subsequently, a 20X solution was made by dissolving the KOH saponified HMA in serum-free RPMI media containing 20% fatty acid-free BSA at 37°C, followed by an additional 15 min incubation at 37°C. Since sodium myristate was used as a control, it was also incubated in serum-free RPMI media containing 20% fatty acid-free BSA at 37°C prior to addition to cells. Subsequently, Jurkat T cells (1 x 107 cells) were incubated with either 1mM HMA or 1mM sodium myristate conjugated to fatty-acid free BSA (final concentration in cell culture 1%) for 1 h at 37oC in RPMI media (without FBS, supplements or antibiotics). After incubation, apoptosis was induced using anti-Fas (150 ng/mL) or STS (2.5 μM) with cycloheximide (5 μg/mL) or vehicle alone (DMSO). Samples were collected every 2 h for an 8 h period, washed with cold PBS and lysed with 0.3 mL of 1% sodium dodecyl sulfate (SDS)-radio-immuno-precipitation assay (RIPA) buffer and subjected to 2 rounds of sonication for 15 seconds at an output of 5.5-6.5 on a Branson Sonifier and placed on ice for 2 min in between each cycle.

**Treatment of lymphocytic cell lines with NMT inhibitor, Tris DBA**

2 x $10^6$ cells (CEM, L0, BL2 and Ramos) were grown in 6 well plates and incubated with increasing amounts (0, 1, 2 and 5 μg/ml) of Tris (dibenzylideneacetone) dipalladium (Tris DBA) (Bhandarkar et al., 2008). The viability of

cells treated with Tris DBA was measured with a trypan blue exclusion assay (Hudson, 1976).

**Treatment of lymphocytic cell lines with NMT inhibitors, DDD85646 and DDD73226**

1 x $10^5$ cells [KMH2 (Hodgkins lymphoma), IM9 ("normal" EBV immortalized B cell), BL2, Ramos] (100 μL/well) were plated in 96-well plates and treated with increasing amounts of the pyrazole sulfonamide based NMT inhibitor DDD85646 [Molecular weight (MW) 495.43] (Frearson et al., 2010) and a less potent pyrazole sulfonamide based analog DDD73228 (MW=362.5) for 24, 48 and 72 hours. The drugs were dissolved in DMSO to make 100X stock solutions for each concentration tested, so that the final volume of DMSO in each well was 1%. The viability of cells treated with these inhibitors was measured with a trypan blue exclusion assay (Hudson, 1976).

**Treatment of lymphocytic cell lines with NMT inhibitor, DDD86481**

1 x $10^5$ cells [KMH2 (Hodgkin's lymphoma), IM9 ("normal" EBV immortalized B cell), BL2, Ramos] (100 μL/well) were plated in 96-well plates and treated with increasing amounts of DDD86481 [MW 610.5]. The drugs were dissolved in DMSO to make 100X stock solutions for each concentration tested, so that the final volume of DMSO in each well was 1%. MTS [(3-(4,5-dimethylthiazol-2-yl)-5-(3-carboxymethoxyphenyl)-2-(4-sulfophenyl)-2H-tetrazolium)] assay was used to measure the cytotoxicity of the drug (Cory et al., 1991).

**Cell lysis**

Typically, cells were washed in cold PBS, harvested, lysed in 0.1% SDS-RIPA buffer or 0.1% SDS-RIPA-HEPES buffer (when cells were labeled with alkyne-fatty acids) that was supplemented with 1X complete protease inhibitor. The cell suspension was rocked for 15 min at 4°C. Cell lysates were then centrifuged at 16,000 g for 10 min at 4°C and the post-nuclear supernatant was collected. Protein concentrations were measured using PierceTM bicinchoninic acid (BCA) protein assay kit (Thermo Fisher Scientific, Waltham, MA).

**Lysis of Lymphoma tissue samples**

Human diffuse large B-cell lymphoma (DLBCL) and follicular lymphoma (FL) tissues were kind gifts from Dr. Raymond Lai (Cross Cancer Institute, Alberta, Canada). The frozen tumor tissues were cut into small (~1 mm3) pieces and mixed with 1% SDS-RIPA with 1X complete protease inhibitor. Samples were homogenized using a small Dounce homogenizer and then sonicated repeatedly for 2 min with 1 min intervals (on ice) in between at an output of 6.0 (Branson Sonifier 450) until the tumor tissues dissolved in the lysis buffer. The samples were then centrifuged at 16,000 g for 10 min at 4oC and the post-nuclear supernatant was collected for western blotting analyses.

**Western blotting using Odyssey scanner**

This method was only used for the cleaved caspase-3 blot provided in, all other western blotting was done using the standard procedure. Following electrophoresis, gels were transferred onto a nitrocellulose membrane and blocked in 5% NFM in PBS with 0.1% Tween 20 (PBS-T) for 1 hour. The primary antibody (rabbit anti-cleaved caspase-3; Cell Signaling) was also diluted at 1:2000 in 5%NFM in PBS-T, added to the membrane and incubated for 24 h. Next, the membranes were subjected to 6 wash cycles, lasting 5 min each; 2 washes in PBS, 2 washes in PBS-T, and finally 2 washes in PBS. The Secondary antibody (Alex Fluor 680 goat-anti-rabbit; Life Technologies) was added after diluting in PBS-T (1:5000) and the blots were covered in aluminum foil and incubated with the secondary antibody for 1h and subjected to the same 6X wash cycle with PBS and PBS-T as before. Blots were scanned on Odyssey ® fluorescence imaging system from LI-COR Inc. at a scanning resolution of 84 μm (Intensity: 5 to 7) on channel 700 (for rabbit).

**Subcellular fractionation**

HeLa cells were grown to confluency in 150 mm plates and were induced to undergo apoptosis with anti-Fas (300 ng/mL) (extrinsic) or STS (intrinsic) for a period of 5 h. Cells were then metabolically labeled with alkyne-C14 (described in detail in the section below) (Yap et al., 2010) and subjected to hypotonic lysis.

Briefly, cells were washed with cold PBS buffer, scraped off the plates using a cell lifter, and collected. Samples were then centrifuged at 2000 g for 5 min, the supernatant was aspirated and cells were resuspended in 600 μL of hypotonic buffer that causes cells to swell (Mg resuspension buffer) and incubated on ice for 20 min. The cell suspension was transferred to a Dounce homogenizer and homogenized with 45 strokes using the tight pestle. Consequently, 400 μL of EDTA-free 2.5X homogenization buffer (HB) was added to the homogenate and the cells were homogenized with

15 strokes in the Dounce homogenizer using the loose pestle. The homogenate was then centrifuged at 1000 g for 5 min and the post-nuclear supernatant (PNS) was collected. Next, 200 μL of PNS was saved and remainder (~750 μL) was centrifuged at 100,000 g for 45 min in a Beckman TLA 120.2 rotor, which resulted in a cytosolic fraction (S100) and a light membrane pellet (P100).

The P100 pellet was washed once with 1X HB and the P100 fractions were adjusted to the volume of the cognate S100 fraction using 1X HB. Following this, the resulting post-nuclear supernatant (PNS), cytosolic fractions (S100) and membrane fractions (P100) fractions were adjusted to contain 1% SDS. Consequently, the same fraction volume was subjected to western blot analysis and protein levels were quantified using Image J software (http://rsbweb.nih.gov/ij/).

**Metabolic labeling of cells using bio-orthogonal ω-alkynyl myristic acid and click chemistry**

Metabolic labeling of cells using ω-alkynyl myristic acid

Cells were labeled with 100 to 25 μM ω-alkynyl myristic acid 30 min prior to harvesting the cells and lysed in 0.1%SDS-RIPA-HEPES buffer. Protein (50 - 30 μg) from the resulting cell lysates were reacted with 100 μM azido-biotin using click chemistry and processed as described previously (Yap et al., 2010). Briefly, cells were starved of fatty acids by incubating in media (RPMI or DMEM) which were supplemented with 5% dextran-coated charcoal-treated FBS (DCC-FBS) for 1 h prior to labeling. Next, ω-alkynyl-myristic acid was dissolved in DMSO to generate 25 or 100 mM stock solutions. Typically, the cellular uptake of ω-alkynyl myristic acid typically was improved with saponification and conjugation to BSA. Therefore, prior to labeling, ω-alkynyl myristic acid was saponified with 20% molar excess of potassium hydroxide at 65°C for 15 min. Next, serum-free culture media (prewarmed) containing 20% fatty acid-free BSA at 37°C was added to the saponified ω-alkynyl myristic acid and incubated for an additional 15 min at 37°C.

Subsequently, cells deprived of fatty acids were washed once with warm PBS and incubated in fresh RPMI or DMEM without any supplements. Next, the appropriate volume of 20X fatty acid-BSA conjugate in serum-free media was added to the cells, to ensure that the final concentration of BSA was 1% and □-alkynyl myristic acid was at the indicated concentration (25 μM or 100 μM) for each respective experiment. DMSO or unlabeled fatty acids conjugated to BSA were used as controls for the experiments performed. Cells were labeled with ω-alkynyl myristic acid for 30 min at 37°C in a 5% CO2 humidified incubator prior to harvesting. Cells were harvested in 0.1% SDS-RIPA-HEPES buffer that was supplemented with 1X complete protease inhibitor (EDTA-free).

**Click chemistry**

After labeling cells with ω-alkynyl myristic acid, the resulting lysates were subjected to click chemistry with azido-biotin to enable the detection of the myristoylated proteins by ECL as described previously (Yap et al., 2010). Typically, cell lysates (30 - 50 μg of protein) were adjusted to contain 1% SDS and incubated with 100 μM Tris-(benzyltriazolylmethyl)amine (TBTA), 1 mM CuSO4, 1 mM Tris-carboxyethylphosphine (TCEP), and 100 μM azido-biotin at 37°C for 30 min in darkness, in order for the click reaction to proceed. Next, 10 volumes of ice-cold acetone was added to stop the click reaction and proteins were precipitated at -20°C overnight. Subsequently, the acetone precipitated proteins were centrifuged at 16,000 g for 10 min, resuspended in 1X SDS-PAGE sample buffer with 20 mM DTT. Samples were then heated at 95oC for 5 min and subjected to SDS-PAGE, and transferred on to PVDF membranes for western blot analysis.

**Treatment of PVDF membranes with neutral Tris-HCl or KOH**

Protein samples were loaded on duplicate SDS-PAGE gels for the experiments where PVDF membranes were treated with KOH or neutral Tris-HCl. After electrophoresis, the KOH treated PVDF membranes were incubated in 100 ml of 0.1 N KOH in methanol [1 N KOH in H2O: methanol 1:9 (v/v)], whereas the other was incubated in 0.1 N Tris-HCl pH 7.0 in methanol [1 N Tris-HCl, pH 7.0:methanol 1:9 (v/v)] at room temperature for 45 min with gentle shaking. Subsequently, the treated membranes were washed thoroughly with PBS, probed with Streptavidin-HRP or Neutravidin-HRP and detected with ECL.

**Radioactive NMT activity assay in cells undergoing apoptosis**

NMT activity was measured by adapting a protocol developed by the Sharma laboratory (King and Sharma, 1991; Raju and Sharma, 1999). A stock solution of [3H]-myristoyl-CoA was prepared freshly and synthesized as described previously (Towler and Glaser, 1986). To make a 200 μL stock solution of [3H]-myristoyl-CoA, 97 μL of myristoyl-CoA generation buffer was combined with 20 □L of 50 mM ATP, 10□□□L of 20 mM LiCoA, 60 μL of pseudomonas acyl-

CoA synthetase and 13 μL of [9,10 - 3H]-myristic acid. The solution was mixed gently and incubated at 37°C for 30 min.

For this assay, COS-7 cells transiently transfected with plasmids encoding for V5-NMT1 and V5-NMT2 were induced to undergo apoptosis with STS (2.5 μM) and cycloheximide (5 μg/ml) or not, and, harvested at 0, 1, 2, 4 and 8 h time points. The cells were sonicated using a Branson sonifier 450 and -20 μg of lysate (lysed in 50 mM NaH2PO4 pH 7.4, 0.25M sucrose buffer) was used for each reaction. For each NMT assay reaction, 3.85 μL of NMT assay buffer, 1.25 μL 20% Triton X-100 and 2.5 μL (0.1 mM) myristoylatable (BID_G: GNRSSHSRLG) or non-myristoylatable (BID_A: ANRSSHSRLG) decapeptide (purchased from Peptide 2.0 Inc.) corresponding to the N-terminal sequence of ct-Bid (1mM stock in ethanol) was added to a microcentrifuge tube and kept on ice before the start of the reaction. At the start of the reaction, 7.4 μL (10 pMol) of freshly synthesized of [3H] myristoyl-CoA was added to each microcentrifuge tube containing the NMT assay mixture at 15 second intervals and 25 μL reactions were incubated for 15 min at 30oC. The reaction was terminated by spotting 15 μL of the reaction mixture onto a P81 phosphocellulose paper disc (Whatman) at 15 second intervals and dried with a hair dryer for 30 seconds.

Next, the p81 phosphocellulose paper discs were transferred to the washing unit and washed 3 times, 30 min each, with NMT assay wash buffer for a total period of 90 min. Subsequently, the radioactivity remaining on the phosphocellulose (which corresponds to the myristoylated peptide) was quantified in 5 mL of liquid scintillation mixture using a Beckman Coulter LS6500 scintillation counter. The NMT activity was calculated as fmol/min/μg of protein.

**Use of a radioactive assay to measure NMT activity in purified His-tagged NMTs**

The NMT activity of full length and truncated His-tagged NMTs were measured using the same protocol as described above. However, only 1 μg of purified protein (volume was brought up to 10 μL in NMT assay buffer) was used in this assay.

**In vitro NMT cleavage assay**

The in vitro NMT cleavage assay was performed by incubating 10 □g of purified His-NMT1 and His-NMT2 with 1 μg of recombinant human active caspase-3 or -8 in caspase cleavage assay buffer in 100 μL reactions for 1 hour at 37 oC. Reaction was terminated with the addition of 10 μM general caspase inhibitor z-VAD-FMK and subsequently 5X sample loading buffer were added. Reacted samples were separated on a 10% SDS-PAGE gel and transferred onto a PVDF membrane. The bands were visualized by Coomassie blue staining and cleavage fragments were excised from the membrane and sent for Edman degradation to Alphalyse in Palo Alto, CA.

**Protein purification of recombinant His-NMTs**

Lemo21(DE3) pLysS competent cells (New England Biolabs) were transformed with His-NMT1 and His-NMT2 vectors according to manufacturer's protocol. NMT1 and NMT2 proteins were prepared as follows: a 3 mL starter culture was grown in LB broth (1% tryptone, 0.5% yeast extract, 0.5% NaCl, 100 μg/mL ampicillin and 34 μg/mL Chloramphenicol) for 4 h at 37oC, while shaking at 225 rpm. The entire culture was used to inoculate a 50 mL culture which was grown at 37oC for 16 h. Next, the bacterial cells were pelleted by centrifugation at 6000 x g for 10 min at room temperature. The bacterial pellet was resuspended in 10 mL LB and 5 mL of the resuspension was used to inoculate 500 mL of LB that was incubated at 37oC with vigorous shaking (225 rpm) until an OD600 of 0.5-0.6 was reached.

Next, protein expression was induced by 0.5 mM Isopropyl β-D-1-thiogalactopyranoside (IPTG) addition at 30oC with vigorous shaking (225 rpm) for 4 h. The suspension was centrifuged at 6000 x g at 4oC for 20 min and the bacterial pellet was frozen overnight. Subsequently, the thawed bacterial pellet was resuspended in 25 mL bacterial lysis buffer supplemented with complete protease inhibitor (CPI) from Roche and incubated on ice for 30 min. Samples were then sonicated 3 times for 1 min with 1 min intervals in between at an output of 5.0 (Branson Sonfier 450) and incubated at 37oC for 30 min prior to centrifugation at 15,000 x g for 1 h at 4oC.

Meanwhile, Ni-NTA agarose beads (His-Pure Ni-NTA resin from Thermo Fisher Scientific) were packed into columns according to manufacturer's instructions and washed with column buffer three times. The clear supernatant obtained from the previous centrifugation was then loaded into the column and incubated with gentle shaking at 4oC for 1 h. The sample was then eluted from the column by gravity flow and the column was washed twice with 5 mL wash buffer (column buffer with 20 mM imidazole, pH 8.0). Next, 2 x 5 mL of elution buffer 1 (column buffer with 75 mM imidazole, pH 8.0) was used to elute the first two fractions and 2 x 5 mL of elution buffer 2 (column buffer with 150 mM imidazole, pH 8.0) was used to elute the next two fractions. The last fraction was eluted with elution buffer 3 (column buffer with 250 mM imidazole, pH 8.0). Finally, 20% glycerol was added to each of the fractions collected before freezing at -80oC. Samples from each step of the protein purification process was collected, run on a 12.5% SDS-PAGE gel and stained with Coomassie blue in order to determine which fraction(s) had the highest concentration of protein.

Since samples from the first four elutions had the highest protein concentration, they were pooled and dialysed

to remove any imidazole present. Hence, the pooled samples were loaded on to Spectra/Por 2 (Spectrum Laboratories) dialysis tubing with a molecular weight cut off (MWCO) 12 - 14 kDa to remove any small degraded protein fragments. Protein samples were then dialyzed in 4 L of chilled dialysis buffer for 2 h at 4oC with gentle stirring, followed by overnight dialysis with the same buffer at 4oC with another 4 L of fresh, chilled buffer. Dialyzed protein samples were concentrated by spinning in Amicon Ultra-15 centrifugal filter with MWCO 30 kDa (Millipore) at 5,000 g, 4oC until desired volumes were achieved.

**qRT-PCR of B cell lymphoma cell lines**

RNA was isolated from IM9, KMH2, Ramos and BL2 cells using TRIzol® reagent according to manufacturer's protocols. Next, the High Capacity cDNA Reverse Transcription kit with a random primer scheme from Applied Biosciences was used to synthesize cDNA from the isolated RNA according to manufacturer's instructions. Quantitative real time PCR (qRT PCR) reactions were set-up using the TaqMan® Universal Master Mix II and NMT1, NMT2 and 18 Taqman® probes purchased from Life Technologies (Carlsbad, CA) and three replicates of each reaction were set up according to supplier's guidelines. qRT PCR was performed using a Mastercycler® ep realplex thermocycler (Eppendorf) and results were analyzed using the Realplex software (Eppendorf).

**Trypan Blue Exclusion Assay**

The viability of cells treated with Tris DBA, DDD73228, and DDD85646 was measured by incubating cells with TC10TM Trypan Blue Dye (Biorad) (Hudson, 1976), according to manufacturer's instructions. Cell viability was then quantified using the TC10TM automated cell counter (Biorad).

**MTS [(3-(4,5-dimethylthiazol-2-yl)-5-(3-carboxymethoxyphenyl) - 2-(4-sulfophenyl)-2H-tetrazolium)] Assay**

The viability of cells treated with DDD86481 was measured using the CellTiter 96 AQueous Non-Radioactive cell proliferation assay (MTS) (Cory et al., 1991) from Promega according to manufacturer's instructions.

**Immunohistochemistry**

B cell lymphoma tumors were fixed in formalin and embedded in paraffin and cut on a microtome to desired thickness (~5 microns or μm) and affixed onto a Superfrost® plus positively charged slide (Fisher Scientific). Before staining the slides containing tumor tissues were deparaffinized by dipping in xylene 3 times for 10 min each, a series of washes in ethanol [20 dips in 100% ethanol (repeat 4 times), 20 dips in 80% ethanol, and 20 dips in 50% ethanol] and a final wash in running cold water for 10 min.

For antigen retrieval, slides were loaded in a slide holder and placed in a Nordicware® microwave pressure cooker and 800 mL of 10 mM citrate buffer pH 6.0 was added to it. The pressure cooker was tightly closed, placed in a microwave and microwaved on high for 20 min. Next, slides were washed in cold running water for 10 min. Peroxidase blocking was performed by soaking slides in 3% $H_2O_2$ in methanol for 10 min and washing with warm running water for 10 min before washing in PBS for 3 min.

Next, excess PBS was removed and a hydrophobic circle was drawn around the sample with a PAP pen (Sigma-Aldrich). Rabbit anti-NMT1 (Proteintech) or rabbit anti-NMT2 (Origene) were diluted with Dako antibody diluent buffer (Dako, Agilent Technologies) at 1:50 dilution and added with a Pasteur pipette (~400 □L per slide) and incubated in a humidity chamber overnight at 4oC. Subsequently, slides were washed in PBS twice for 5 min each and ~4 drops of DAKO EnVision+System-HRP labeled polymer (anti-Rabbit) (Dako, Agilent Technologies) was added to each slide and incubated at room temperature for 30 min. Slides were washed again in PBS twice for 5 min each, 4 drops of Liquid DAB (diaminobenzidine) + substrate chromogen (prepared according to manufacturer's instructions; Dako, Agilent Technologies) was added, developed for 5 mins and rinsed under running cold water for 10 min.

The slides were then soaked in 1% CuSO4 for 5 min, rinsed briefly with running cold water, counter stained with haematoxylin for 60 sec and rinsed with running cold water until water ran clear. Next, slides were dipped in lithium carbonate 3 times and rinsed with running tap water briefly. Slides were then dehydrated in a series of steps; 20 dips in 50% ethanol, 20 dips in 80% ethanol and 20 dips in 100% ethanol (repeat 4 times) and finally in xylene (3 times for 10 min each). Coverslips were then added to the slides and microscopy of the tumor samples were performed using a Nikon eclipse 80i microscop. Images were created using a QImaging scientific camera (Qimaging).

**Example 1**

Figure 1 depicts the analysis of NMT1 and NMT2 expression in normal cells and various B cell lymphomas

and T cell leukemias. This figure shows the near complete absence of expression of NMT2 in B Lymphoma cell lines (BL-2, Ramos), which express only NMT1 in comparison to normal B cells (EBV transformed human B lymphocytes, L0) and human leukemic T cell lines (Jurkat, MOLT-4, CEM).

While not wishing to be bound by theory, those cells which express only one NMT isozyme, for example Burkitt's lymphoma cells which shows the near complete absence of NMT2, are likely to have altered myristoylated protein profiles.

A sample which has a reduced amount myristoylated protein in a sample (optionally as compared to a control) is indicative of an NMT deficient sample, or NMT deficient cancer. Such an NMT deficient cancer is suitable to treatment with an inhibitor or NMT1.

**Example 2**

Figure 2 depicts the sensitivity of various B cell lymphomas and T cell leukemias to the NMT inhibitors tris-dibenzylideneacetone-dipalladium (Tris-DBA). Various B and T cells were incubated for 24h with increasing concentration of Tris DBA. Cell viability was measured using trypan blue exclusion method and adjusted to 100% for control. Cell survival curves measured by trypan blue exclusion show that B cell lymphomas are more sensitive to the NMT inhibitor tris-dibenzylideneacetone-dipalladium (Tris-DBA).

**Example 3**

Figure 3 depicts the inhibition of N-myristoyltransferase (NMT) by tris-dib enzylideneacetone-dipalladium (Tri s-DBA).

NMT activity was assayed using a peptide myristoylation assay with purified recombinant NMT1 and NMT2. NMT activity was calculated from the amount of radiolabeled myristoylpeptide produced and detected on phosphocellulose paper (adapted from King et al. 1991, Anal Biochem.).

This figure shows that tris-dibenzylideneacetone-dipalladium (Tris-DBA) inhibits NMT *in vitro* using purified recombinant NMTs enzymes.

**Example 4**

Figure 4 depicts the results of immunoblotts in which lymphoma cell lines were probed with antibodies against NMT 1 (Panel A) and NMT 2 (Panel B). The legend of Figure 4 corresponds as follows: IM9: B lymphoblast; BL2: Burkitt's lymphoma; CEM: T cell leukemia; Karpas 299: T cell lymphoma; Sup-M2: ALCL; UCONN ALCL (ALCL: Anaplastic large-cell lymphoma); DAUDI: Burkitt's lymphoma; Ramos: Burkitt's lymphoma BJAB: Burkitt's lymphoma; HD-MYZ: Hodgkin lymphoma; KM-H2: Hodgkin lymphoma; L428: Hodgkin lymphoma; Jurkat: T cell leukemia.

**Example 5**

Figure 5 depicts the effectiveness of NMT inhibitors on Burkitt's Lymphoma cell line Ramos in comparison to immortalized normal B lymphocytic cell line (IM9) after 48 hours, at different concentrations.

**Example 6**

In this example, transfection of Ramos B lymphoma cells (which, as shown herein, expresses NMT1) with pcDNA3.1-V5-NMT2 increased survival to TrisDBA (5 ug/ml) 2.5 fold vs control cells transfected with empty plasmid vector. In Figure 6, 20 X $10^6$ Ramos B lymphoma cells were transfected with 32$\mu$g of DNA (pcDNA3.1-V5-empty or pcDNA3.1-V5-NMT2) using the Neon Transfection System (Invitrogen) following the recommended protocol for Ramos cell line (1,350 Volt, 30 ms). Transfected cells were centrifuged 5 minutes at 1200rpm to remove dead cells and cellular debris. Cells in the supernatant were allowed to recover for 6 hours in complete RPMI. After a PBS wash, cells were resuspended and grown in RPMI containing TrisDBA (5ug/ml) for 24hours then counted using the trypan blue exclusion method (Panel A). Cells were lysed and western blotting (ECL) was performed to confirm expression NMT2 with antibodies against NMT2, and GAPDH for loading control (Panel B).

**Example 7**

In this example, qRT-PCR was performed with Taqman NMT1 and NMT2 probes using an 18S probe as an internal control. The difference in the number of cycle times ($\Delta$ct) was calculated by subtracting the cycle time (ct) at which we see an exponential increase in the expression of the 18S internal control for each cell type from the NMT cycle time, again at a point where exponential increase of the signal is seen. As shown in Table 1, below, the ratio of NMT2

to NMT1 expression is decreased (up to 60 fold) in B lymphoma cell lines. While not wishing to be bound by theory, these results may suggest that that a reduction in mRNA encoding for NMT2 may be responsible for the reduction of NMT 2 protein levels assessed by Western blotting.

Table 1 Analysis of NMT mRNA expression by qRT-PCR

| | | mRNA sequence | Δct (ctNMT-ct18S) | NMT mRNA expression normalized to 18S | NMT1/NMT2 |
|---|---|---|---|---|---|
| Immortalized Normal B cell line | IM9 | NMT1 | 1.25 | 0.42 | 3.5 |
| | | NMT2 | 3.12 | 0.12 | |
| | L0 | NMT1 | 4.02 | 0.06 | 0.5 |
| | | NMT2 | 3.06 | 0.12 | |
| B cell lymphoma cell line | Ramos | NMT1 | -1.21 | 2.31 | 25.6 |
| | | NMT2 | 3.42 | 0.09 | |
| | BL2 | NMT1 | -0.088 | 1.06 | 53 |
| | | NMT2 | 5.83 | 0.02 | |

**Example 8**

In this example, in Figure 7, differences in the NMT2 protein levels present in various lymphocytic cell lines and solid lymphoma tumors are shown. (A) Levels of NMT1 and NMT2 are assessed by western blotting in various types of human solid lymphoma (Diffuse Large B Cell Lymphoma (DLBCL) and Follicular Lymhoma (FL) tumor lysates. NMTs were detected by western blotting using the same antibody concentrations for both panels shown: rabbit anti-NMT1 (1:2500), mouse monoclonal anti-NMT2 (1:2500). It is shown that numerous tumour samples of each type fall under the average of NMT2 expression level (0.392 +/_ 0.30).

In Panel A, it is shown that Burkitt's lymphoma cell lines BL-2, Daudi, Ramos and BJAB are devoid of NMT2.

In Panel B, it is show that 4 of 5 DLBCL and 1 of 6 FL human tumor lysates have marked reduction in NMT2 .

**Example 9**

In this example, in Figure 8 Panels A and B, the differences in the NMT2 protein levels present in various solid lymphoma tumors were determined by immuno-histochemistry:

B cell lymphoma tumors were fixed in formalin and embedded in paraffin and cut on a microtome to desired thickness (~5 microns or μm) and affixed onto a Superfrost® plus positively charged slide (Fisher Scientific). Before staining the slides containing tumor tissues were deparaffinized by dipping in xylene 3 times for 10 min each, a series of washes in ethanol [20 dips in 100% ethanol (repeat 4 times), 20 dips in80% ethanol, and 20 dips in 50% ethanol] and a final wash in running cold water for 10 min.

For antigen retrieval, slides were loaded in a slide holder and placed in a Nordicware® microwave pressure cooker and 800 mL of 10 mM citrate buffer pH 6.0 was added to it. The pressure cooker was tightly closed, placed in a microwave and microwaved on high for 20 min. Next, slides were washed in cold running water for 10 min. Peroxidase blocking was performed by soaking slides in 3% H2O2 in methanol for 10 min and washing with warm running water for 10 min before washing in PBS for 3 min.

Next, excess PBS was removed and a hydrophobic circle was drawn around the sample with a PAP pen (Sigma-Aldrich). Rabbit anti-NMT1 (Proteintech) or rabbit anti-NMT2 (Origene) were diluted with Dako antibody diluent buffer (Dako, Agilent Technologies) at 1:50 dilution and added with a Pasteur pipette (~400 μL per slide) and incubated in a humidity chamber overnight at 4oC. Subsequently, slides were washed in PBS twice for 5 min each and ~4 drops of DAKO EnVision+System-HRP labeled polymer (anti-Rabbit) (Dako, Agilent Technologies) was added to each slide and incubated at room temperature for 30 min. Slides were washed again in PBS twice for 5 min each, 4 drops of Liquid DAB (diaminobenzidine) + substrate chromogen (prepared according to manufacturer's instructions; Dako, Agilent Technologies) was added, developed for 5 mins and rinsed under running cold water for 10 min.

The slides were then soaked in 1% CuSO4 for 5 min, rinsed briefly with running cold water, counter stained with haematoxylin for 60 sec and rinsed with running cold water until water ran clear. Next, slides were dipped in lithium carbonate 3 times and rinsed with running tap water briefly. Slides were then dehydrated in a series of steps; 20 dips

in 50% ethanol, 20 dips in 80% ethanol and 20 dips in 100% ethanol (repeat 4 times) and finally in xylene (3 times for 10 min each). Coverslips were then added to the slides and microscopy of the tumor samples were performed using a Nikon eclipse 80i microscop. Images were created using a QImaging scientific camera (Qimaging).

NMT Immunohistochemical staining of normal lymph nodes, Burkitt's lymphoma (BL) and diffuse large B cell lymphoma (DLBCL or LCL). A) NMT1 staining: Both normal lymph nodes, and each of three independent cases of untreated Burkitt lymphoma (BL1-3) and DLBCL (LCL1-3) stain uniformly and strongly positive (brown peroxidase reaction product color [shown as dark grey]) for NMT1 protein. No differences were observed. The negative control was obtained by omitting the primary antibody. Upper row: N1, N2 normal lymph nodes, Neg negative control. Middle row: three Burkitt lymphoma (BL1-3) cases. Lower row: three DLBCL (LCL1-3) cases. Both normal lymph nodes and lymphoma show strong stains. B) NMT2 staining: Both normal lymph nodes stain uniformly and strongly positive (Brown color , shown as dark grey) for NMT2 protein. In marked contrast, each of three independent cases of untreated Burkitt lymphoma and DLBCL show only very weak staining for NMT2 (shown as light blue or light grey). The negative control was obtained by omitting the primary antibody. Upper row: N1, N2 normal lymph node, Neg negative control. Middle row: three Burkitt's lymphoma (BL1-3); Lower row: three DLBCL(LCL1-3).

Figure 8 Panel C and Panel D demonstrate the expression of NMT1 is not significantly increased in cell lines that lack NMT2 expression. In Panel C the $\log_2$(micro-array fluorescence intensity NMT) for NMT1 and NMT2 is plotted for all the cell lines of the CCLE database. In Panel D the $\log_2$(micro-array fluorescence intensity NMT) for NMT1 and NMT2 is plotted for the 100 cell lines of the CCLE database with the lowest NMT2 expression level.

Figure 8 Panel E depicts an example in which NMT2 determinations were quantitated using light microscopic computer assisted densitometry measurements. Visually, these numbers related well to the strength of staining of the malignant lymphocytes. The cut points chosen were strong vs no/weak staining.

**Example 10**

In this example, in Figure 9, residual viability of various B lymphocytic cell lines treated with DDD85646 for 72 hours is shown (Figure 9A). Curves in figure 9A were plotted according to a 4 parameter equation as described in Leatherbarrow, R.J. (2009) GraFit Version 6, Erithacus Software Ltd., Horley, U.K. for the GraFit analysis using the equation:

$$y = \frac{Range}{1+\left(\frac{x}{\mathrm{IC}_{50}}\right)^{s}} + \mathrm{Background} ,$$

where Range is the fitted uninhibited value minus the Background, and s is a slope factor. The equation assumes that y falls with increasing x. A and B. Combined plots (n=4).

In Figure 9, Panel A, increasing concentrations of DDD85646 were used to treat BL cell lines (BL-2 and Ramos) along with the relevant controls [IM9 ("normal" B lymphocyte) and KMH2 (HL cell line) that expresses both NMTs]. A Trypan blue assay was used to measure the cytotoxity of the DDD85646 at 24, 48 and 72 hours. Although data were collected at 24h and 48h time points (data not shown), the most noticeable effect of DDD85646 was observed at the 72h time point.

Survival rates were decreased in the B-lymphoma cell lines used (Ramos: DDD85646 $EC_{50}$=0.37+/- 0.05 μM and BL2: DDD85646 $EC_{50}$=0.43+/-0.2 μM) in a DDD85646 concentration dependent manner. The survival rates of the control cell lines, IM-9 (DDD85646 $EC_{50}$=7.08+/-2.32 μM) and KMH2 (DDD85646 EC50=29.6+/-10.6 μM) were higher. The $EC_{50}$ data are summarized as follows.

**Table 2**

| Cell type | EC50 (μM) | NMT1 | NMT2 | Type of cell |
|---|---|---|---|---|
| B1-2 | 0.43 +/- 0.20 | yes | No | Burkitt's lymphoma |
| Ramos | 0.37 +/- 0.05 | yes | No | Burkitt's lymphoma |
| IM-9 | 7.08 +/- 2.32 | yes | yes | Immortalized B cell |
| KMH2 | 29.6 +/- 10.6 | yes | yes | Non-Hodgkin Lymphoma |

DDD85646 exhibited a low $EC_{50}$ (the concentration that kills 50% of cancer cells) in BL cells, and exhibited a higher $EC_{50}$ in HL (KMH2) cells. Thus, DDD85646 exhibited a high selective killing index for cancer cells. As used herein, we define selective killing index as the ratio the $EC_{50}$ of "normal" immortalized B cell /$EC_{50}$ of malignant cells. The

selective killing index for DDD85646 was 16.4 and 19.1 for BL-2 and Ramos cells, respectively.

In Figure 9, Panel B, DDD86481 was used to treat BL cells and control cell lines [IM9 ("normal" B lymphocyte) and KMH2 (HL cell line) that expresses both NMTs]. Cell viability was measured using the MTS assay at 48h and 72h, although we did not observe a significant change to cell viability at 48h (data not shown), we found that the survival rate of the BL cell lines tested decreased significantly at the 72 h time point (Ramos: DDD86481 $EC_{50}$=42 nM and BL2: DDD86481 $EC_{50}$=58 nM) in a DDD86481 concentration dependent manner. The survival rates of the control cell lines, IM-9 (DDD86481 EC50=2.2 μM) and KMH2 (DDD86481 EC50=12.6 μM), were higher. The selective killing index calculated for DDD86481 was 37.9 and 52.3 for BL-2 and Ramos cells, respectively. The $EC_{50}$ data are summarized as follows.

**Table 3**

| **Cell type** | **EC50 (μM)** | **NMT1** | **NMT2** | **Type of cell** |
|---|---|---|---|---|
| B1-2 | 0.058 | yes | No | Burkitt's lymphoma |
| Ramos | 0.042 | yes | No | Burkitt's lymphoma |
| IM-9 | 2.2 | yes | yes | Immortalized B cell |
| KMH2 | 12.6 | yes | yes | Non-Hodgkin Lymphoma |

**[0313]** [00210][00209] The following Table, presenting the cell type description and EC50's for DDD85646 and DDD864841, B lymphoma cells (BL-2 and Ramos) expressing only one NMT (NMT1) are ~15-72 times more sensitive to DDD85646 or DDD86481 than immortalized "normal" B cells or non-Hodgkin Lymphoma cell lines expressing both NMTs.

**Table 4**

| Cell type | DDD85646 EC50(μM) | DDD86481 EC50(μM) | NMT1 | NMT2 | type |
|---|---|---|---|---|---|
| B1-2 | 0.43 +/-0.20 | 0.058 | Yes | No | Burkitt's lymphoma |
| Ramos | 0.37 +/-0.05 | 0.042 | Yes | No | Burkitt's lymphoma |
| IM-9 | 7.08 +/-2.32 | 2.2 | Yes | yes | Immortalized B cell |
| KMH2 | 29.6 +/-10.6 | 12.6 | yes | yes | Non-Hodgkin Lymphoma |

The Following Table provides a summary of pharmacologic $EC_{50}$'s and $EC_{90}$'s data for DDD86481 in Burkitt lymphoma cell lines (BL-2 and Ramos) and "normal" immortalized B cells (Data calculated from Fig. 9B).

The selectivity indexes show the ratios of the $EC_{50}$'s and $EC_{90}$'s, which indicate that DDD86481 preferentially kills cancer cells by a factor >300 fold (since the concentration required to kill the normal B cell line is that much higher).

**Table 5**

| Cell Line | DDD86481 $EC_{50}$ (μM) | Selectivity Index $EC_{50}$ normal cell/ $EC_{50}$ cancer cell | DDD86481 $EC_{90}$ (μM) | Selectivity Index $EC_{90}$ normal cell/ $EC_{90}$ cancer cell |
|---|---|---|---|---|
| IM9 "Normal" Immortalized B cells | 2.2 | N/A | 133.5 | N/A |
| BL-2 Burkitt Lymphoma | 0.058 | 37.9 | 0.21 | 635.7 |
| Ramos Burkitt Lymphoma | 0.042 | 52.4 | 0.4 | 333.7 |

In Figure 9C, DDD73226, was used to treat BL cell lines (BL-2 and Ramos) along with the relevant controls [IM9 ("normal" B lymphocyte) and KMH2 (HL cell line) that expresses both NMTs)]. The cytotoxity of DDD73226 was measured using a trypan blue exclusion assay at 72h. No significant changes to cell viability were observed at 24 and 48h (data not shown). Also, there was no significant change to the viability of IM9, KMH2 and Ramos cells when cells were treated with DDD73226 at concentrations as high as 100 μM at the 72h time point. There was, however, a slight decrease in cell viability observed in BL2 cells treated with 100 μM DDD73226 at 72 h, when compared to other cells. While not wishing to be bound by theory, this may be because in addition to having severely decreased NMT2 levels,

BL2 also has lower NMT1 levels when compared to Ramos and other cell lines, and therefore may be more susceptible to the action of even less potent NMT inhibitors.

In Figure 9D (Panels i and ii), a time-course experiment is presented wherein IM-9 and BL2 cells were labeled with 100 μM ω-alkynyl-myristate for 1h after treatment with DDD86481 (0, 1 and 10 μM) for 0, 1 or 4h. Protein samples were reacted with azido-biotin using click chemistry and visualized by western blotting with NeutrAvidin™-HRP The inhibitory action of DDD86481 was rapid, with nearly complete inhibition of myristoylation in the cells (both IM-9 and BL2) treated with 1 μM DDD86481 after one hour of treatment.

In Figure 9 E, the minimal dose of DDD86481 required to inhibit myristoylation in IM-9, BL2 and Ramos cell lines, was determined. Cells were treated with DDD86481 at 0, 10, 50, 100, 500 and 1000 nM concentrations for 2h with metabolic cell labeling with alkynyl-myristate in the last 1h of treatment. DDD86481 inhibited myristoylation in a concentration dependent manner in all cell lines tested. BL-2 and Ramos cell lines were more sensitive to the inhibitory action of DDD86481, as a decrease in the incorporation of the alkyne-myristate label into the myristoylated proteins was apparent starting at 50 nM for both BL cell lines when compared to IM-9 where incorporation of alkyne-myristate label decreased starting at the 100 nM concentration.

**Table 6** shows preliminary pre-clinical characterization of DDD85646 or DDD86481

| [] = Range | | **DDD00085646** | **DDD00086481** |
|---|---|---|---|
| Structure | | 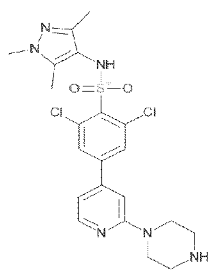 | 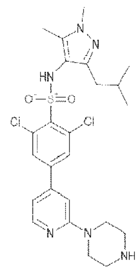 |
| Human NMT | | 4nM | < 1 nM |
| Cli | Mouse (mL/min/g) | 0.6 | <0.5 |
| | Rat (mL/min/g) | 0.5 | 1.0 |
| | Human (mL/min/g) | 1.2 | 0.7 |
| CYP450 inhibition | | 2C19 (19% at 1uM) | ND |
| Caco-2 Papp (A:B +{verapamil]) | | 43 [50] nm/sec | ND |
| Fu plasma (mouse/human) | | 0.110/0.176 | 0.067 (mouse) |
| hERG (patch clamp) | | 27.7 | ND |
| Mouse IV | Clb(mL/min/kg) | 5 [3-7] | 3 [2-3] |
| | Vss (L/kg) | 0.6 [0.4-0.7] | 0.4 [0.3-0.4] |
| | $T_{1/2}$ (hours) | 1.3 [1.3-1.4] | 1.5 [1.0-2.1] |
| Mouse PO 10mg/kg | Cmax (ng/mL) | 2686 [2122-3755] | 11201 [6986-13416] |
| | Tmax (hours) | 0.25 [0.25-2] | 2 |
| | $T_{1/2}$ (hours) | 1.2 [1.0-1.4] | 5.7 [2.7-8] |
| | F(%) | 20 [11-32] | 93 [51-100] |
| B:B Ratio | | 0.08 | 0.04 |
| Fu Brain | | 0.068 | ND |
| Pgp | | YES (Efflux ratio = 5.4 Caco-2: >6 rat) | ND |

**Example 11**

In this example, in Figure 10, the sensitivity of various immortalized normal L0 B lymphocytes (obtained from Dr. Riabowol, U. of Calgary), malignant B lymphoma cells (Ramos and BL2), and, T cell leukemia (CEM) is shown to the NMT inhibitor TrisDBA for 24 hours (A) Residual viability of various cell lines treated with TrisDBA for 24 hours (n=9) (B) Effect of TrisDBA on N-myristoyltransferase activity in COS7 cells transiently expressing NMT1 or NMT2 incubated for 24h with TrisDBA. In vitro the IC50 for TrisDBA is approximately 2 μM for NMT1 and 5 μM for NMT2. The Burkitt

lymphoma cell lines Ramos and BL-2 are more sensitive to the NMT inhibitor TrisDBA.

**Example 12**

In this example, in Figure 11, NMT expression levels in the 967 cancer cell lines encyclopedia (CCLE) database was determined. Panel A) The CCLE database was queried for NMT1 and NMT2 expression and respective NMT levels were plotted. NMT2 shows a large range of expression (~3-11) compared to NMT1 (~6-9). Panel B) Box and Whisker plots of cancer cell lines from several cancer subtypes are plotted and compared to the plot of all 967 cancer cell lines. Student T-tests were performed comparing each group to all tumours. ** denotes P values < 0.001. Panel C) The CCLE database was queried for the 50 cell lines that expressed the lowest NMT2 levels and shown in a table format. Tumour cell lines derived from various Haematopoietic and Lymphoid tissues represent 76% (38 of 50) of the lowest NMT2 expressing cell lines and are highlighted in various colours.

**Example 13**

In this example, in Figure 12, NMTs are cleaved during apoptosis but remain active. Panel A and B. The lysates of Jurkat cells induced to undergo anti-Fas or staurosporine mediated apoptosis were probed with anti-NMT1 and anti-NMT2 by western blotting and show a time dependent cleavage of both enzymes. Western blotting was performed on the same samples using antibodies against PAK2 and GAPDH. Panel C and D. Incorporation of ω-alkynyl-myristate in Jurkat cells induced to undergo anti-Fas or staurosporine mediated apoptosis illustrates a change in myristoylation profiles as cells are starting to die and suggest that although the NMTs are cleaved they are still active. Jurkat cells were metabolically labelled with 25μM-alkynyl-myristate after induction of apoptosis with anti-FAS (100ng/ml) and cycloheximide (5 μg/ ml) (C) or staurosporine (2.5μM) and cycloheximide (5 μg/ ml) (D). Protein samples were reacted with azido-biotin using click chemistry and visualized by western blotting with NeutrAvidinTM-HRP. Prior to assessment of label incorporation by western blotting membranes were incubated in 0.1 M KOH (B and D). Panel (E) At the various indicated times, NMT activity was assayed using ct-Bid N-terminal decapeptide and [$^3$H]-myristoyl-CoA. While both NMT enzymes are cleaved both remain catalytically active and only NMT1 exhibits a significant loss of activity. (F) Reconstitution of NMT1 cleavage by caspases-8 and -3, and, NMT2 cleavage by caspase-3 in vitro (Coomassie stained gel). Purified His-NMT1 and His-NMT2 (5 ug of each) was incubated with active Caspase 3 and 8 (500ng of each) for 1 hour at 37°C. A general caspase inhibitor (z-VAD-fmk) was added to stop the reaction from proceeding further at the end of the incubation. The samples were immediately boiled with 5x sample loading buffer with Beta-Mercaptoethanol and loaded on a 10% Acrylamide gel and transferred on to a PVDF membrane. The membrane was then stained with Coomassie blue to visualize proteins. The cleaved fragments, which were sent for Edman degradation are boxed and allowed the identification of caspase cleavage sites in NMT1 after D72 and NMT2 after D25 and D67.

**Example 14**

In this example, in Figure 13, purification of recombinant GST- and His6-tagged hNMT1 and hNMT2 is shown. Panel (A) GST-NMT1 and (B) GST-NMT2 purification on glutathione agarose, (C) His6-NMT1 and (D) His6-NMT2 purification by Ni-chelating chromatography and Resource S ion exchange chromatography. In Figure 13, Panels C and D, the lanes are as follows. Panel (C) GelA: Purification of NMT1: 1) All Blue Marker, 2) Ni-NTA column pool, 3) Gel filtration pool, 4) FT of Resource S, 5) Fraction 8 (f8), 6) f16, 7) f18, 8) f19, 9) f20, 10) f21, 11) f22, 12) f41, 13) f42, 14) f66, 15) f67. Panel (D) Gel B: Purification of NMT2: 1) All Blue Marker, 2) Ni-NTA column pool, 3) Gel filtration pool, 4) FT of Resource S, 5) Fraction 21 (f21), 6) f28, 7) f38, 8) f39, 9) f40, 10) f41, 11) f42, 12) f44.

**Example 15**

In this example, in Figure 14, comparison of enzyme activities between purified recombinant full-length His6-NMT1 and recombinant "caspase-truncated" ct-$His_6$-NMT1 is shown. Full-length $His_6$-NMT1 (aa 73-496). NMT activity was assayed using a peptide myristoylation assay adapted from King et al. 1999, Anal Biochem. Experiments in duplicate.

**Example 16**

In this example, in Figure 15, comparison of the EC50 and IC50 of various NMT inhibitors and different cell lines is shown. The graph shows the correlation between the biochemical potency of 8 NMT inhibitors in the biochemical enzymatic assay (IC50) and their potency in an alamar blue cell viability assay using 7 cell lines (EC50). Regression analysis was conducted on each cell line and gave a mean R2 value of 0.9 (range0.82-0.96). This high level of correlation between the various IC50 and EC50 is strong evidence that the activity of the molecules in the cell viability assay is due

to their inhibitory activity at NMT. Note that 2 pairs of molecules have similar potencies in the biochemical assay.

**Example 17**

In this example, in Figure 16, DDD86481 induction of apoptosis in BL cells is shown.

"Normal" immortalized B lymphocytes (IM9), KMH2 (Hodgkin's lymphoma) and BL (BL2 and Ramos) cells were incubated with increasing concentrations (nM) of DDD86481 and monitored the cleavage of poly-ADP-ribose polymerase-1 (PARP-1) and caspase-3 after the 72h time point. Western blotting was performed on cell lysates to monitor the cleavage or PARP-1 and caspase-3 as well as the presence of GAPDH as loading control (composite gels). Both PARP-1 and caspase-3 cleavage occurred in a dose-dependent fashion when BL cell lines were treated with the inhibitor, indicating that these cells undergo apoptosis. No PARP-1 or caspase-3 cleavage was observed in the "normal" IM9 cells treated with the inhibitor. PARP-1 cleavage in KMH2 (HL) cells was observed at higher concentrations, although it was minimal when compared to PARP-1 cleavage observed in the BL cells. Thus, we found that BL cells are more inclined to undergo apoptosis than "normal" B cells when treated with DDD86481.

**Example 18**

In this example, in Figure 17, there is provided an estimation of a threshold for the loss of NMT2 in human tumors. In Table 7, the expression level of NMT2 mRNA is shown as $\log_2$ (micro-array fluorescence intensity NMT2) for the indicated cell lines of the CCLE data set where available.

**Table 7**

| Cell Line | NMT2 mRNA expression levels* |
|---|---|
| IM-9 (immortalized B Lymphocytes) | N/A |
| KMH2 (Hodgkin's lymphoma) | N/A |
| BL-2 (Burkitt lymphoma) | 3.96 |
| Daudi (Burkitt lymphoma) | N/A |
| Ramos (Burkitt lymphoma) | N/A |
| DB (DLBCL) | 3.94 |
| Pfeiffer | 4.78 |
| (DLBCL) | |
| Toledo (DLBCL) | 5.64 |

In Figure 17, Panel A, the indicated lymphoid cell lines were probed for the presence of NMT2 by western blotting. In Figure 17, Panel B, by comparing Panel A and Table 7, we demonstrate at a $\log_2$(micro-array fluorescence intensity NMT2) = 5.64 observed in Toledo cells, there is no NMT2 detectable by western blotting. Although the actual threshold for the loss of NMT2 expression might be higher than 5.64, we are, in this example, using this number as a threshold to establish the approximate prevalence of the loss of NMT2 in human population (Panel B). The data indicate that the loss of NMT2 is not only prevalent in Burkitt and Diffuse Large B Cell Lymphomas but also in a large variety of other tumor types (see also Figure 11C for a list of 50 cell line expressing the least NMT2 and table for the prevalence of the loss of NMT2 in various cancer types).

Accordingly, there is provided herein a method of identifying those NMT2-deficitent cancers suitable for treatment with one or more NMT1 inhibitors. In one example, a patient sample with mRNA levels or concentration below about a threshold value, indicates said patient is a good candidate for treatment with an NMT1 inhibitor. In some examples, an mRNA level below about the threshold is referred to as low expression. In one example, a patient sample with mRNA levels or concentration above about a threshold value, indicates that said patient is a poor candidate for treatment with an NMT1 inhibitor. In some examples, an mRNA level above about the threshold is referred to as high expression.

It will be appreciated that in addition to, or instead of, the threshold value established in respect of Toledo cells, alternate sources may serve as a suitable threshold values. In some example, the source or control used to obtain a threshold value is the same cell type as the cancer being tested for. In some examples, the source or control threshold values are stored or found in a database.

**Example 19**

In this example, in Figure 18, the use of a desthiobiotin-PEG-azide probe to pull-down post-translationally ω-alkynyl-myristoylated proteins in leukemic Jurkat T cells using streptavidin-sepharose beads is shown.

Desthiobiotin (shown in I) is an analogue of biotin that reversibly bind to avidin and avidin-like proteins. We designed and ordered the synthesis of desthiobiotin-PEG-azide (I.) and biotin-PEG-azide (II.).

In (B) Jurkat T cells were grown in the presence of ω-alkynyl-myristate and induced to undergo anti-Fas mediated apoptosis in the presence of cycloheximide. Following cell lysis, lysates were reacted with desthiobiotin-PEG-azide using click chemistry or not, mixed with neutravidin-sepharose beads, washed and eluted with 10mM biotin. Panel B (i) shows that desthiobiotinylated-post-translationally myristoylated protein can be pulled down and recovered efficiently from neutravidin beads lanes 5,6,7 and B (iii). In panel B (ii), a control in which the desthiobiotin-PEG-azide was omitted from the click reaction is shown. In that case, very few proteins bound to the neutravidin-beads and were eluted (only a faint band can be seen at 75kDa). These results indicate the development of a method to enable a proteomic analyses and assess the cellular contents of co- and post-translationally myristoylated proteins or myristoylomes.

**Example 20**

In this Example, Figure 19, depicts scaled-up use of a desthiobiotin-PEG-azide probe to pull-down post-translationally ω-alkynyl-myristoylated proteins in leukemic Jurkat T cells using streptavidin-magnetic beads. Jurkat Cells were induced to undergo apoptosis with 2.5 μM of staurosporine for 3 h then labelled with 100 μM myristate (C14) (Panel A) or alkynyl-myristate (Alk C14) (Panel B) both conjugated to BSA for 1 h at 37°C. Cells were collected, lysed and reacted with azido-desthiobiotin using click chemistry. Desthiobiotin-alkynyl-myristoylated proteins were bound to streptavidin magnetic beads, washed and eluted with 50 mM Biotin with 2% SDS at 37°C for 15 min. Elution 1 (B panel) contains the majority of desthiobiotin-myristoylated proteins while little are found in the elution 1 from cells labelled with myristate (A panel). Exposure time: 5 seconds.

**Example 21**

In this example, in Figure 20, myristoylation profiles of "normal" immortalized B cells (IM9) and BL cells (BL2 and Ramos) labeled with alkynyl-myristate are shown. Cells were metabolically labelled with 100 μM alkynyl-myristate for 1 hour prior to harvesting. Protein samples were reacted with azido-biotin using click chemistry, 25 μg of protein from each cell lysate separated by SDS-PAGE and visualized by western blotting using NeutrAvidinTM-HRP. Arrows indicate biotinylated-alkynyl-myristoylated proteins, which are present in "normal" immortalized B lymphocyte IM9, but were not seen in BL cells (BL2 and Ramos).

**Example 22**

In this example, in Figure 21, time and dose dependent cytotoxicity graphs from the combination of DDD86481 and doxorubixin are shown. B lymphocytic cell lines IM-9 (A) and BL-2 (B) were treated with increasing concentrations of DDD86481 (0, 0.001, 0.01, 0.1, 1.0, 10 and 100μM) at 0 (Blue), 17 (Red) and 86 nM (Green) hydroxydoxorubicin for 24, 48 or 72 hours (top to bottom). The MTS assays demonstrate cell kill induced by concentrations of doxorubicin and DDD86481 that individually are much less toxic; this effect is consistent with a synergistic interaction. This is characterized by a 6 fold lowering of the $EC_{50}$'s when both compounds are used together in comparison to the $EC_{50}$'s for DDD86481 used alone at 24 and 48 hour time points.

**Example 23**

In this example, in Table 8, below, there is provided an evaluation of the approximate prevalence of the loss of NMT2 in multiple human cancers in North America. We established a minimal cut-off value of 5.64 for the $\log_2$(microarray fluorescence intensity) that corresponds to cells with no NMT2 (Toledo, see Example 18) and found that 114 of 967 cell lines fall under this threshold (~12%). North American incidence rates for each tumor type, number of patients that indicate benefit from an NMT inhibitor therapy (incidence X % under the threshold) and rationale for the medical needs of selected cancers are also shown.

# TABLE 8

| Cancer Type | Percentage of cell lines below NMT2 threshold | Canada Incidence | US Incidence | Applicable North American market for therapeutic | Rationale for priority investigation |
|---|---|---|---|---|---|
| B cell Lymphoma | 88% | 6,630 | 59,280 | 58,000 | High residual unmet medical need |
| Burkitt's lymphoma | 82% | 130 | 1,050 | 970 | High residual unmet medical need |
| Diffuse Large B Cell Lymphoma | 70% | 2,730 | 24,400 | 18,990 | High residual unmet medical need |
| Acute Myeloid Leukemia | 41% | 1,130 | 14,590 | 6,450 | High residual unmet medical need |
| Myeloma | 36% | 2,500 | 22,350 | 8,950 | High incidence, residual unmet medical need |
| Ovarian Clear Cell Carcinoma | 29% | 550 | 22,240 | 6,610 | |
| Transitional Cell Carcinoma (Ureter and bladder cancer) | 21% | 7,090 | 67,960 | 15,760 | No molecularly targeted therapies available |
| Chronic Myelogenous Leukemia (CML) | 20% | 537 | 5,920 | 1,290 | |
| Lymphoma-CLL | 20% | 2,090 | 15,680 | 3,550 | |
| Small Cell Lung Carcinoma | 13% | 3,830 | 34,270 | 4,950 | No molecularly targeted therapies available |
| Breast Carcinoma | 12% | 2,400 | 234,580 | 28,440 | High incidence |
| Colorectal Adenocarcinoma | 12% | 22,700 | 142,700 | 19,850 | High incidence |
| Pancreas Adenocarcinoma | 9% | 4,700 | 45,220 | 4,490 | Very high unmet medical need and without molecularly target therapies |
| Ovarian Carcinoma | 9% | 2,600 | 22,240 | 2,240 | |
| Non-Small Cell Lung Carcinoma | 8% | 21,670 | 193,900 | 17,250 | Common with high residual unmet medical need |
| Osteosarcoma | 8% | 350 | 800 | 90 | |
| Melanoma | 7% | 6,000 | 76,690 | 5,790 | |
| Gastric Adenocarcinoma | 5% | 3,300 | 21,600 | 1,250 | |
| Endometrial Adenocarcinoma | 5% | 5,600 | 49,560 | 2,760 | |
| Esophageal Squamous Carcinoma | 5% | 2,000 | 16,190 | 910 | |
| Total | | 98,537 | 1,071,220 | 208,590 | |

## Example 24

In this example, in Figure 22, immunoblotting was conducted with cells incubated with DMSO, Staurosporine, $\alpha$ FAS, or carrier alone. The resulting immunoblotts were probed with anti-NMT1 antibody, anti-NMT2 antibody, anti-PARP-1 antibody, and anti-GAPDH antibody. These data include at the NMT is cleaved upon induction of apoptosis.

In particular, in this example, in Figure 22A, the role(s) of NMTs in living and dying cells was investigated. Jurkat T cells induced to undergo programmed cell death with anti-Fas or STS were analyzed for their content in NMTs. Jurkat T cells were treated with DMSO, staurosporine (2.5 $\mu$M) or anti-Fas (300 ng/ml) with cycloheximide (5 $\mu$g/mL) and samples were collected at 0, 2, 4, 6 and 8 h time points. Cells were lysed and the presence of NMT1, NMT2, PARP-1 and GAPDH was assessed by Western blotting using ECL. The treatment of Jurkat T cells with either anti-Fas or STS resulted in the time dependent cleavage of both NMT1 and NMT2 (Figure 22A). The cleavage of NMT1 began 2 h after the induction of cell death, while that of NMT2 was only detectable after 4 h of apoptosis.

The cleavage of NMT1 migrating at an apparent 67 kDa protein (predicted M.W. = 56.8 kDa) resulted in an apparent ~46 kDa fragment (Fig. 3.1). The cleavage of NMT2 migrating as an apparent 65 kDa protein (predicted M.W. = 56.9) produced an apparent ~55 kDa fragment at early time points, which was converted into a shorter fragment migrating at or below ~46 kDa at later time points (4 and 8 h). This shorter NMT2 fragment was seen overlapping with a non-specific protein band. However, it was more visible in the Fas-treated Jurkat cell lysates in figures 3.2 (8h) and 3.4 A (4h and 8h). Thus, the cleavages of NMT1 and NMT2 result primarily and initially in the loss of ~11 kDa and ~10kDa fragments, respectively. The reason for the discrepancies between the apparent migrations and predicted molecular weights of NMTs is not known. The timing of the cleavage of NMTs paralleled that of the apoptotic marker PARP-1 suggesting it is due to the action of caspases

NMT1 is a substrate of caspases-3 or -8, and NMT2 is a substrate of caspase-3

In the experiment of Figure 22 Panel B, to investigate whether caspases are involved in the cleavage of NMTs, we induced anti-Fas mediated apoptosis in Jurkat T cells in the presence or absence of well-characterized irreversible inhibitors of caspases-3, -8, and -9 along with a general inhibitor of caspases and analyzed the cellular contents for both NMTs. Jurkat T cells were treated with 10 $\mu$M caspase inhibitors for one hour and then treated with anti-Fas (300 ng/mL) and cycloheximide (5 $\mu$g/mL) to induce apoptosis. Samples were collected at 0, 4, and 8 h time points. Cells were lysed and the presence of NMT1, NMT2 and PARP-1 was assessed by western blotting using ECL. The blots were then stripped and reprobed with anti¬tubulin (loading control) (Composite gels).

The cleavage of NMT1 was abrogated by the general and caspase-8-specific inhibitors, whereas it was only partially blocked by the caspase-3-specific inhibitor (Figure 22B), which suggests that NMT1 is a substrate of caspases-3 or -8. In contrast, NMT2 cleavage was noticeably inhibited by all caspase inhibitors used (Figure 22B). This suggests that NMT2 is likely a substrate of caspase-3 since the inhibition of the initiator caspases-8 or -9, would in turn inhibit the

cleavage and activation of the effector caspase-3. The progression of apoptosis induced by anti-Fas was verified by western blotting with anti-PARP-1 antibody Figure 22B). The extent of PARP-1 cleavage was concomitant with and commensurate to that of NMT1 and NMT2.

**Example 25**

In this example, in Figure 23, it is shown that caspase truncated NMT2 is 3-4 times more active than full length NMT2. NMT activity of purified full length and caspase-cleaved hexahistidine(His)-NMTs was assayed using a peptide myristoylation assay. N-Myristoyltransferase activity was calculated from the amount of radiolabeled myristoylpeptide produced and detected on phosphocellulose paper (adapted from King et al. 1991, Anal Biochem.). Each NMT assay was performed in triplicates. Control used is elution 5 from the His-NMTlpurification. Caspase truncated NMT2 is 3-4 times more active than full length NMT2

**Example 26**

In this example, in Figure 24, it is shown that reduction of NMT2 levels in BL cells involves the action of histone deacetylases.

In the experiments of Figure 24, we sought to assess the possible involvement of gene silencing at the NMT2 locus. The acetylation of the ε-amino group of lysine residues on histones by histone acetylases (HATs) results in the reduction of the positive charge of histones, which relaxes the chromatin conformation and allowing transcription machinery to have better access to DNA (Barneda-Zahonero, B., and M. Parra. 2012. Histone deacetylases and cancer. Mol Oncol. 6:579-589.). Therefore, histone acetylation is typically associated with gene activation. Conversely, the removal of acetyl groups from histones by histone deacetylases (HDACs) induces chromatin condensation and results in the transcriptional repression or silencing of genes.

To test whether the reduction in mRNA NMT2 was due to chromatin silencing, we treated "normal" B cells (IM9) and malignant BL cells with suberoylanilide hydroxamic acid (SAHA) (also named vorinostat), a class I and class II histone deacetylase inhibitor, for 24 hours and monitored the levels of NMT1 and NMT2 by western blot. In particular, "Normal" B cells (IM9) and malignant BL cells (Ramos, BL2) treated with 1 μM SAHA (HDAC class I/II inhibitor) for 24 h. Cells were then lysed, subjected to SDS-PAGE and western blotting was performed with NMT1, NMT2, p21/WAF1 and GAPDH antibodies.

We found that use of SAHA increased NMT2 levels in BL cells where NMT2 levels are typically depleted, whereas NMT1 levels remained relatively unchanged. p21/WAF1, a protein binds to and inhibits the activity of cyclin dependent kinase 1 (CDK1) or CDK2 complexes, was used as a control to verify the effectiveness of SAHA, which is known to increase the p21/WAF1 gene expression in cells. All cells treated with SAHA contained increased p21/WAF1 levels. While not wishing to be bound by theory, these data suggest that a class I or class II HDAC silences the NMT2 gene by deacetylating histones and thereby compacting the NMT2 locus in BL cells.

In one example, an HDAC inhibitor is used to treat NTM2 defficient cancer.

**Example 27**

In this example, in Figure 25, it is shown that NMT2 protein levels are reduced in various BL cell lines. Lysates from various lymphocytic cell lines gathered from local investigators and analyzed for their NMT content. The results demonstrate that NMT2 protein levels were reduced in all the Burkitt lymphoma (BL2, Daudi, Ramos and BJAB) cell lines tested when compared to the "normal", immortalized lymphoblastic B cell lines, IM9, L0 and VDS.

**Example 28**

In this example, in Figure 26, it is shown that proteosomal degradation is not the cause of NMT2 depletion in BL cells. In these experiments, "Normal" immortalized B lymphocytes (IM9) and malignant B lymphoma cells [Hodgkin's lymphoma (KMH2) and BL (Ramos, BL2)] treated with 10 μM of proteosomal inhibitor (MG-132) for 5h. Western blotting was performed on cell lysates to monitor levels of NMT1, NMT2, Mcl-1, and GAPDH.

To investigate whether NMT2's degradation was increased as a result of a destabilizing mutation or the presence of an unknown factor that could destabilize NMT2 in cancer cells, we treated normal and malignant lymphocytes with the proteosomal degradation inhibitor MG-132 for 5h. Cell lysates were subjected to western blotting to analyze the presence of NMT 1, NMT2 and myeloid cell leukemia-1 protein (Mcl-1), which was used as a control to check the effectiveness of MG-132 since it is subject to proteosomal degradation. The results indicate that the NMT2 levels did not increase in BL cells upon treatment with MG-132, suggesting a destabilizing mutation or destabilizing factor present in malignant lymphocytes leading to the degradation of NMT2 is not present. The treatment of cells with MG-132 lead

to increased levels of Mcl-1 in all cell types.

**Example 29**

In this Example, in Figure 27, it is shown NMT1 is cleaved by caspase-8, but not NMT2. Jurkat T cells (A) and Jurkat T cells expressing a caspase-8 dominant negative mutant (B) were treated with DMSO or anti-Fas (150 ng/mL) and samples were collected at 0, 4 and 8 h time points. Cells were lysed and the presence of NMT1, PARP-1 and cleaved caspase-8 was assessed by western blotting using ECL. * denotes non-specific bands.

The cleavage of NMT1 was investigated in wild-type Jurkat T and Jurkat T cells expressing a caspase-8 dominant negative mutant (C8DN) (Juo et al., 1998). The expression of C8DN in apoptotic Jurkat T cells abrogated the cleavage of NMT1 when compared to levels seen in the wild-type Jurkat T cells (Figs. 27 A and B). A minimal amount of NMT2 cleavage was observed in Jurkat-C8DN cells after 8 h of apoptosis induction (Fig. 27 B). The cleavage of PARP-1 and caspase-8 was also inhibited in the Jurkat T-C8DN cells.

**Example 30**

In this Example, in Figure 28, both NMT1 and NMT2 were cleaved by caspase-3. MCF7 (A) and MCF7 expressing caspase 3 (MCF7/caspase 3) (B) were treated with DMSO or STS (2.5 μM) with cycloheximide (5 μg/mL) and samples were collected at 0, 4 and 8 h time points. Cells were lysed and the presence of NMT2, PARP-1 and cleaved caspase 3 was assessed by western blotting using ECL.

It was found that both NMT1 and NMT2 were not cleaved in apoptotic MCF-7 cells at the 8 h time point (Fig. 28 A). In contrast, both enzymes were readily cleaved in apoptotic MCF-7 cells stably expressing caspase-3 (Kagawa et al., 2001) (Fig. 28 B). The cleavage of the known caspase-3/-7 substrate PARP-1 was also confirmed in both MCF-7 cell lines when apoptosis was induced (Figs. 27 A and B) (Walsh et al., 2008). Therefore, both NMTs appear to be substrates of caspase-3.

**Example 31**

In this example, in Figure 29, the caspase cleavage sites of NMT1 and NMT2 as identified by Edman degradation are shown in bold font and the positively charged lysine (K) box is highlighted on the NMT1 and NMT2 amino acid sequences (amino acids 1 to 80).

N-terminal sequencing revealed that NMT2 was cleaved at both D-25 and D-67 sites (Fig. 29). The cleavage sites for both NMT1 and NMT2 were located in the N-terminus of the enzymes and not in the C-terminal catalytic domain, which suggests that the cleaved enzymes may still be active during apoptosis.

**Example 32**

In this Example, in Figure 30, depicts confirmation of NMT cleavage sites by site-directed mutagenesis. HeLa cells transiently expressing the wild-type and mutant V5-NMT constructs were incubated with STS (2.5 μM). Cells transfected with V5-NMT1 or V5-NMT2 constructs were lysed at 4 h and 5 h time points, respectively. Western blotting was performed on the samples using V5 and α-tubulin (loading control) antibodies.

To validate the NMT cleavage sites revealed by N-terminal sequencing, we constructed N-terminally tagged V5-NMT vectors and used the Quickchange® site-directed mutagenesis kit (Stratagene) to create point mutations at the NMT cleavage sites. The engineered D72E mutation in V5-NMT1 abrogated the caspase-cleavage of V5-NMT1 in appropriately transfected HeLa cells induced to undergo apoptosis with STS for 4 h while the WT V5-NMT1 levels were severely diminished in these cells (Fig. 30). This confirmed D72 as the caspase-cleavage site in NMT1.

Because N-terminal sequencing revealed two caspase cleavage sites (D25 and D67) for NMT2 (Fig. 30), we mutated both D25 and D67 residues into glutamate (E) residues independently [V5-NMT2 (D25E), V5- NMT2 (D67E)] and together [V5-NMT2 (D25,67E)]. We observed that the cleavage of the V5-NMT2 (D25,67E) double mutant was severely abrogated in appropriately transfected apoptotic HeLa cells while the single mutants [V5-NMT2 (D25E), V5-NMT2 (D67E)] had varying effects (Fig. 30) and the wild-type V5-NMT2 was mostly cleaved after 5 h after apoptosis induction (Fig. 3.8). When comparing the integrity of the single mutants during apoptosis, we observed that V5-NMT2 (D25E) was reproducibly slightly less cleaved than V5-NMT2 (D67E) (Fig. 30), suggesting that D25 might be the primary cleavage site of NMT2, whereas D67 may be a secondary cleavage site.

**Example 33**

In this example, in Figure 31, depicts changes to the myristoylation profile as cells undergo apoptosis. Jurkat

cells were metabolically labelled with 25 μM alkynyl-myristate after induction of apoptosis with anti-Fas (150 ng/mL) and cycloheximide (5 μg/ mL). Protein samples were reacted with azido-biotin using click chemistry and visualized by western blotting with NeutrAvidinTM-HRP. Prior to the assessment of label incorporation by western blotting, membranes were incubated in 0.1 M Tris-HCl or 0.1 M KOH.

Jurkat T cells were induced to undergo apoptosis using anti-Fas and metabolically labeled for 30 min with ω-alkynyl-myristic acid prior to havesting cells at various time points (0, 1, 2, 4 and 8 h). Cycloheximide was also added to cells to inhibit protein translation as part of the apoptotic stimulus, thereby blocking co-translational myristoylation during apoptosis. Cellular lysates were reacted with azido-biotin using click chemistry and biotinylated¬myristoylated proteins were visualized by western blot analysis using NeutrAvidinTM-HRP, as described previously (Yap et al., 2010).

The myristoylation profile at time 0 h correlated with the co-translational myristoylation pattern observed previously in non-apoptotic cells (Fig. 31) (Martin et al., 2008; Yap et al., 2010). The deliberately low exposure shows only a few myristoylated proteins in the cell lysates. Treatment of the membranes with 0.1M KOH confirmed that ω-alkynyl-myristic acid is incorporated into proteins via an alkali resistant amide bond, since the alkali treatment removed the label from only a few protein bands.This alkaline treatment hydrolyzes thioester bonds found in palmitoylated proteins (Armah and Mensa-Wilmot, 1999; Zhao et al., 2000; Vilas et al., 2006). After induction of apoptosis with anti-Fas and cycloheximide for 1h, co-translational myristoylation was reduced, presumably as protein translation is inhibited by cycloheximide. This was followed by a change in the cellular content of myristoylated proteins as illustrated by the major differences in electrophoretic myristoylated protein profiles starting at 2 h post-induction of apoptosis and ongoing for the duration of the experiment (Fig. 31).

## Example 34

In this example, in Figure 32, depicts changes to NMT levels as cells undergo apoptosis. Jurkat cells were metabolically labelled with 25 μM alkynyl-myristate after induction of apoptosis with anti-Fas (150 ng/mL) and cycloheximide (5 μg/ mL). Western blotting was performed on the same samples as in Figure 31 using antibodies against NMT1, NMT2 and GAPDH. (*) denotes non-specific bands

These results shows that although NMTs are cleaved to various extents during apoptosis, myristoylation activity appears to remain in cells up to 8 h after induction of apoptosis.

## Example 35

In this example, in Figure 33, depicts induction of COS7 cells transiently expressing V5-NMT1 and V5-NMT2 to undergo apoptosis with staurosporine and cycloheximide. NMT activity was assayed using a peptide myristoylation assay and western blotting was performed on the sample using antibodies against v5 and alpha-tubulin (loading control).

Figure 34 depicts initial NMT activity in the lysates of transiently transfected COS7 cells. COS7 cells transiently expressing V5-NMT1 and V5-NMT2 were incubated with STS (2.5 μM) and cycloheximide (5 μg/mL). NMT activity was assayed using a peptide myristoylation assay as described in materials and methods. N-Myristoyltransferase activity was calculated from the amount of radiolabeled myristoylpeptide produced and detected on phosphocellulose paper (adapted from King et al. 1991, Anal Biochem.). Activity levels were normalized to NMT1 activity at t=0h. NMT1 represents the average of three independent experiments done in duplicates. NMT2 represents the average of four independent experiments done in duplicates.

Figure 35 depicts NMT activity in COS7 cells transiently expressing V5- NMT1 and V5-NMT2 incubated with staurosporine (2.5 μM) and cycloheximide (5 μg/mL). NMT activity was assayed using a peptide myristoylation assay as described under materials and methods. N¬Myristoyltransferase activity was calculated from the amount of radiolabeled myristoylpeptide produced and detected on phosphocellulose paper (adapted from King et al.1991, Anal Biochem.). NMT activity was normalized to 100% at t = 0 h for each NMT. NMT1 represents the average of three independent experiments done in duplicates. NMT2 represents the average of four independent experiments done in duplicates. Differences are denoted by (*) and show statistical significance (* < p=0.05, ** < p=0.005) when compared to the 0 h time point for both NMTs.

To assess whether cleaved NMTs were still catalytically active, we measured the V5-NMT enzymatic activity of transiently transfected cells expressing either V5-NMTs using a filter based-peptide assay (King and Sharma, 1991; Raju and Sharma, 1999) during the onset of apoptosis. COS-7 cells transiently transfected with V5-NMT1, V5-NMT2 or empty vector were induced to undergo STS/cycloheximide-mediated apoptosis and used as a source of enzyme. NMT activity was measured at different times of apoptosis using [3H]-myristoyl-CoA and myristoylatable- or non-myristoylatable (G->A) truncated Bid decapeptides were used as substrates (King and Sharma, 1991; Raju and Sharma, 1999). NMT activity was calculated from the amount of radiolabeled peptide that remained bound to the phosphocellulose paper and detected by scintillation counting.

Although the transfected COS-7 cells expressed similar levels of chimeric NMTs (Fig. 32), those expressing

V5-NMT1 showed nearly a 5-fold higher NMT activity than those expressing V5-NMT2 at t=0h (Fig. 34). The amount of intact V5 -NMTs found in cell lysates decreased over time of apoptosis induction (Fig.33) and followed a similar trend as the endogenous NMTs (Fig. 32), although nearly all of the over-expressed NMT1 was cleaved after 4h and 8h of apoptosis and all of over-expressed NMT2 after 8h (Fig. 3.33). Although greater than 90% of V5-NMT1 is cleaved (Fig. 3.33) at 4h after apoptotic induction, the NMT activity in those cells remained relatively unchanged up to 8 h after cell death was initiated. There was a slight trend towards the increase (although not significant) in NMT catalytic activity at t=2h and 4h, in the lysates of COS-7 cells over-expressing NMT1 when compared to activity at t=0h (Fig. 3.35). This suggests that cleaved V5-NMT1 is catalytically active during apoptosis when post-translational myristoylation is initiated. However, we observed a significant decrease in NMT activity (20%, $p<0.05$) at 8h after induction of apoptosis when compared to the 0h time point in the cells transfected with V5-NMT1 (Fig. 35).

The NMT activity of cells expressing V5-NMT2 was not significantly affected from 0h to 4h, until the caspase cleavage of V5-NMT2 resulted in a statistically significant ($p<0.005$) decrease (33% decrease when compared to activity to t=0h) in enzymatic activity after 8 h of apoptosis induction (Fig. 35). Interestingly, although the NMT2 levels are drastically reduced due to caspase cleavage during apoptosis (Fig. 33), 66% of NMT2 activity still remains after 8h of apoptosis induction (Fig. 33), indicating that NMT2 also plays a role in post-translational myristoylation of proteins during apoptosis

**Example 36**

In this example, in Figure 36, depicts purification of recombinant hexahistidine(His)-tagged full-length and caspase-cleaved hNMT1. Purification was performed using Ni-NTA chromatography (see materials and methods). Purified proteins were visualized by staining gels with coomassie blue gel stain. (FT: flow through, W: wash and E: eluted fractions

Figure 37 depicts purification of recombinant hexahistidine(His)-tagged full-length and caspase-cleaved hNMT2. Purification was performed using Ni-NTA chromatography (see materials and methods). Purified proteins were visualized by staining gels with coomassie blue gel stain. (FT: flow through, W: wash and E: eluted fractions).

Figure 38 depicts NMT activity of purified full length and caspase-cleaved hexahistidine(His)-NMTs assayed using a peptide myristoylation assay. N-Myristoyltransferase activity was calculated from the amount of radiolabeled myristoylpeptide produced and detected on phosphocellulose paper (adapted from King et al.1991, Anal Biochem.). Each NMT assay was performed in triplicates. Control used is elution 5 from the His-NMT1purification.

We generated His-tagged full-length (His-NMTl and His-NMT2) and caspase truncated (His-73NMT1, His-26NMT2 and His-68NMT2) human NMT vectors and used these vectors for bacterial expression and protein purification (Fig. 36 and 337). Using the filter-based peptide NMT assay (King and Sharma, 1991; Raju and Sharma, 1999), we found that both full-length and caspase cleaved truncated NMT1 and NMT2 were catalytically active when compared to the control in an in vitro setting (Fig. 37). The cleavage of NMT2 appear to enhance its activity as caspase cleaved truncated NMT2 seemed to have ~3-fold (His-26NMT2) and ~4-fold (His-68NMT2) more activity when compared to full-length NMT2 (Fig 38).

**Example 37**

In this example, in Figure 39 depicts subcellular fractionation of endogenous NMTs in HeLa cells during apoptosis. HeLa cells were treated with DMSO, STS (2.5 μM) or anti-Fas (300 ng/mL) with cycloheximide (5 μg/mL) and samples were collected at the 5 h time point and subjected to subcellular fractionation as described in materials and methods. The same volume of P100 and S100 fractions were subjected to western blotting using NMT1 and NMT2 antibodies.

Figure 40 depicts quantification of amount of NMT in different fractions after the subcellular fractionation of endogenous NMTs in HeLa cells during apoptosis. HeLa cells were treated with DMSO, STS (2.5μM) or anti-Fas (300 ng/mL) with cycloheximide (5 μg/mL) and samples were collected at the 5 h time point and subjected to sub-cellular fractionation (Fig. 39). The levels of full-length (Full) and cleaved NMT1 (A) and NMT2 (B) between the P100 (P) and S100 (S) fractions were quantified using Image J (http://rsbweb.nih.gov/ij/). Percentages shown were calculated as levels of each band over total of the two fractions (P100+S100). The graphs represents the average of three independent experiments,

Figure 41 depicts sub-cellular fractionation of HeLa cells undergoing apoptosis labelled with alkynyl-myristate. Prior to sub-cellular fractionation (Figs. 39 and 40), HeLa cells were metabolically labelled with 25 μM alkynyl-myristate after induction of apoptosis. After fractionation, protein samples were reacted with azido-biotin using click chemistry and visualized by western blotting with NeutrAvidinTM-HRP. Prior to assessment of label incorporation by western blotting membranes were incubated in 0.1 M neutral Tris-HCl (A) or 0.1 M KOH (B).

The caspase cleavage of many proteins often results in change in cellular localization (Enari et al., 1998; Zha

et al., 2000; Jakobi, 2004; Vilas et al., 2006); therefore, to delineate the localization of the cleaved NMTs during apoptosis we performed subcellular fractionation experiments (Fig. 39) in normal and apoptotic cells. We found that NMT1 was primarily found localized to the ribosomal/membrane fraction in untreated HeLa cells (63.9% in pellet) (Fig. 40). There was a discernible increase of cleaved caspase-truncated NMT1 in the cytosolic fractions of HeLa cells induced to undergo apoptosis with STS or anti-Fas together with cycloheximide (54% in cytosol in anti-Fas treated cells and 60% in cytosol in STS treated cells) (Figs. 39 and 40). Conversely, the majority of NMT2 (61.7%) localized to the cytosol prior to apoptosis induction (Figs. 39 and 40). However, the larger caspase-cleaved NMT2 fragment (~55 kDa) mainly localized to the membrane pellet (94.7% in Fas-treated and 80% in STS-treated) in apoptotic cells (Figs. 39 and 40). We did not observe the presence of the smaller NMT2 cleaved fragment (~46 kDa) during our fractionations, possibly because cells were induced to undergo apoptosis for a maximum of 5 h.

When cells were metabolically labeled with alkynyl-myristate prior to induction of apoptosis or not and subjected to subcellular fractionation, we observed a change in the myristoylation profile after the induction of apoptosis as seen in Figure 31 and found out that the post-translationally myristoylated proteins mainly localized to membranes (P100) when compared to the cytosol (S100) (Fig. 40). This suggests that the addition of a myristoyl moiety to these post-translationally myristoylated proteins appear sufficient to provide stable membrane anchoring.

**Example 38**

In this example, Figure 42 depicts Effect of 2-hydroxymyristic acid (HMA) on the induction of apoptosis. Jurkat T cells were treated with or without HMA (1 mM) and apoptosis was induced with anti-Fas (150 ng/ml) and cycloheximide (5 μg/ml). The control cells were treated with DMSO. Samples were collected at 0, 2, 4, 6 and 8 h time points. Cells were lysed and samples were separated by SDS-PAGE and immunoblotted with antibodies against PARP-1, PAK2, NMT1 and NMT2 (composite gels).

Cleavage of PARP-1 occurred 2 h sooner in cells treated with ImM HMA and anti-Fas as compared to cells treated with ImM sodium myristate and anti-Fas. A similar trend was also seen in cells exposed to HMA/STS but to a lesser extent than what was seen with anti-Fas, in the presence of HMA/STS cells which exhibited more PARP-1 and PAK2 cleavage at 2 h post incubation of apoptosis than cells treated with STS and ImM sodium myristate (Fig. 42). A similar stimulation of the cleavage of NMT1 and NMT2 was also observed. This indicates that the inhibition of NMTs accelerates the induction of apoptosis in cells. Since the inhibition of NMT potentiated the onset of apoptosis, it appears that NMTs play overall, a pro-survival role in cells.

All publications, patents and patent applications mentioned in this Specification are indicative of the level of skill those skilled in the art to which this invention pertains and are herein incorporated by reference to the same extent as if each individual publication patent, or patent application was specifically and individually indicated to be incorporated by reference.

The invention being thus described, it will be obvious that the same may be varied in many ways. Such variations are not to be regarded as a departure from the spirit and scope of the invention, and all such modification as would be obvious to one skilled in the art are intended to be included within the scope of the following claims.

## Claims

1. A product comprising the NMT1 inhibitor DDD86481 or DDD85646, and a chemotherapeutic treatment, for combined use in the treatment of a cancer deficient in NMT2 in a subject, wherein the cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Plasma Cell Myeloma, Intestinal Adenocarcinoma, Lung mixed Adenosquamous Carcinoma, Lung Small Cell Carcinoma, Lung Cancer, Oesophagus Squamous Cell Carcinoma, Bone Cancer, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, or esophageal squamous carcinoma.

2. The product of claim 1, wherein the chemotherapeutic treatment is a drug combination.

3. The product of claim 2, wherein the cancer is lymphoma and the drug combination is CHOP (cyclophosphamide, doxorubicin, vincristine, and prednisone), GAP-BOP (cyclophosphamide, doxorubicin, procarbazine, bleomycin,

vincristine, and prednisone), m-BACOD (methotrexate, bleomycin, doxorubicin, cyclophosphamide, vincristine, dexamethasone, and leucovorin), ProMACE-MOPP (prednisone, methotrexate, doxorubicin, cyclophosphamide, etoposide, leucovorin with standard MOPP), ProMACE-CytaBOM (prednisone, doxorubicin, cyclophosphamide, etoposide, cytarabine, bleomycin, vincristine, methotrexate, and leucovorin), MACOP-B (methotrexate, doxorubicin, cyclophosphamide, vincristine, prednisone, bleomycin, and leucovorin), IMVP-16 (ifosfamide, methotrexate, and etoposide), MIME (methyl-gag, ifosfamide, methotrexate, and etoposide), DHAP (dexamethasone, - 16 high dose cytarabine, and cisplatin), ESHAP (etoposide, methylprednisone, high dosage cytarabine, and cisplatin), CEFF(B) (cyclophosphamide, etoposide, procarbazine, prednisone, and bleomycin), CAMP (i.e., lomustine, mitoxantrone, cytarabine, and prednisone), VABCD (i.e., vinblastine, doxorubicin, dacarbazine, lomustine and bleomycin), ABDIC (doxorubicin, bleomycin, dacarbazine, lomustine, and prednisone), CBVD (lomustine, bleomycin, vinblastine, dexamethasone), PCVP (vinblastine, procarbazine, cyclophosphamide, and prednisone), CEP (lomustine, etoposide, and prednimustine), EVA (etoposide, vinblastine, and doxorubicin), MOPLACE (cyclophosphamide, etoposide, prednisone, methotrexate, cytarabine, and vincristine), MIME (methyl-gag, ifosfamide, methotrexate, and etoposide), MINE (mitoquazone, ifosfamide, vinorelbine, and etoposide), MTX-CHOP (methotrexate and CHOP), CEM (lomustine, etoposide, and methotrexate), CEVD (lomustine, etoposide, vindesine, and dexamethasone), CAVP (lomustine, melphalan, etoposide, and prednisone), EVAP (etoposide, vinblastine, cytarabine, and cisplatin), or EPOCH (etoposide, vincristine, doxorubicin, cyclophosphamide, and prednisone).

**4.** The product of any one of claims 1 to 3, wherein the cancer is a B cell lymphoma.

**5.** The product of any one of claims 1 to 4, wherein the B-cell lymphoma is follicular lymphoma, diffuse large B-cell lymphoma, mantle cell lymphoma, B-CLL/SLL, immunocytoma/Waldenstrom's, and MALT-type/monocytoid B cell lymphoma, Burkitt's lymphoma, follicular lymphoma, precursor B-LBL, precursor T-LBL, or anaplastic large cell lymphoma.

**6.** A product comprising the NMT1 inhibitor DDD86481 and doxorubicin, for combined use in the treatment of a cancer deficient in NMT2 in a subject, wherein the cancer is B-cell lymphoma.

**7.** The product of any one of claims 1 to 6, wherein the B-cell lymphoma is Burkitt's lymphoma.

**8.** The product of any one for claims 1 to 7, wherein the subject is a human.

**9.** A kit comprising a product comprising the NMT1 inhibitor DDD86481 or DDD85646, and a chemotherapeutic treatment, for combined use in the treatment of a cancer deficient in NMT2 in a subject, wherein the cancer is lymphoma, B cell lymphoma, follicular lymphoma, diffuse large B-cell lymphoma, mantle cell lymphoma, B-CLL/SLL, immunocytoma/Waldenstrom's, MALT-type/monocytoid B cell lymphoma, Burkitt's lymphoma, a pediatric lymphoma, anaplastic large cell lymphoma, acute myeloid leukemia, Blast Phase Chronic Myeloid Leukaemia, Plasma Cell Myeloma, Intestinal Adenocarcinoma, Lung mixed Adenosquamous Carcinoma, Lung Small Cell Carcinoma, Lung Cancer, Oesophagus Squamous Cell Carcinoma, Bone Cancer, Breast Ductal Carcinoma, Stomach Diffuse Adenocarcinoma, Thyroid Medullary Carcinoma, urinary Tract Transitional Cell Carcinoma, myeloma, ovarian clear cell carcinoma, transition cell carcinoma (ureter and bladder cancer), chronic myelogenous leukemia (CML), lymphoma-CLL, breast carcinoma, colorectal adenocarcinoma, pancreas adenocarcinoma, ovarian carcinoma, non-small cell lunch carcinoma, osteosarcoma, melanoma, gastric adenocarcinoma, endometrial adenocarcinoma, or esophageal squamous carcinoma, optionally wherein the chemotherapeutic treatment is a drug combination.

**10.** The kit of claim 9, wherein the cancer is lymphoma and the drug combination is CHOP (cyclophosphamide, doxorubicin, vincristine, and prednisone), GAP-BOP (cyclophosphamide, doxorubicin, procarbazine, bleomycin, vincristine, and prednisone), m-BACOD (methotrexate, bleomycin, doxorubicin, cyclophosphamide, vincristine, dexamethasone, and leucovorin), ProMACE-MOPP (prednisone, methotrexate, doxorubicin, cyclophosphamide, etoposide, leucovorin with standard MOPP), ProMACE-CytaBOM (prednisone, doxorubicin, cyclophosphamide, etoposide, cytarabine, bleomycin, vincristine, methotrexate, and leucovorin), MACOP-B (methotrexate, doxorubicin, cyclophosphamide, vincristine, prednisone, bleomycin, and leucovorin), IMVP-16 (ifosfamide, methotrexate, and etoposide), MIME (methyl-gag, ifosfamide, methotrexate, and etoposide), DHAP (dexamethasone, - 16 high dose cytarabine, and cisplatin), ESHAP (etoposide, methylprednisone, high dosage cytarabine, and cisplatin), CEFF(B) (cyclophosphamide, etoposide, procarbazine, prednisone, and bleomycin), CAMP (i.e., lomustine, mitoxantrone, cytarabine, and prednisone), VABCD (i.e., vinblastine, doxorubicin, dacarbazine, lomustine and bleomycin), ABDIC (doxorubicin, bleomycin, dacarbazine, lomustine, and prednisone), CBVD (lomustine, bleomycin, vinblastine, dexamethasone), PCVP (vinblastine, procarbazine, cyclophosphamide, and prednisone), CEP (lomustine, etoposide, and prednimustine), EVA (etoposide, vinblastine, and doxorubicin), MOPLACE (cyclophosphamide, etoposide, prednisone, methotrexate, cytarabine, and vincristine), MIME (methyl-gag, ifosfamide, methotrexate, and etoposide), MINE (mitoquazone, ifosfamide, vinorelbine, and etoposide), MTX-CHOP (methotrexate and CHOP), CEM (lomustine, etoposide, and methotrexate), CEVD (lomustine, etoposide, vindesine, and dexamethasone), CAVP (lomustine, melphalan, etoposide, and prednisone), EVAP (etoposide, vinblastine, cytarabine, and cisplatin), or EPOCH (etoposide, vincristine, doxorubicin, cyclophosphamide, and prednisone).

**11.** The kit of claim 9 or 10, wherein the cancer is a B cell lymphoma.

**12.** The kit of any one of claims 9 to 11, wherein the B-cell lymphoma is follicular lymphoma, diffuse large B-cell lymphoma, mantle cell lymphoma, B-CLL/SLL, immunocytoma/Waldenstrom's, and MALT-type/monocytoid B cell lymphoma, Burkitt's lymphoma, follicular lymphoma, precursor B-LBL, precursor T-LBL, or anaplastic large cell lymphoma.

**13.** A kit comprising the NMT1 inhibitor DDD86481 and doxorubicin, for combined use in the treatment of a cancer deficient in NMT2 in a subject, wherein the cancer is B-cell lymphoma.

**14.** The kit of any one of claims 9 to 13, wherein the B-cell lymphoma is Burkitt's lymphoma.

**15.** The kit of any one for claims 9 to 14, wherein the subject is a human.

Figure 1

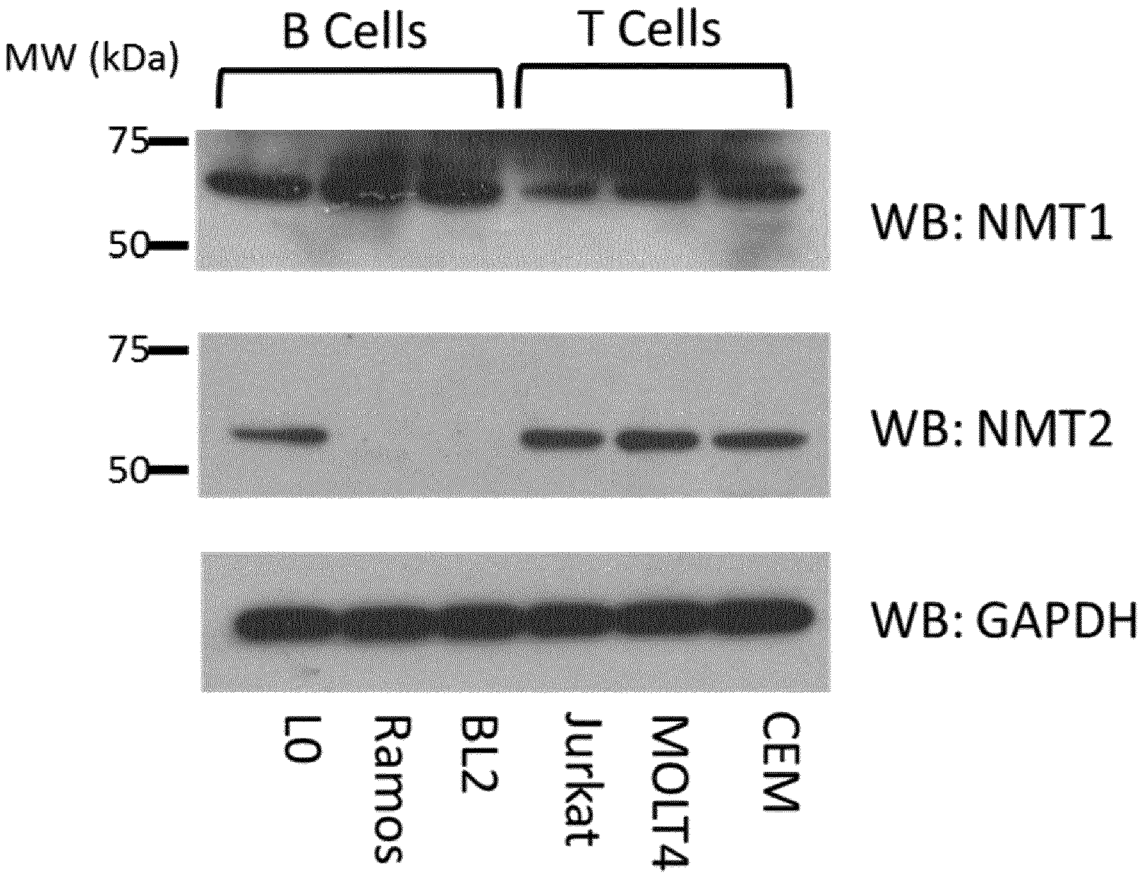
MW (kDa)
B Cells
T Cells
75
50
WB: NMT1
75
50
WB: NMT2
WB: GAPDH
L0
Ramos
BL2
Jurkat
MOLT4
CEM

Figure 2

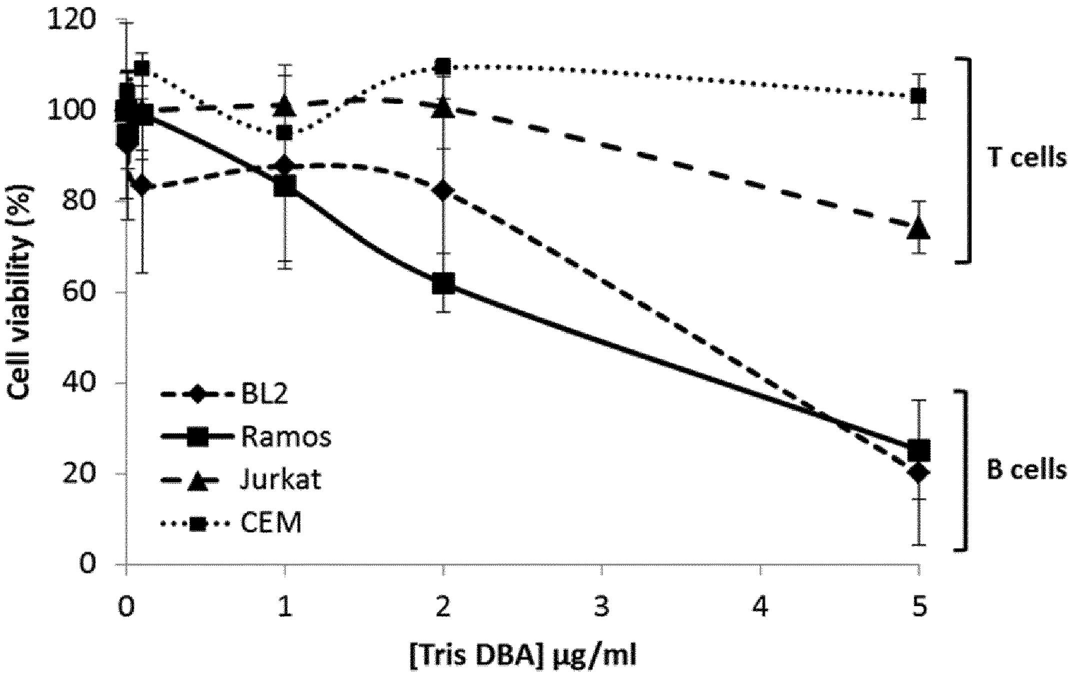
120
100
80
60
40
20
0
Cell viability (%)
0
1
2
3
4
5
[Tris DBA] µg/ml
BL2
Ramos
Jurkat
CEM
T cells
B cells

Figure 3

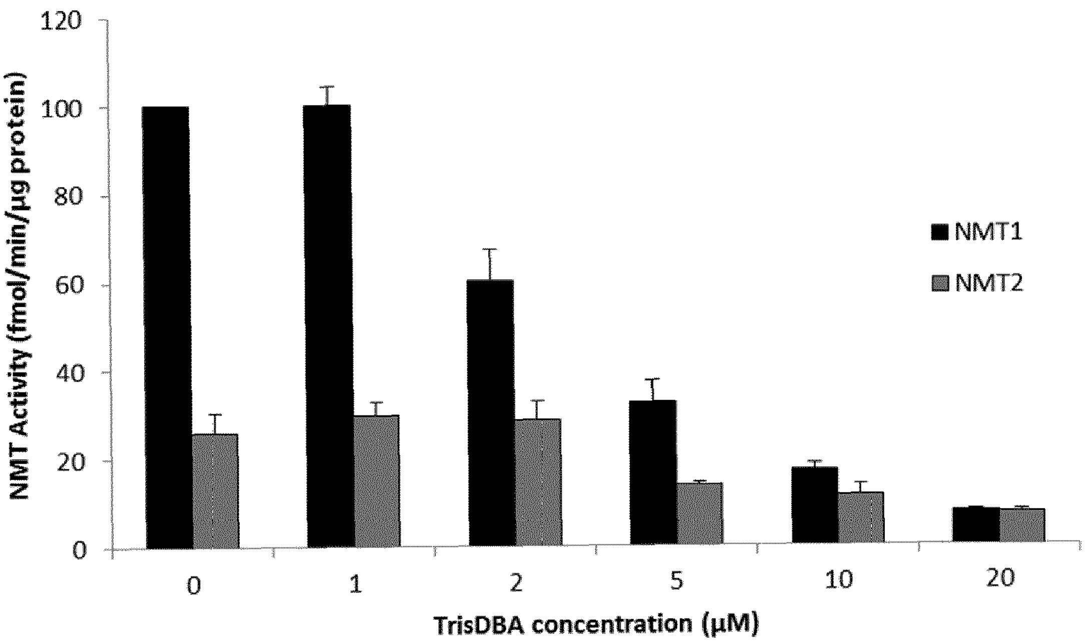
120
100
80
60
40
20
0
NMT Activity (fmol/min/µg protein)
NMT1
NMT2
0
1
2
5
10
20
TrisDBA concentration (µM)

Figure 4

A

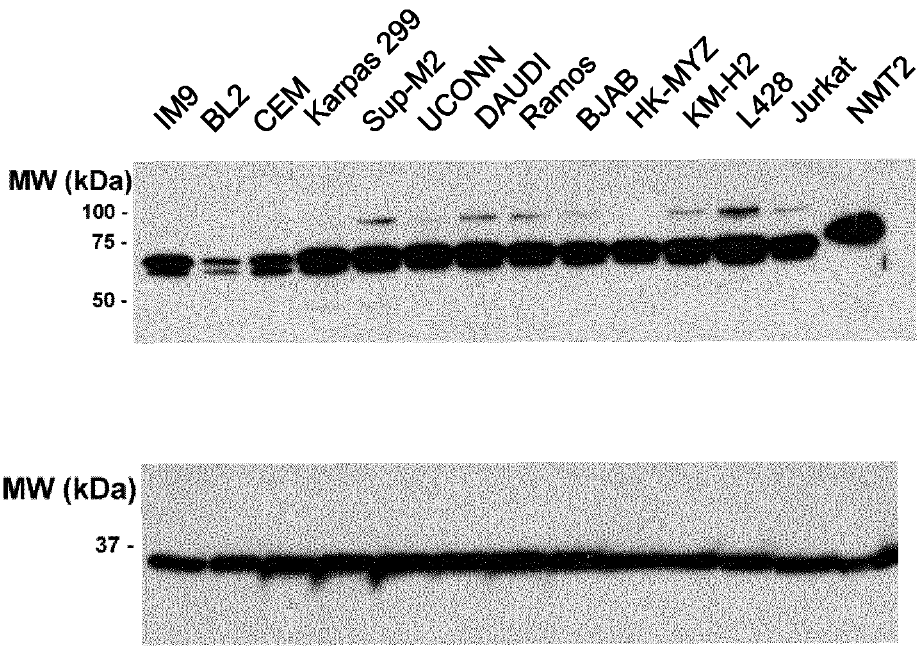


IM9: B lymphoblast

BL2: Burkitt's lymphoma

CEM: T cell leukemia

Karpas 299: T cell lymphoma

Sup-M2: ALCL

UCONN: ALCL

(ALCL: Anaplastic large-cell lymphoma)

DAUDI: Burkitt's lymphoma

Ramos: Burkitt's lymphoma

BJAB: Burkitt's lymphoma

HD-MYZ: Hodgkin lymphoma

KM-H2: Hodgkin lymphoma

L428: Hodgkin lymphoma

Jurkat: T cell leukemia

Figure 4

B

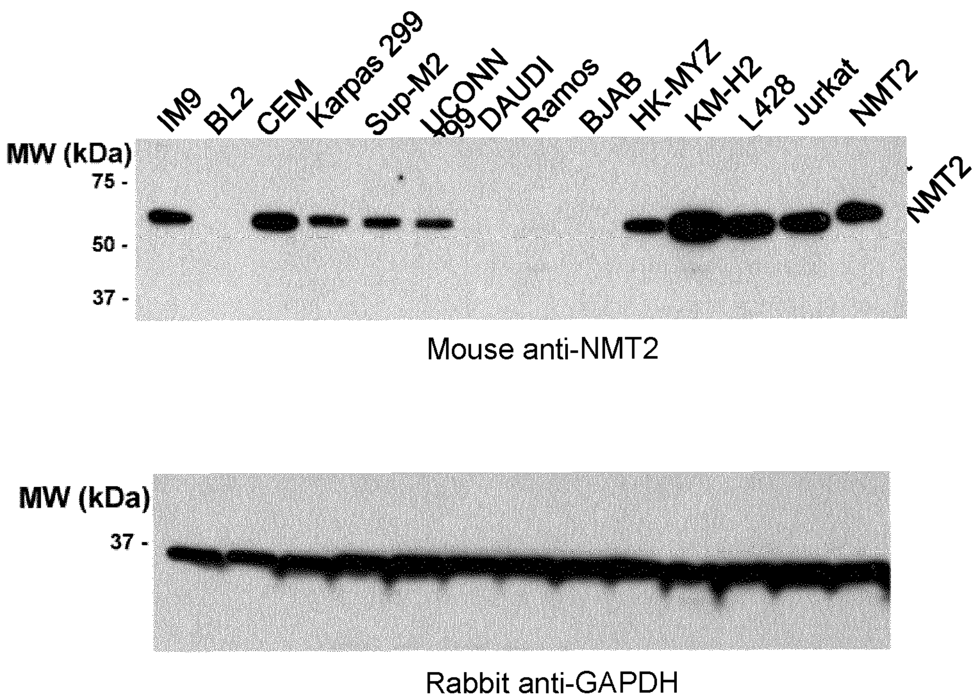


IM9: B lymphoblast
BL2: Burkitt's lymphoma
CEM: T cell leukemia
Karpas 299: T cell lymphoma
Sup-M2: ALCL
UCONN: ALCL
(ALCL: Anaplastic large-cell lymphoma)
DAUDI: Burkitt's lymphoma
Ramos: Burkitt's lymphoma
BJAB: Burkitt's lymphoma
HD-MYZ: Hodgkin lymphoma
KM-H2: Hodgkin lymphoma
L428: Hodgkin lymphoma
Jurkat: T cell leukemia

Figure 5

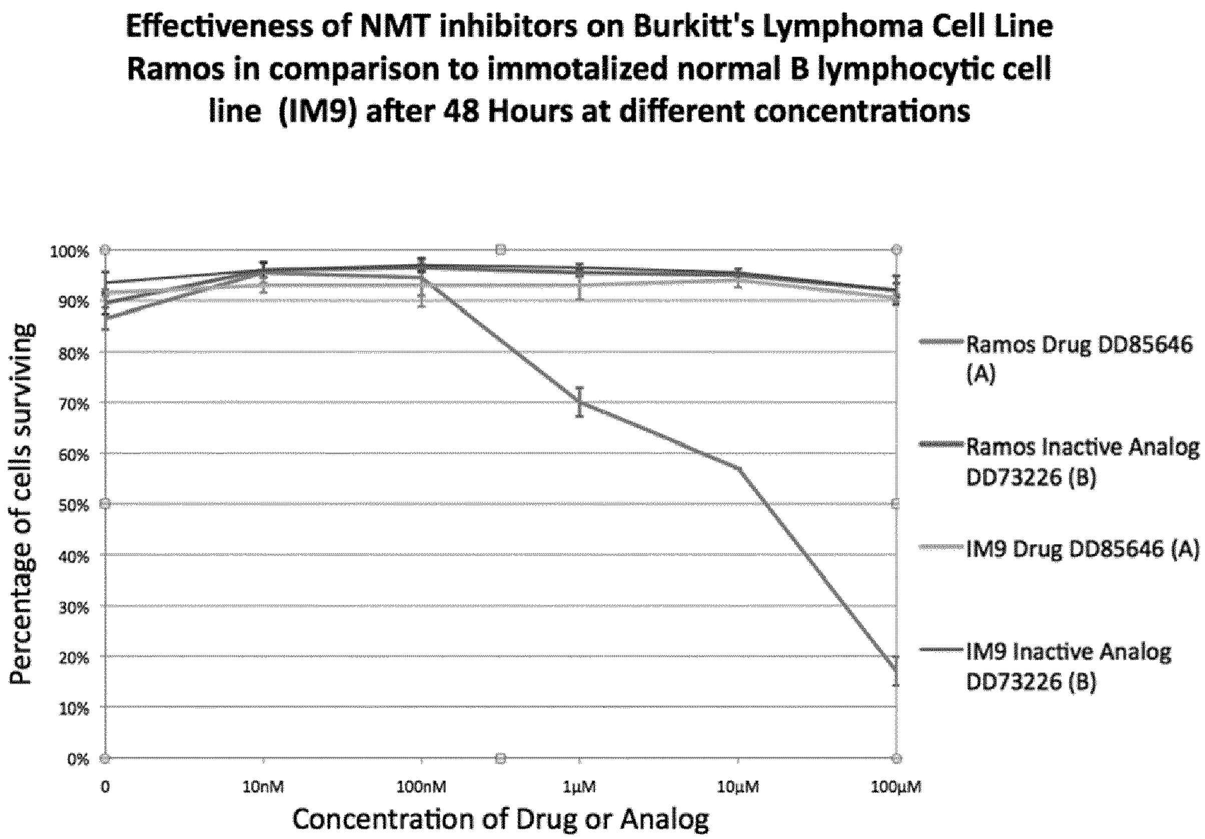
Effectiveness of NMT inhibitors on Burkitt's Lymphoma Cell Line Ramos in comparison to immotalized normal B lymphocytic cell line (IM9) after 48 Hours at different concentrations
100%
90%
80%
70%
60%
50%
40%
30%
20%
10%
0%
Percentage of cells surviving
0
10nM
100nM
1µM
10µM
100µM
Concentration of Drug or Analog
Ramos Drug DD85646 (A)
Ramos Inactive Analog DD73226 (B)
IM9 Drug DD85646 (A)
IM9 Inactive Analog DD73226 (B)

Figure 6

A

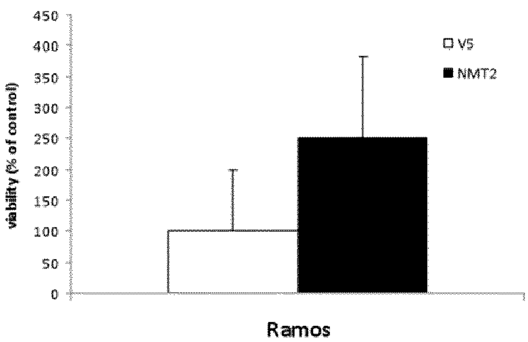
450
400
350
300
250
200
150
100
50
0
viability (% of control)
V5
NMT2
Ramos

B

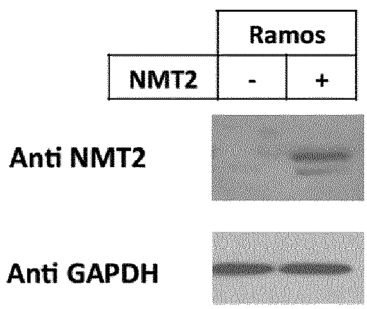
Ramos
NMT2
-
+
Anti NMT2
Anti GAPDH

Figure 7

A

B

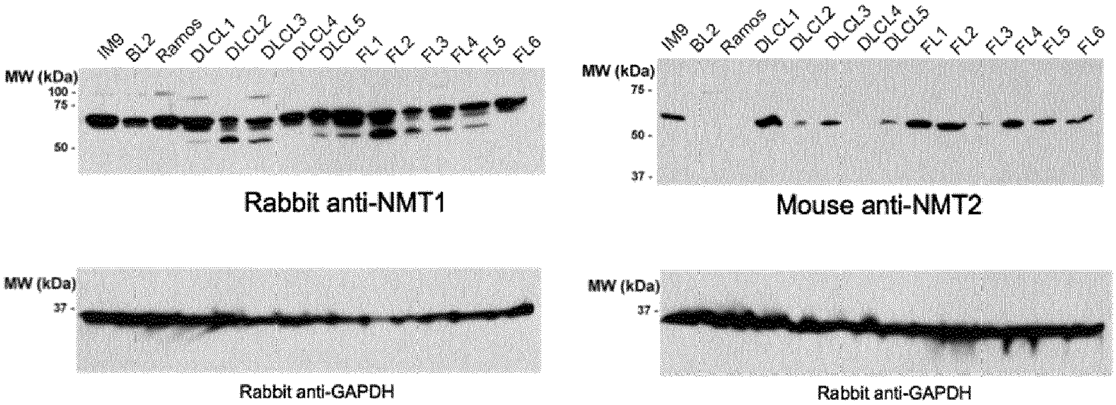


IM9: B lymphoblast
BL2: Burkitt's lymphoma
Ramos: Burkitt's lymphoma
DLCL: Diffuse large B-cell lymphoma
FL: Follicular lymphoma

Figure 8

A

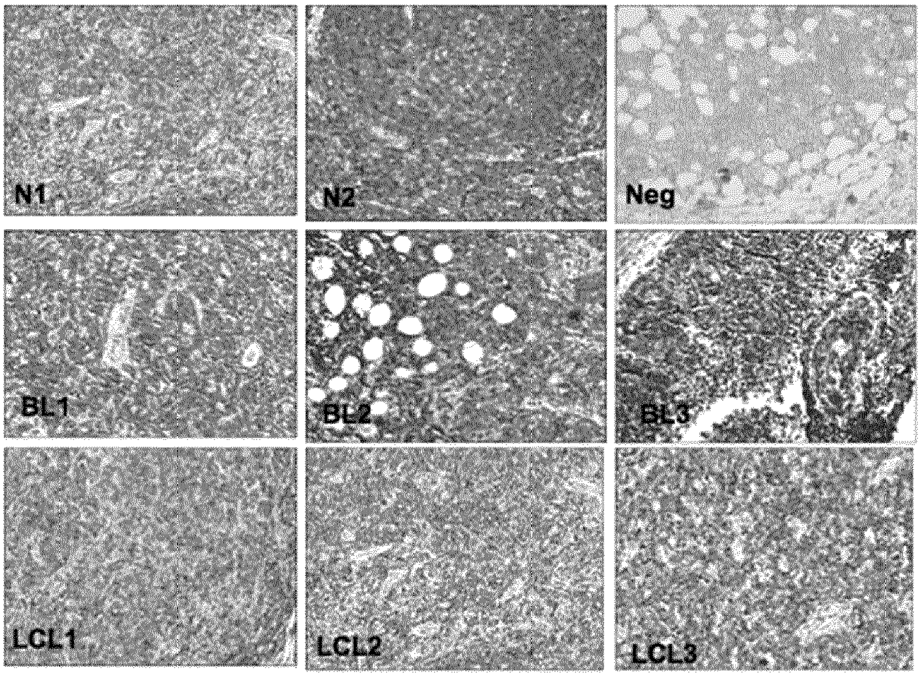


NMT-1 stain. Upper row: N1, N2 normal lymph nodes, Neg Negative control. Middle row: three Burkitt lymphoma cases (BL 1-3). Lower row: three DLBCL (LCL-1-3) cases. Both normal lymph nodes and lymphoma show strong stains. No difference observed.

Figure 8

B

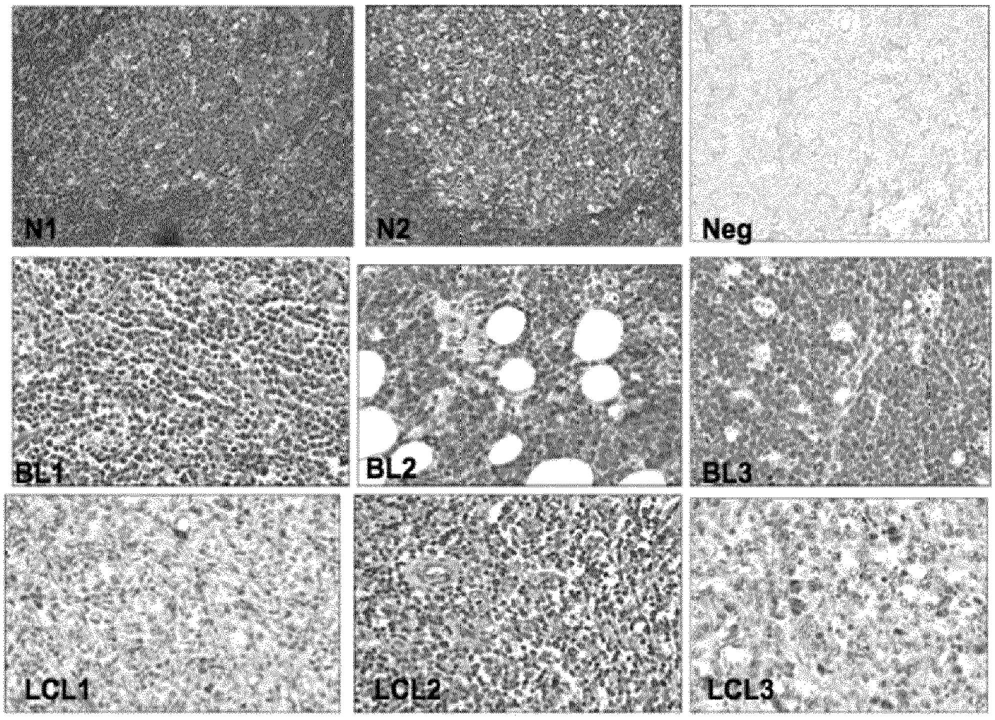


NMT-2 stain. Upper row: N1, N2 normal lymph nodes, Neg Negative control. Middle row: three Burkitt lymphoma cases (BL 1-3). Lower row: three DLBCL (LCL-1-3). The normal lymph nodes show a strong stain whereas the Burkitt lymphoma (BL) and diffuse large B cell lymphoma (LCL) show a weak stain.

Figure 8

C

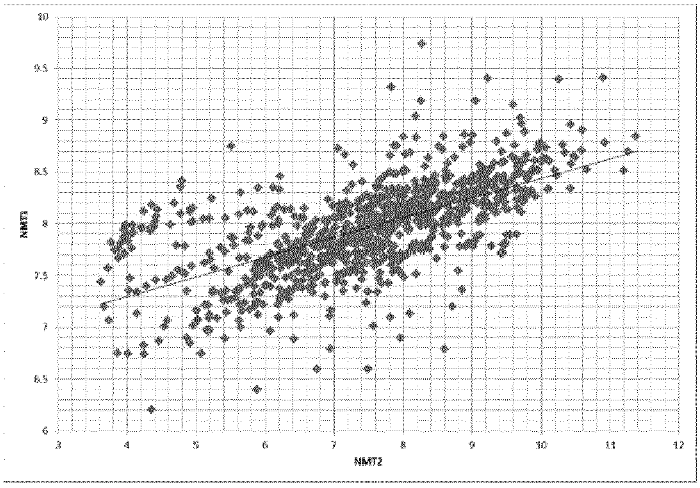


D

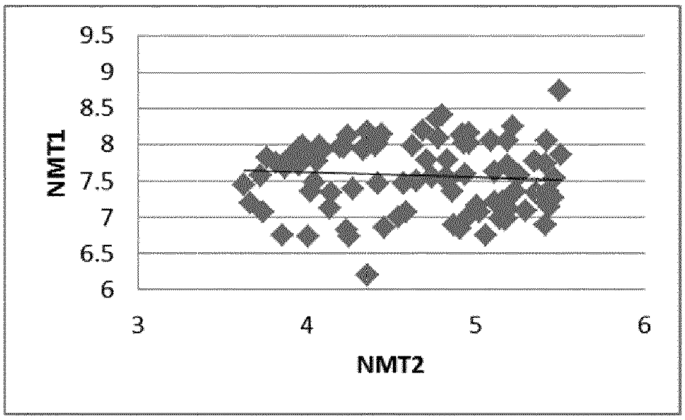

Figure 8

E

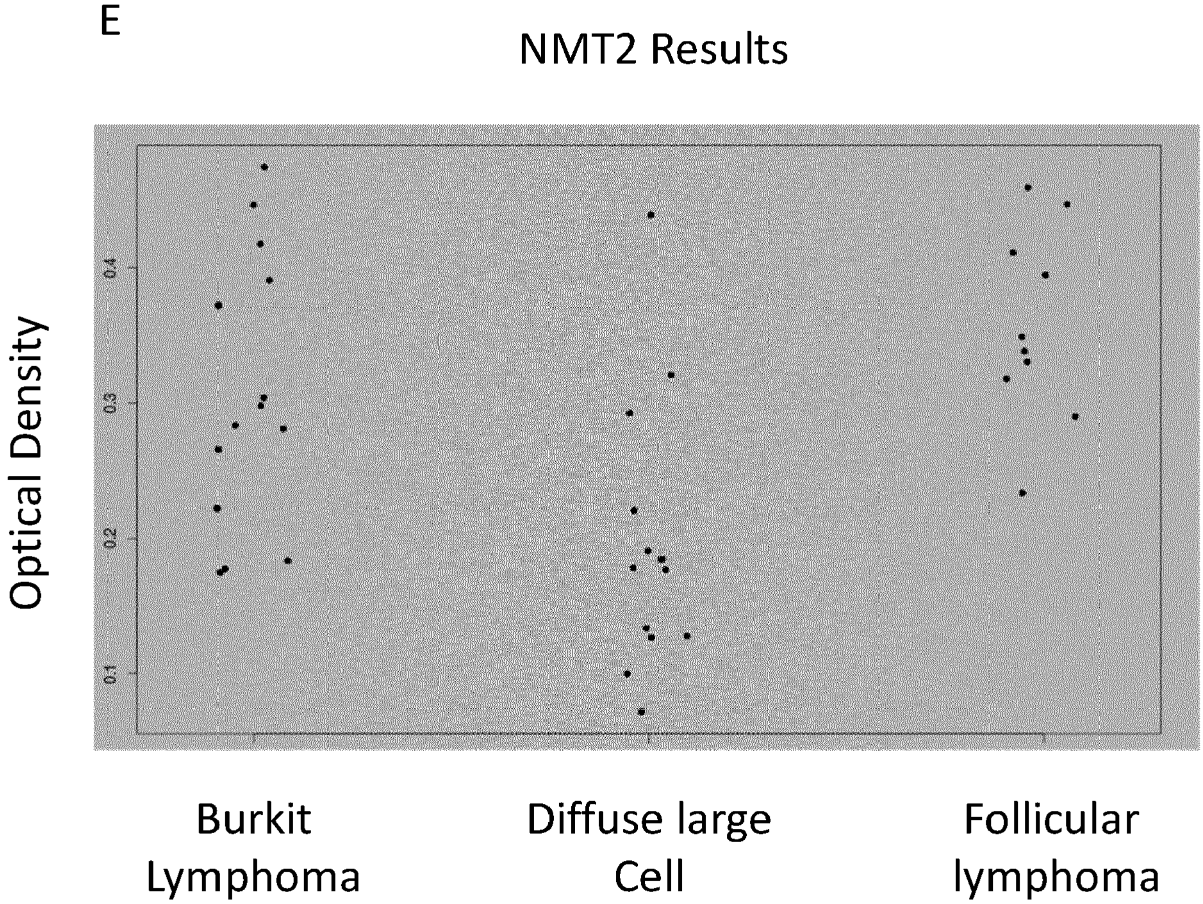
NMT2 Results
Optical Density
0.1
0.2
0.3
0.4
Burkit
Lymphoma
Diffuse large
Cell
Follicular
lymphoma

Figure 9

A

DDD85646 cytotoxicity after 72 h incubation

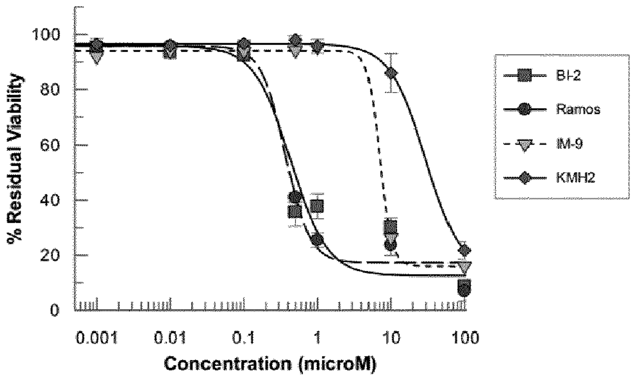


B

DDD86481 cytotoxicityafter 72 h incubation

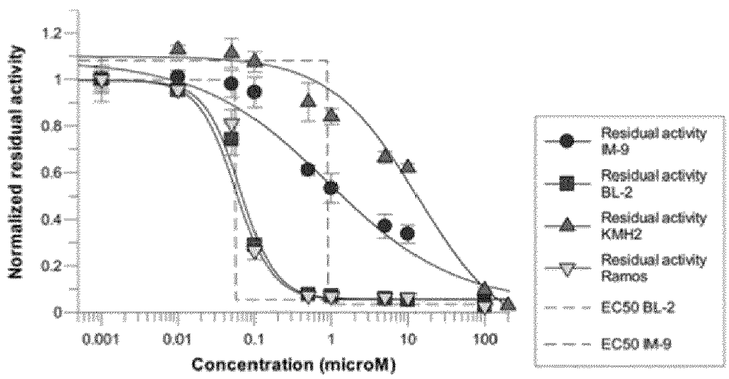

Figure 9

C

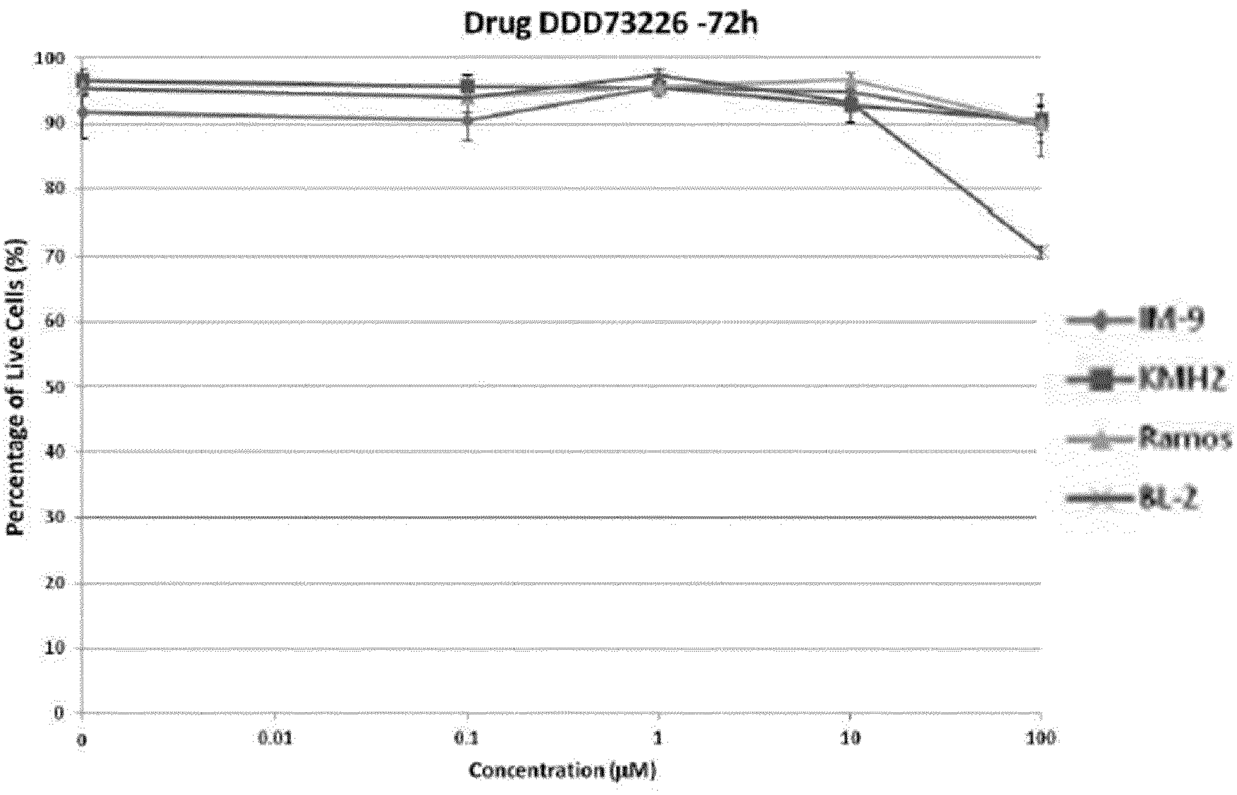
Drug DDD73226 -72h
Percentage of Live Cells (%)
100
90
80
70
60
50
40
30
20
10
0
0
0.01
0.1
1
10
100
Concentration (µM)
IM-9
KMH2
Ramos
BL-2

Figure 9

D

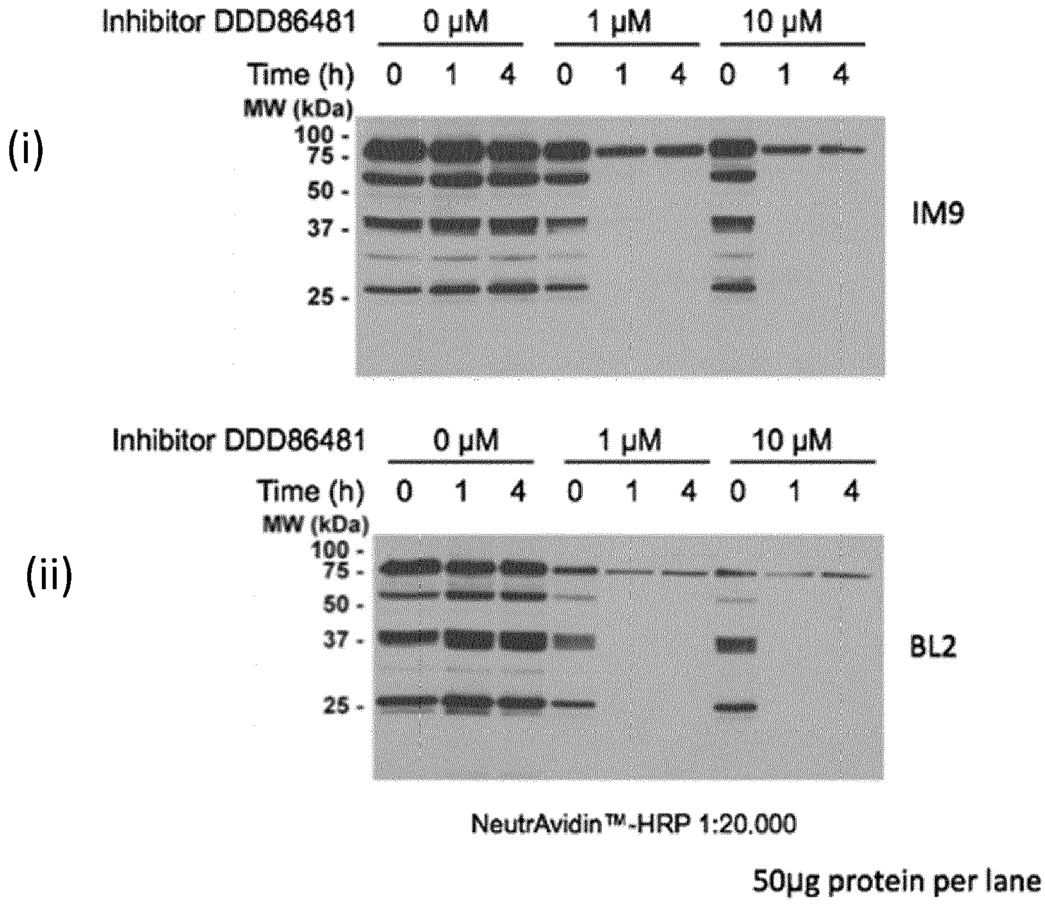
Inhibitor DDD86481
0 μM
1 μM
10 μM
Time (h) 0 1 4 0 1 4 0 1 4
MW (kDa)
100 -
75 -
50 -
37 -
25 -
(i)
IM9
Inhibitor DDD86481
0 μM
1 μM
10 μM
Time (h) 0 1 4 0 1 4 0 1 4
MW (kDa)
100 -
75 -
50 -
37 -
25 -
(ii)
BL2
NeutrAvidin™-HRP 1:20,000
50μg protein per lane

Figure 9

E

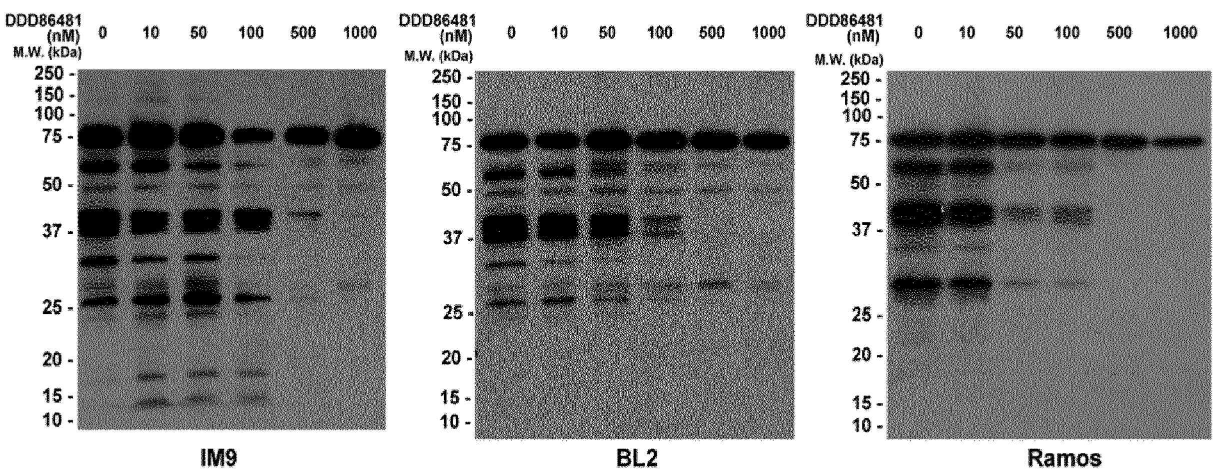


WB: Nutratvidin-HRP

Figure 10

A

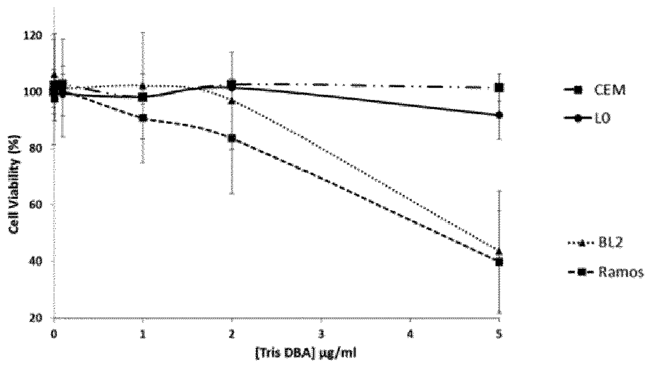
Cell Viability (%)
[Tris DBA] µg/ml
CEM
L0
BL2
Ramos

B

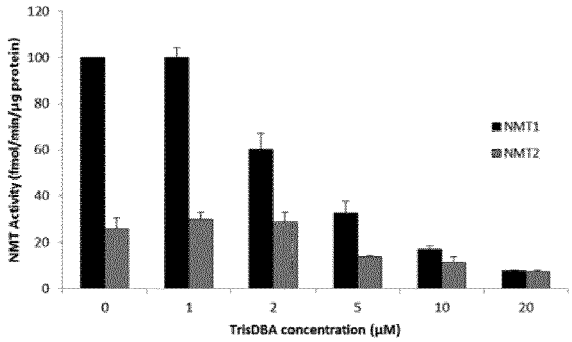
NMT Activity (fmol/min/µg protein)
TrisDBA concentration (µM)
NMT1
NMT2

Figure 11

A

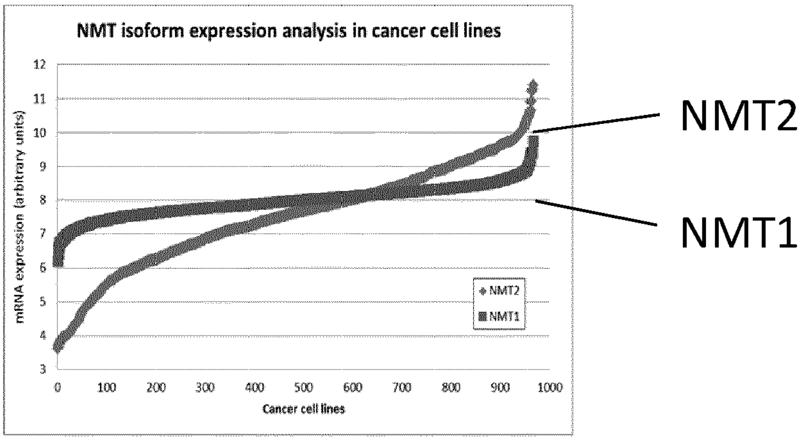
NMT isoform expression analysis in cancer cell lines
mRNA expression (arbitrary units)
Cancer cell lines
NMT2
NMT1
NMT2
NMT1

B

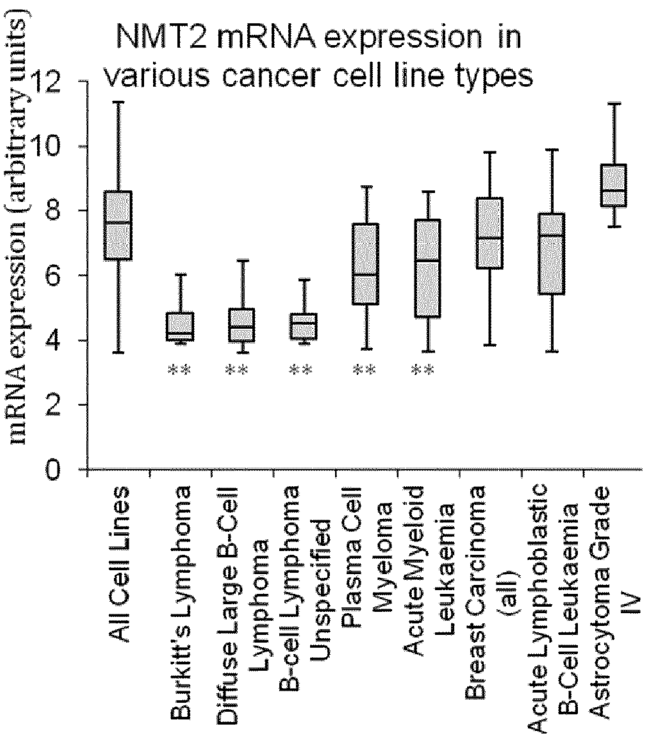
NMT2 mRNA expression in various cancer cell line types
mRNA expression (arbitrary units)
All Cell Lines
Burkitt's Lymphoma
Diffuse Large B-Cell Lymphoma
B-cell Lymphoma Unspecified
Plasma Cell Myeloma
Acute Myeloid Leukaemia
Breast Carcinoma (all)
Acute Lymphoblastic B-Cell Leukaemia
Astrocytoma Grade IV

Figure 11

C

| | Histological Subtype | Cell line | NMT2 Expression | Source |
|---|---|---|---|---|
| Haematopoietic or Lymphoid Source | Acute Myeloid Leukemia | EOL-1 | 3.6642 | DSMZ |
| | Acute Myeloid Leukemia | GDM-1 | 3.7673 | ATCC |
| | Acute Myeloid Leukemia | PL-21 | 4.0117 | ATCC |
| | Acute Myeloid Leukemia | Kasumi-6 | 4.0205 | ATCC |
| | Acute Myeloid Leukemia | MV-4-11 | 4.0953 | HSRRB |
| | Acute Myeloid Leukemia | HL-60 | 4.3381 | ATCC |
| | Acute Myeloid Leukemia | MOLM-13 | 4.3432 | ATCC |
| | Acute Myeloid Leukemia | KO52 | 4.4048 | DSMZ |
| | Acute Myeloid Leukemia | OCI-AML2 | 4.6291 | DSMZ |
| | B Cell Lymphoma Unspecified | MC116 | 3.9043 | ATCC |
| | B Cell Lymphoma Unspecified | RL | 3.9759 | ATCC |
| | B Cell Lymphoma Unspecified | JM1 | 4.0771 | DSMZ |
| | B Cell Lymphoma Unspecified | NU-DUL-1 | 4.3577 | DSMZ |
| | B Cell Lymphoma Unspecified | RI-1 | 4.6862 | ATCC |
| | Blast Phase Chronic Myeloid Leukaemia | EM-2 | 4.1421 | DSMZ |
| | Burkitt's Lymphoma | NAMALWA | 3.8721 | DSMZ |
| | Burkitt's Lymphoma | Daudi | 3.9571 | DSMZ |
| | Burkitt's Lymphoma | EB1 | 3.9797 | ATCC |
| | Burkitt's Lymphoma | SU-DHL-10 | 3.9798 | ATCC |
| | Burkitt's Lymphoma | BL-41 | 4.0698 | ATCC |
| | Burkitt's Lymphoma | EB2 | 4.2023 | ATCC |
| | Burkitt's Lymphoma | GA-10 | 4.2233 | ATCC |
| | Burkitt's Lymphoma | P3HR-1 | 4.2434 | ATCC |
| | Burkitt's Lymphoma | BL-70 | 4.4422 | DSMZ |
| | Diffuse Large B Cell Lymphoma | OCI-LY-19 | 3.6244 | ATCC |
| | Diffuse Large B Cell Lymphoma | DOHH-2 | 3.7235 | ATCC |
| | Diffuse Large B Cell Lymphoma | WSU-DLCL2 | 3.8199 | DSMZ |
| | Diffuse Large B Cell Lymphoma | SU-DHL-5 | 3.8819 | DSMZ |
| | Diffuse Large B Cell Lymphoma | DB | 3.9475 | DSMZ |
| | Diffuse Large B Cell Lymphoma | SU-DHL-8 | 3.9527 | DSMZ |
| | Diffuse Large B Cell Lymphoma | SU-DHL-6 | 3.9735 | DSMZ |
| | Diffuse Large B Cell Lymphoma | KARPAS-422 | 4.4105 | DSMZ |
| | Diffuse Large B Cell Lymphoma | SU-DHL-4 | 4.4221 | DSMZ |
| | Plasma Cell Myeloma | PCM6 | 3.7393 | ATCC |
| | Plasma Cell Myeloma | KHM-1B | 4.0262 | HSRRB |
| | Plasma Cell Myeloma | HuNS1 | 4.2789 | DSMZ |
| | Plasma Cell Myeloma | L-363 | 4.4213 | RIKEN |
| | Plasma Cell Myeloma | RPMI 8226 | 4.6537 | ATCC |
| | Intestinal Adenocarcinoma | COLO 205 | 4.2498 | ATCC |
| | Lung Mixed Adenosquamous Carcinoma | HCC-1195 | 4.3572 | KCLB |
| | Lung Small Cell Carcinoma | NCI-H2029 | 4.2394 | ATCC |
| | Lung Unspecified | BEN | 4.5409 | DSMZ |
| | Oesophagus Squamous Cell Carcinoma | TE-4 | 4.0083 | RIKEN |
| | Bone Unspecified | SK-N-MC | 4.1437 | ATCC |
| | Breast Ductal Carcinoma | UACC-893 | 3.8595 | ATCC |
| | Breast Ductal Carcinoma | HCC202 | 4.59 | ATCC |
| | Stomach Diffuse Adenocarcinoma | OCUM-1 | 4.5727 | HSRRB |
| | Stomach Ductal Carcinoma | HCC1500 | 4.4632 | ATCC |
| | Thyroid Medullary Carcinoma | TT | 4.0649 | ATCC |
| | Urinary Tract Transitional Cell Carcinoma | HT-1376 | 4.0436 | ATCC |

Figure 12

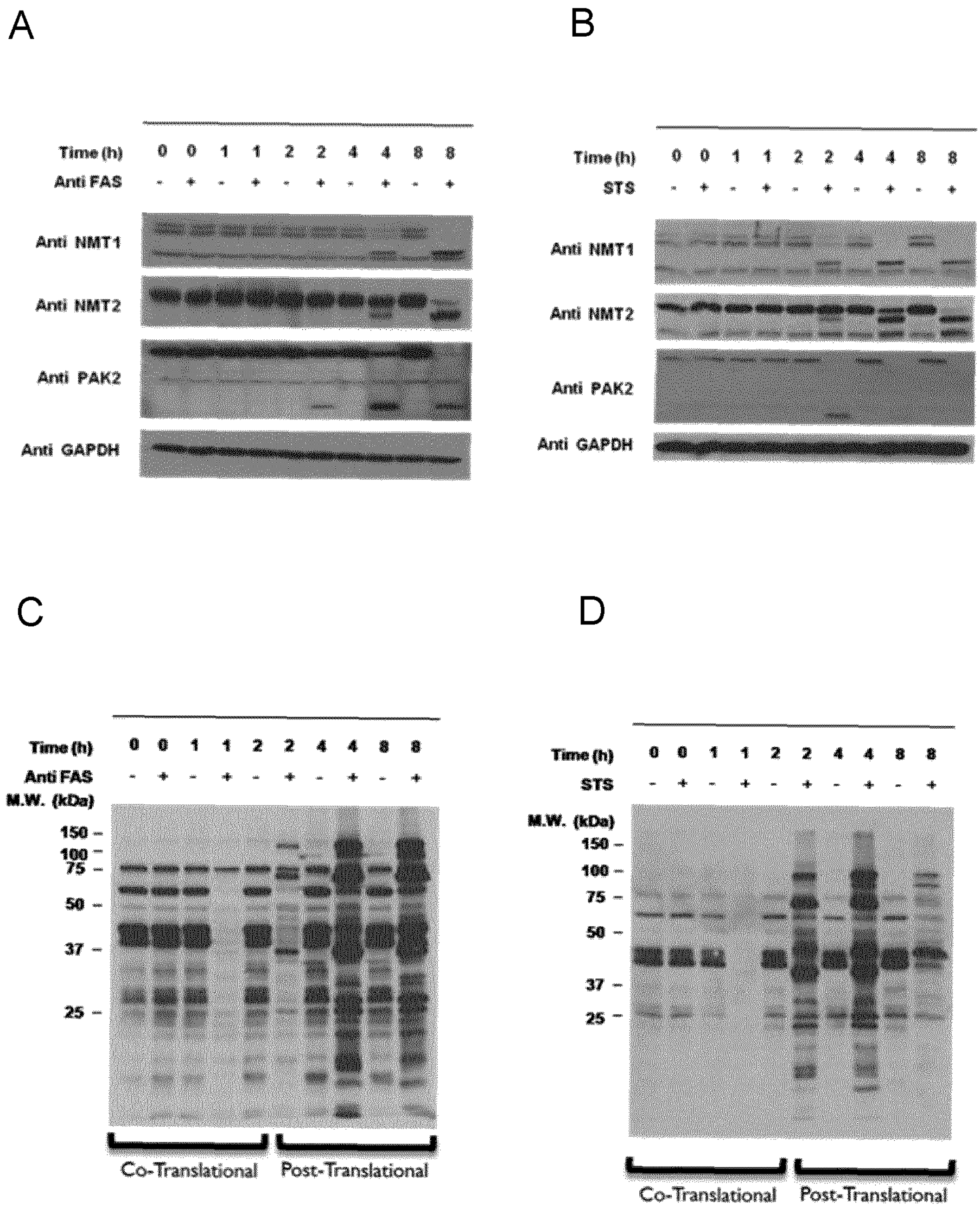
A
Time (h) 0 0 1 1 2 2 4 4 8 8
Anti FAS - + - + - + - + - +
Anti NMT1
Anti NMT2
Anti PAK2
Anti GAPDH
B
Time (h) 0 0 1 1 2 2 4 4 8 8
STS - + - + - + - + - +
Anti NMT1
Anti NMT2
Anti PAK2
Anti GAPDH
C
Time (h) 0 0 1 1 2 2 4 4 8 8
Anti FAS - + - + - + - + - +
M.W. (kDa)
150
100
75
50
37
25
Co-Translational
Post-Translational
D
Time (h) 0 0 1 1 2 2 4 4 8 8
STS - + - + - + - + - +
M.W. (kDa)
150
100
75
50
37
25
Co-Translational
Post-Translational

Figure 12

E

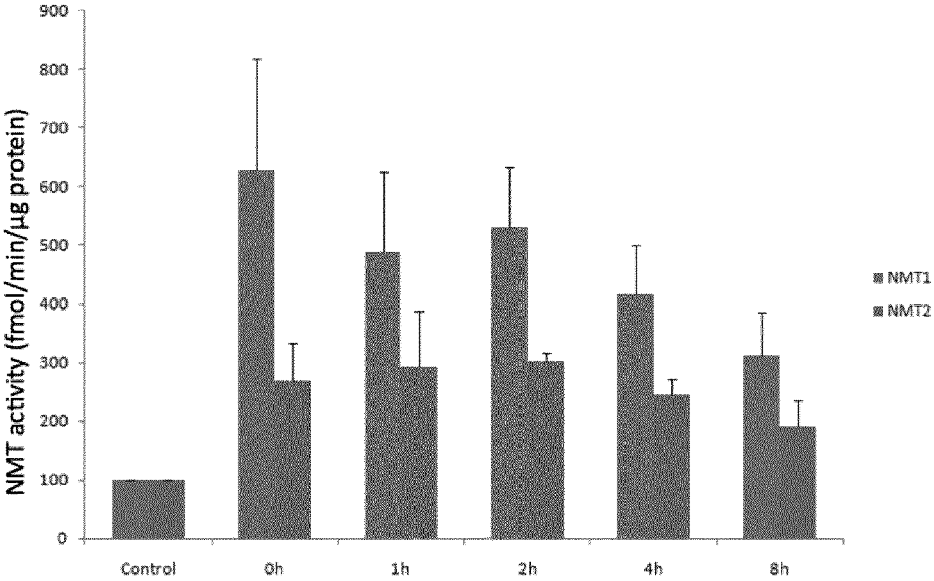
900
800
700
600
500
400
300
200
100
0
NMT activity (fmol/min/µg protein)
Control
0h
1h
2h
4h
8h
NMT1
NMT2

F

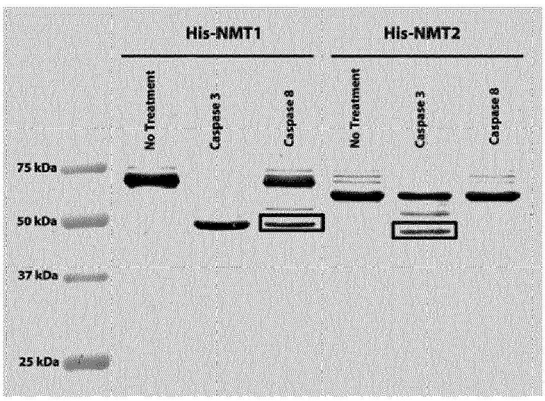
His-NMT1
His-NMT2
No Treatment
Caspase 3
Caspase 8
No Treatment
Caspase 3
Caspase 8
75 kDa
50 kDa
37 kDa
25 kDa

Figure 13

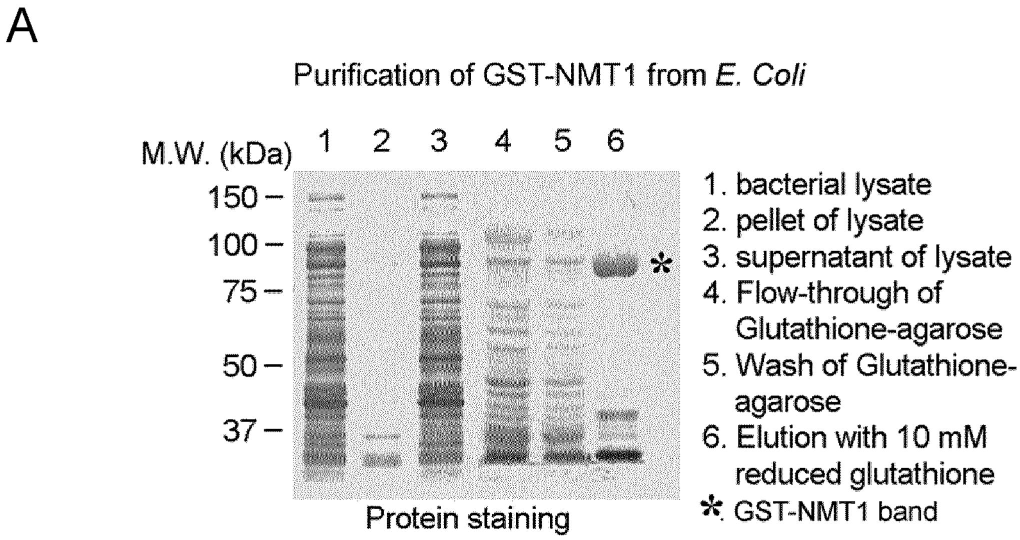
A
Purification of GST-NMT1 from *E. Coli*
M.W. (kDa)
1 2 3 4 5 6
150 –
100 –
75 –
50 –
37 –
*
Protein staining
1. bacterial lysate
2. pellet of lysate
3. supernatant of lysate
4. Flow-through of Glutathione-agarose
5. Wash of Glutathione-agarose
6. Elution with 10 mM reduced glutathione
*: GST-NMT1 band

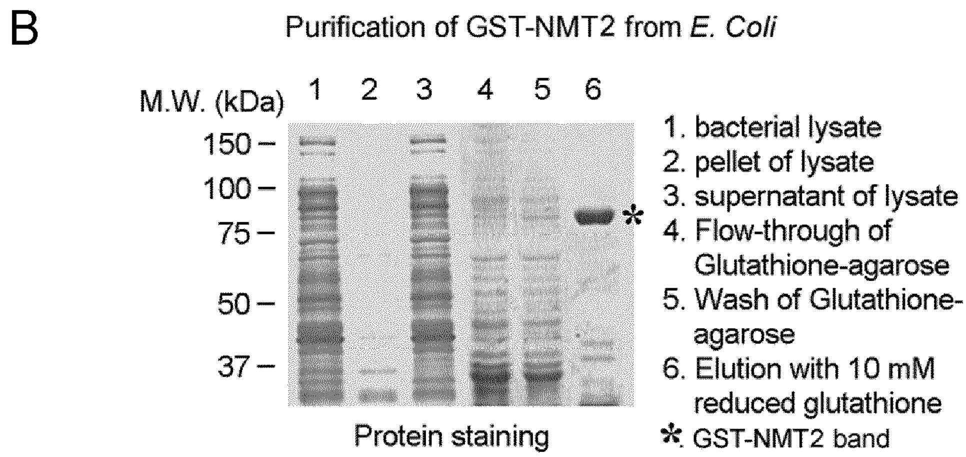
B
Purification of GST-NMT2 from *E. Coli*
M.W. (kDa)
1 2 3 4 5 6
150 –
100 –
75 –
50 –
37 –
*
Protein staining
1. bacterial lysate
2. pellet of lysate
3. supernatant of lysate
4. Flow-through of Glutathione-agarose
5. Wash of Glutathione-agarose
6. Elution with 10 mM reduced glutathione
*: GST-NMT2 band

Figure 13

C

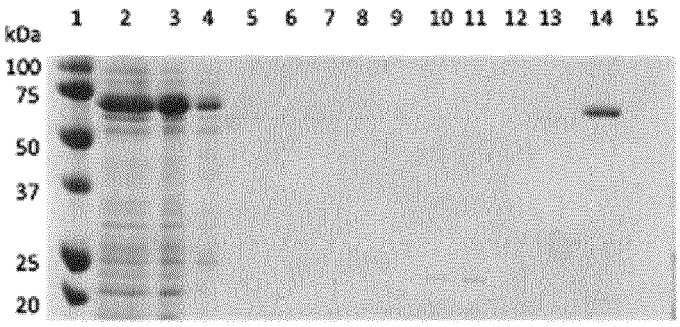


Gel A: Purification of NMT1
1. All Blue marker
2. Ni-NTA column pool
3. Gel Filtration pool
4. FT of Resource S
5. Fraction 8 (f8)
6. f16
7. f18
8. f19
9. f20
10. f21
11. f22
12. f41
13. f42
14. f66
15. f67

Figure 13

D

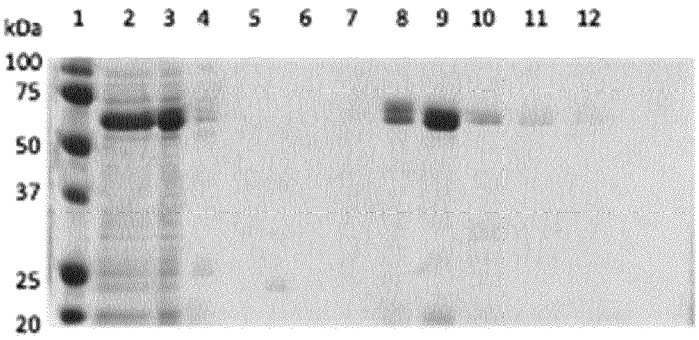


Gel A: Purification of NMT1
1. All Blue marker
2. Ni-NTA column pool
3. Gel Filtration pool
4. FT of Resource S
5. Fraction 21 (f21)
6. f28
7. f38
8. f39
9. f40
10. f41
11. ff42
12. f44

Figure 14

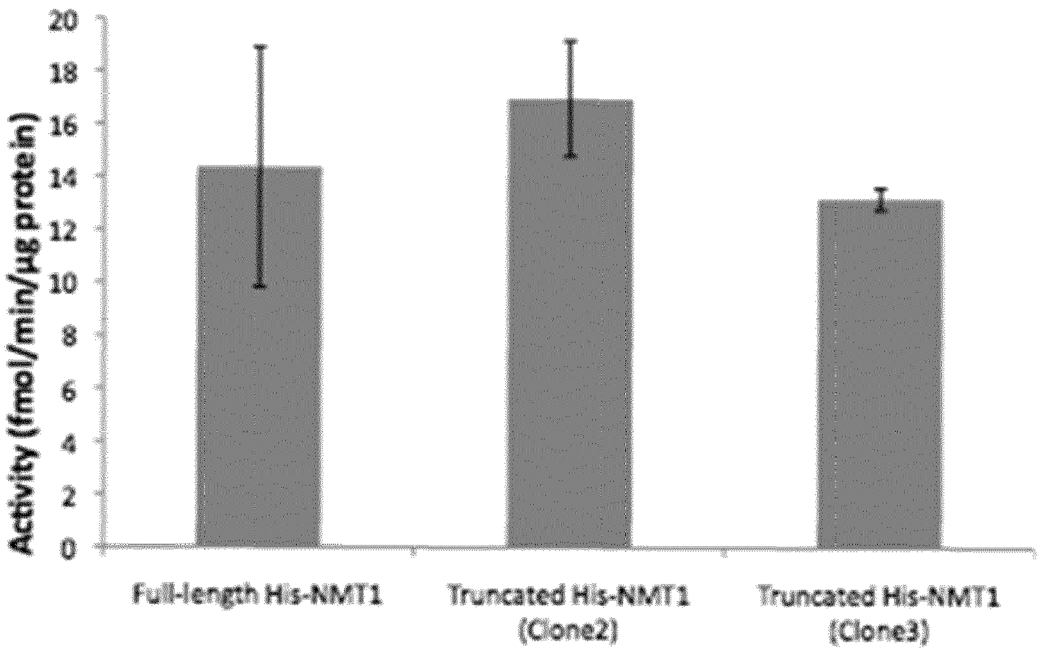
Activity (fmol/min/µg protein)
20
18
16
14
12
10
8
6
4
2
0
Full-length His-NMT1
Truncated His-NMT1 (Clone2)
Truncated His-NMT1 (Clone3)

Figure 15

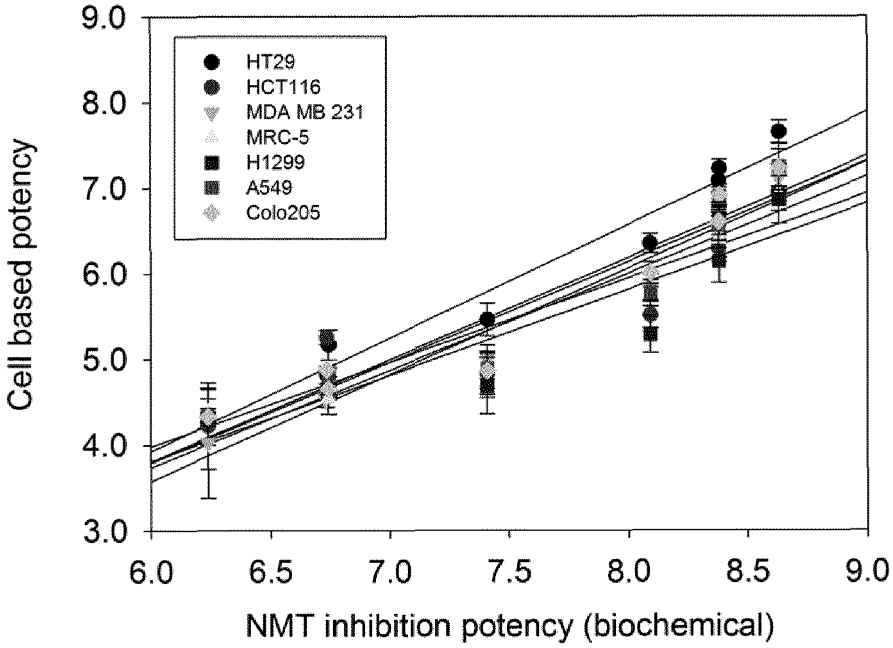
HT29
HCT116
MDA MB 231
MRC-5
H1299
A549
Colo205
9.0
8.0
7.0
6.0
5.0
4.0
3.0
Cell based potency
6.0
6.5
7.0
7.5
8.0
8.5
9.0
NMT inhibition potency (biochemical)

Figure 16

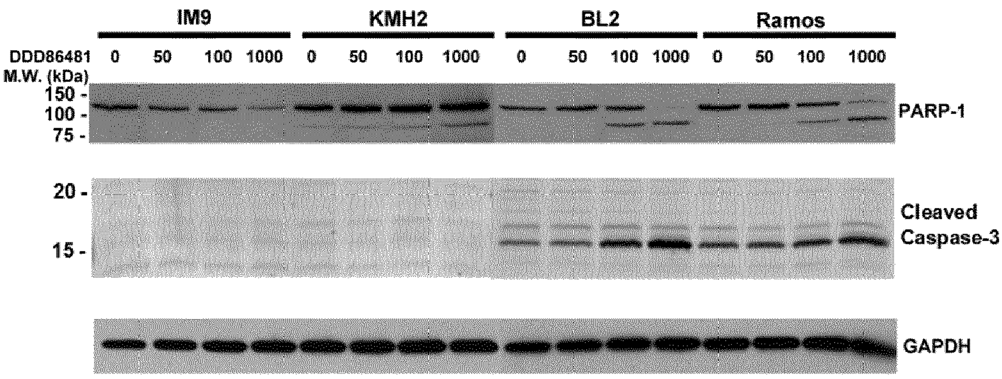
IM9
KMH2
BL2
Ramos
DDD86481
0
50
100
1000
M.W. (kDa)
150 -
100 -
75 -
PARP-1
20 -
15 -
Cleaved Caspase-3
GAPDH

Figure 17

A

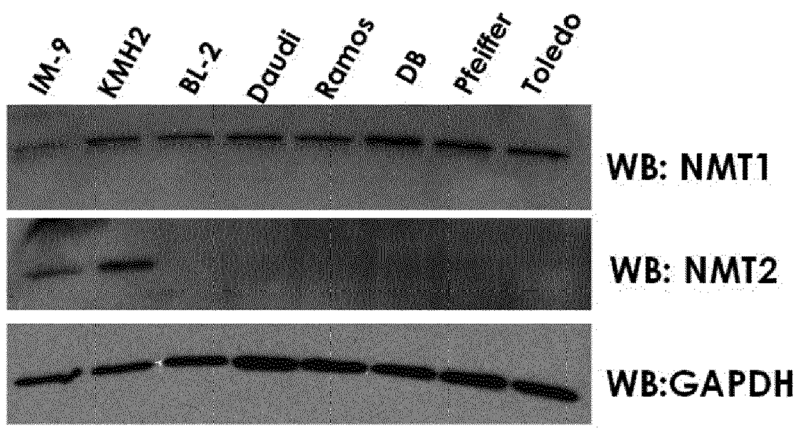
IM-9
KMH2
BL-2
Daudi
Ramos
DB
Pfeiffer
Toledo
WB: NMT1
WB: NMT2
WB:GAPDH

Figure 17

B

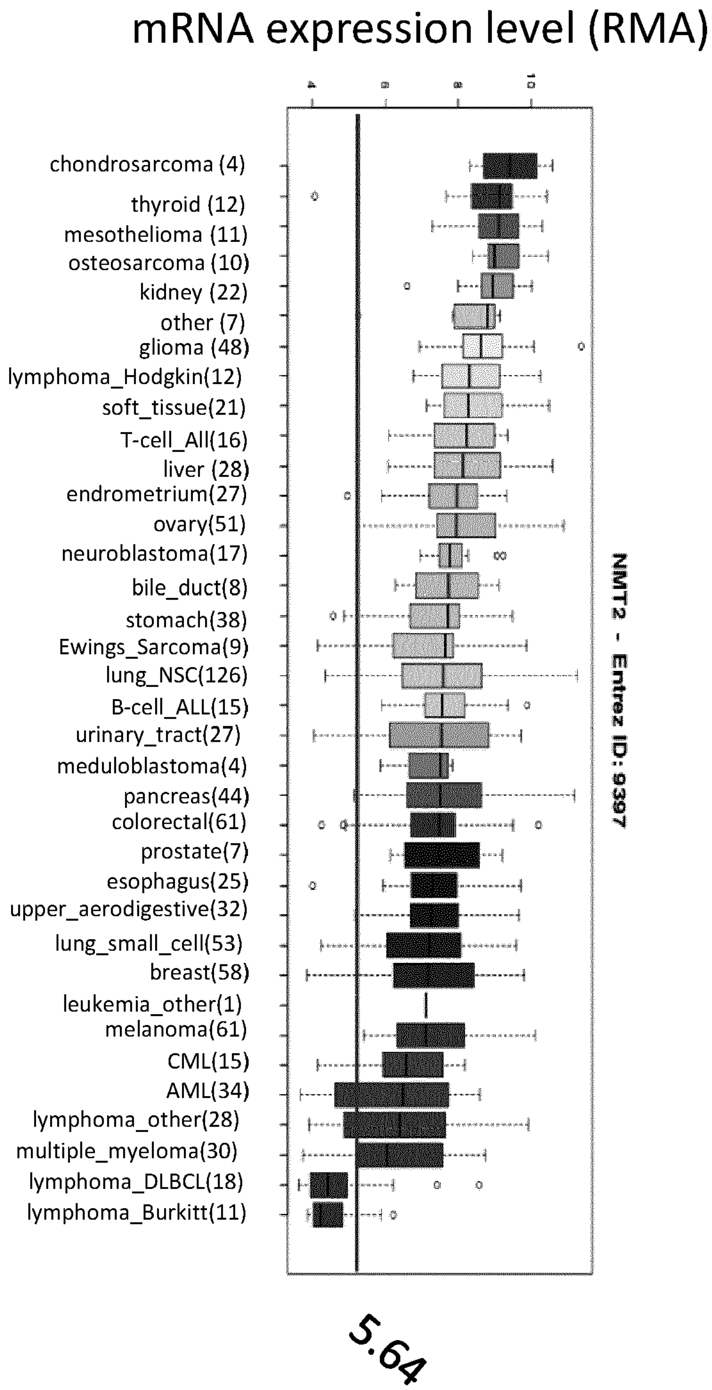
mRNA expression level (RMA)
4
6
8
10
chondrosarcoma (4)
thyroid (12)
mesothelioma (11)
osteosarcoma (10)
kidney (22)
other (7)
glioma (48)
lymphoma_Hodgkin(12)
soft_tissue(21)
T-cell_All(16)
liver (28)
endrometrium(27)
ovary(51)
neuroblastoma(17)
bile_duct(8)
stomach(38)
Ewings_Sarcoma(9)
lung_NSC(126)
B-cell_ALL(15)
urinary_tract(27)
meduloblastoma(4)
pancreas(44)
colorectal(61)
prostate(7)
esophagus(25)
upper_aerodigestive(32)
lung_small_cell(53)
breast(58)
leukemia_other(1)
melanoma(61)
CML(15)
AML(34)
lymphoma_other(28)
multiple_myeloma(30)
lymphoma_DLBCL(18)
lymphoma_Burkitt(11)
NMT2 - Entrez ID: 9397
5.64

# Figure 18

A I.

DesthioBiotin-PEG$_4$-N$_3$

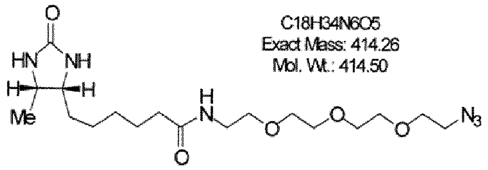

C18H34N6O5
Exact Mass: 414.26
Mol. Wt.: 414.50

II.

Biotin-PEG$_4$-N$_3$

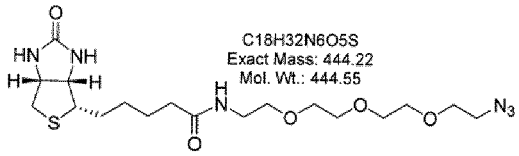

C18H32N6O5S
Exact Mass: 444.22
Mol. Wt.: 444.55

Affinity chromatography of desthiobiotinylated post-translationally myristoylated proteins from Jurkat T cells

B

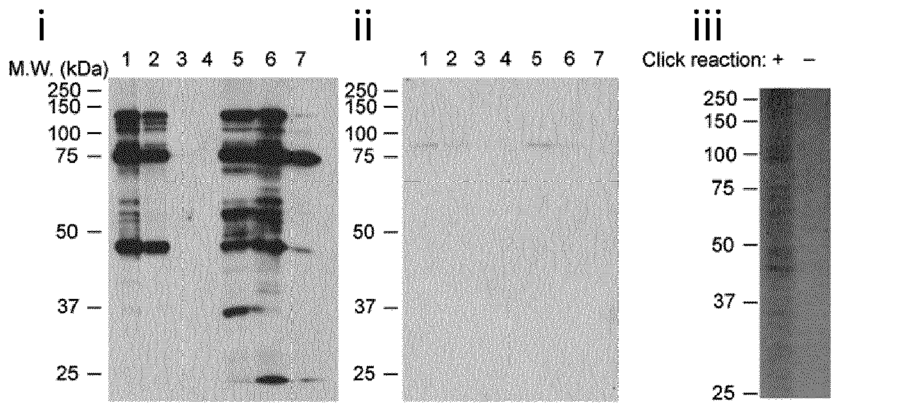


Panel A: cell lysates **with** "Click reaction" prior to chromatography

Panel B: cell lysates without "Click reaction" prior to chromatography

Preparative Coomassie Blue stained gel 400 µl of 2nd elution

Affinity chromatography: NeutrAvidin agarose resins

Panel A & B: Western blot: NeutrAvidin-HRP

| | |
|---|---|
| 1. Jurkat cell lysate | 10 µl of 5 ml |
| 2. Flow-through of NeutrAvidin-agarose | 10 µl of 5 ml |
| 3. 1st wash of NeutrAvidin-agarose | 10 µl of 5 ml |
| 4. 4th wash of NeutrAvidin-agarose | 10 µl of 5 ml |
| 5. 1st elution with 10 mM biotin | 30 µl of 500 µl |
| 6. 2nd elution with 10 mM biotin | 30 µl of 500 µl |
| 7. 3rd elution with 10 mM biotin | 30 µl of 500 µl |

Figure 19

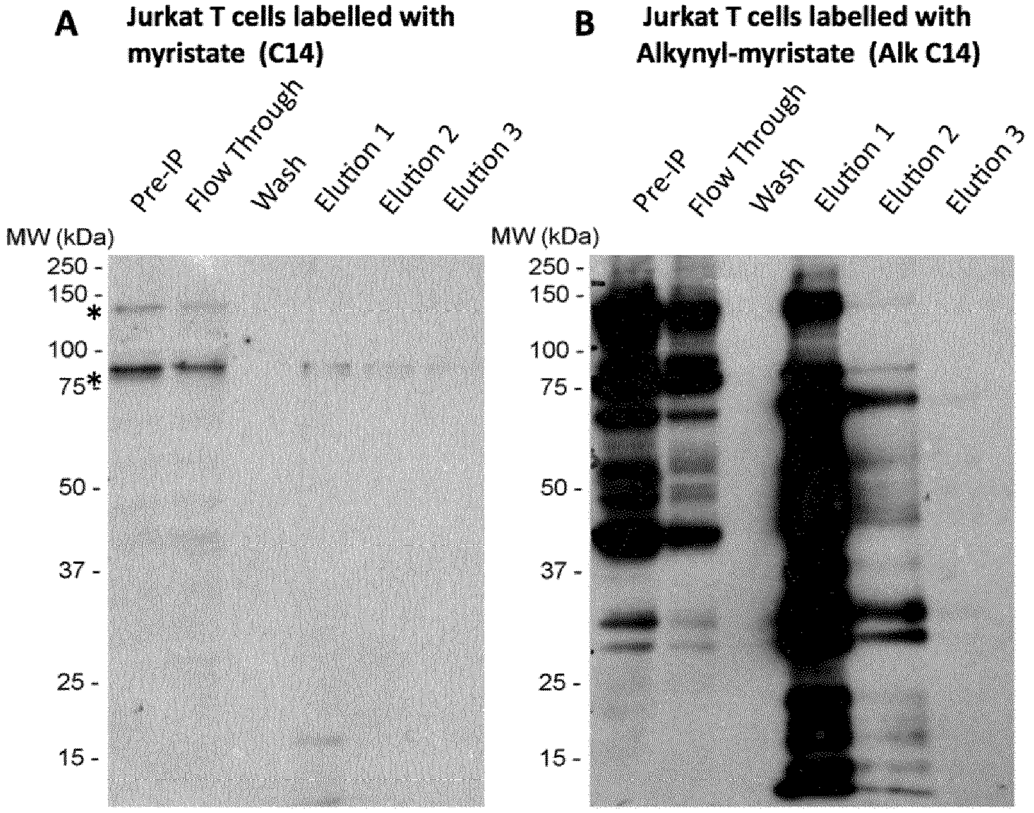


* Endogenously biotinylated proteins

Figure 20

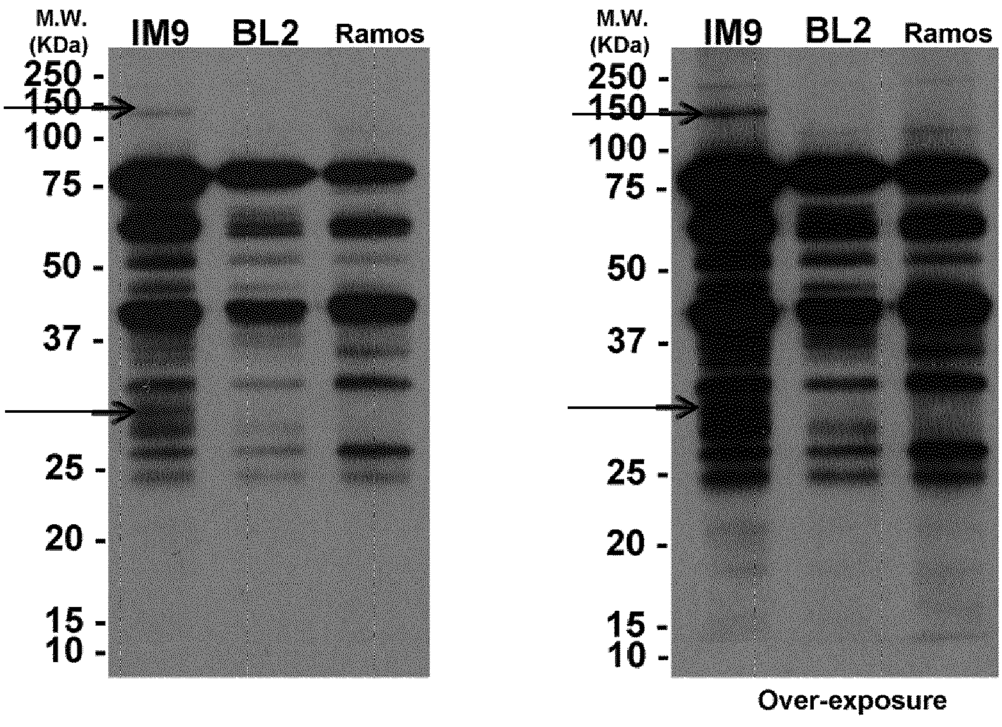


WB: Neutravidin-HRP

Figure 21

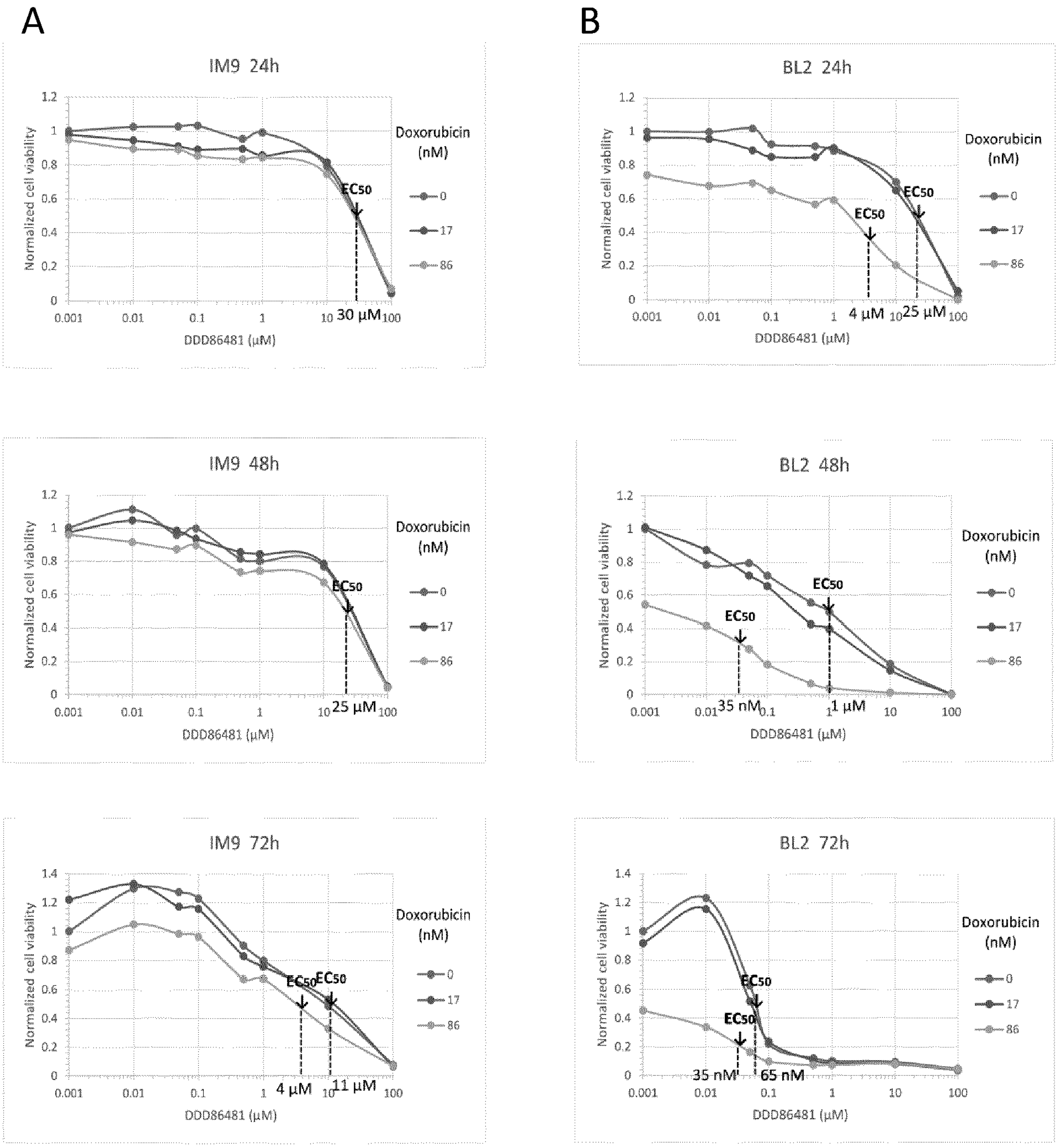
A
B
IM9 24h
IM9 48h
IM9 72h
BL2 24h
BL2 48h
BL2 72h
Normalized cell viability
DDD86481 (μM)
Doxorubicin (nM)
0
17
86
EC50
30 μM
25 μM
4 μM
11 μM
25 μM
35 nM
1 μM
35 nM
65 nM

# Figure 22

A

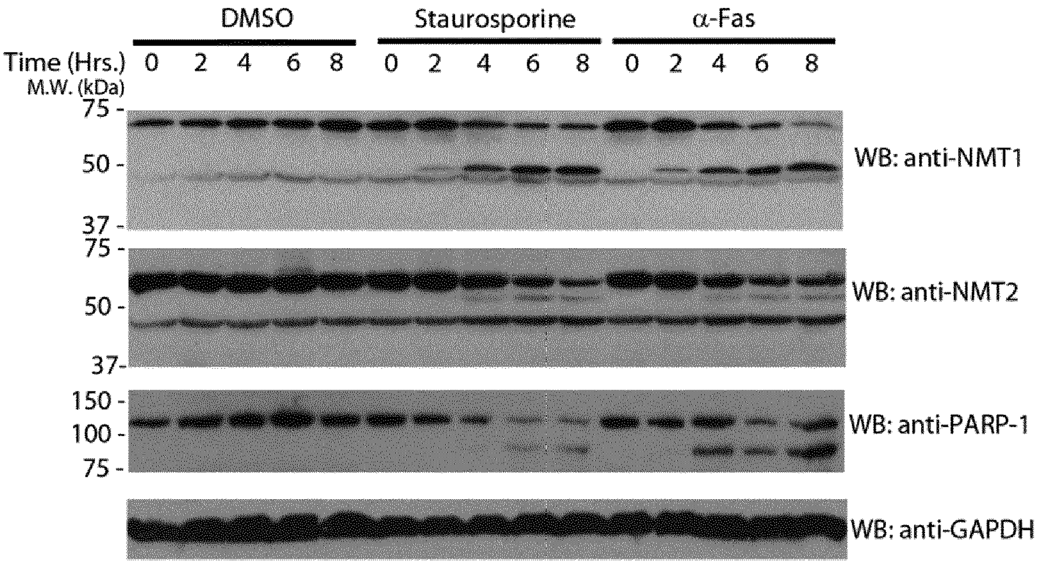
DMSO
Staurosporine
α-Fas
Time (Hrs.) 0 2 4 6 8 0 2 4 6 8 0 2 4 6 8
M.W. (kDa)
75 -
50 -
37 -
WB: anti-NMT1
75 -
50 -
37-
WB: anti-NMT2
150 -
100 -
75 -
WB: anti-PARP-1
WB: anti-GAPDH

B

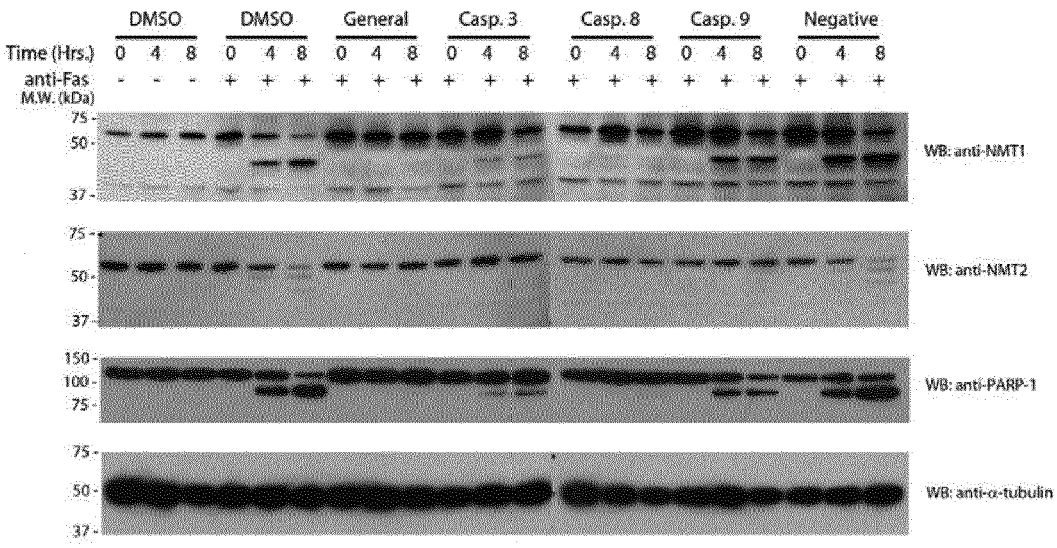
DMSO
DMSO
General
Casp. 3
Casp. 8
Casp. 9
Negative
Time (Hrs.) 0 4 8 0 4 8 0 4 8 0 4 8 0 4 8 0 4 8 0 4 8
anti-Fas - - - + + + + + + + + + + + + + + + + + +
M.W. (kDa)
75 -
50 -
37 -
WB: anti-NMT1
75 -
50 -
37 -
WB: anti-NMT2
150 -
100 -
75 -
WB: anti-PARP-1
75 -
50 -
37 -
WB: anti-α-tubulin

Figure 23

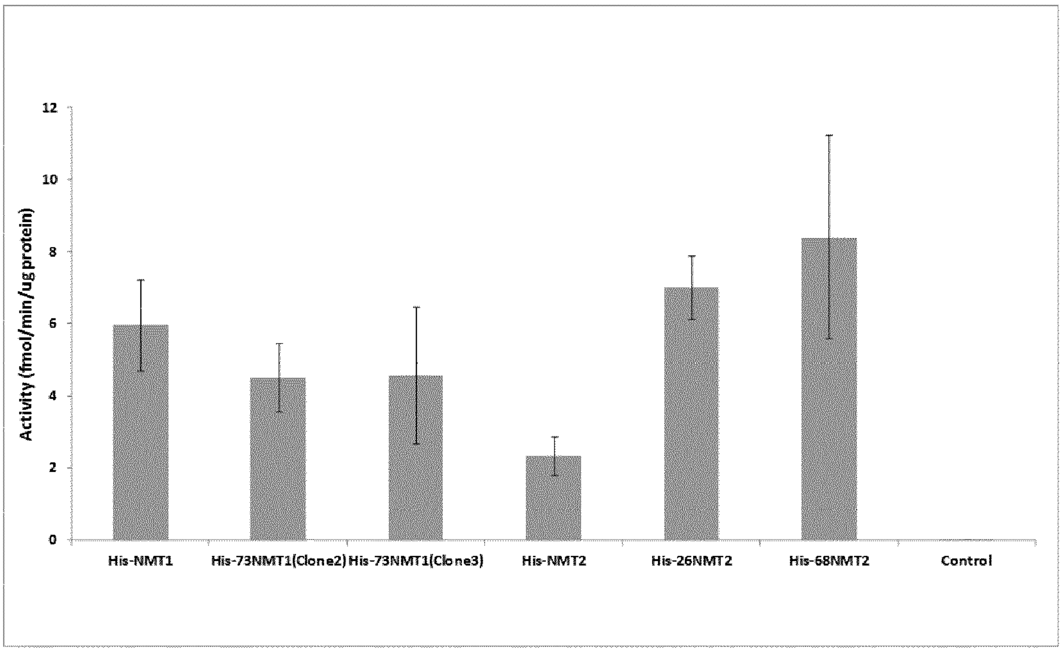
Activity (fmol/min/ug protein)
12
10
8
6
4
2
0
His-NMT1
His-73NMT1(Clone2)
His-73NMT1(Clone3)
His-NMT2
His-26NMT2
His-68NMT2
Control

Figure 24

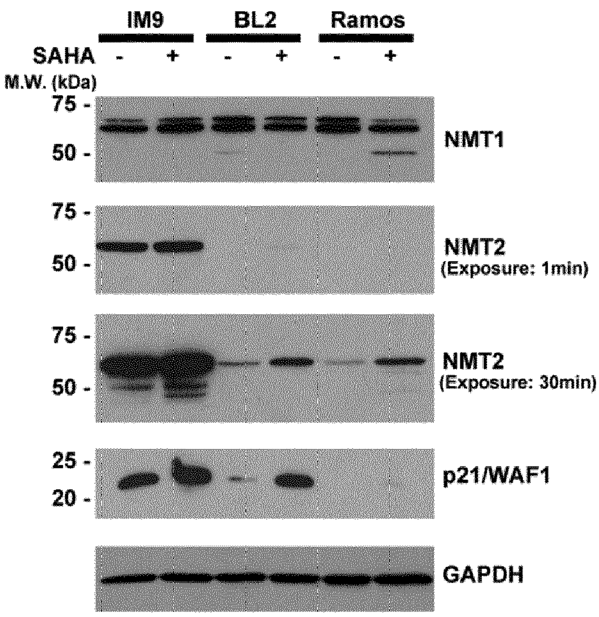
IM9
BL2
Ramos
SAHA
-
+
-
+
-
+
M.W. (kDa)
75 -
50 -
NMT1
75 -
50 -
NMT2
(Exposure: 1min)
75 -
50 -
NMT2
(Exposure: 30min)
25 -
20 -
p21/WAF1
GAPDH

Figure 25

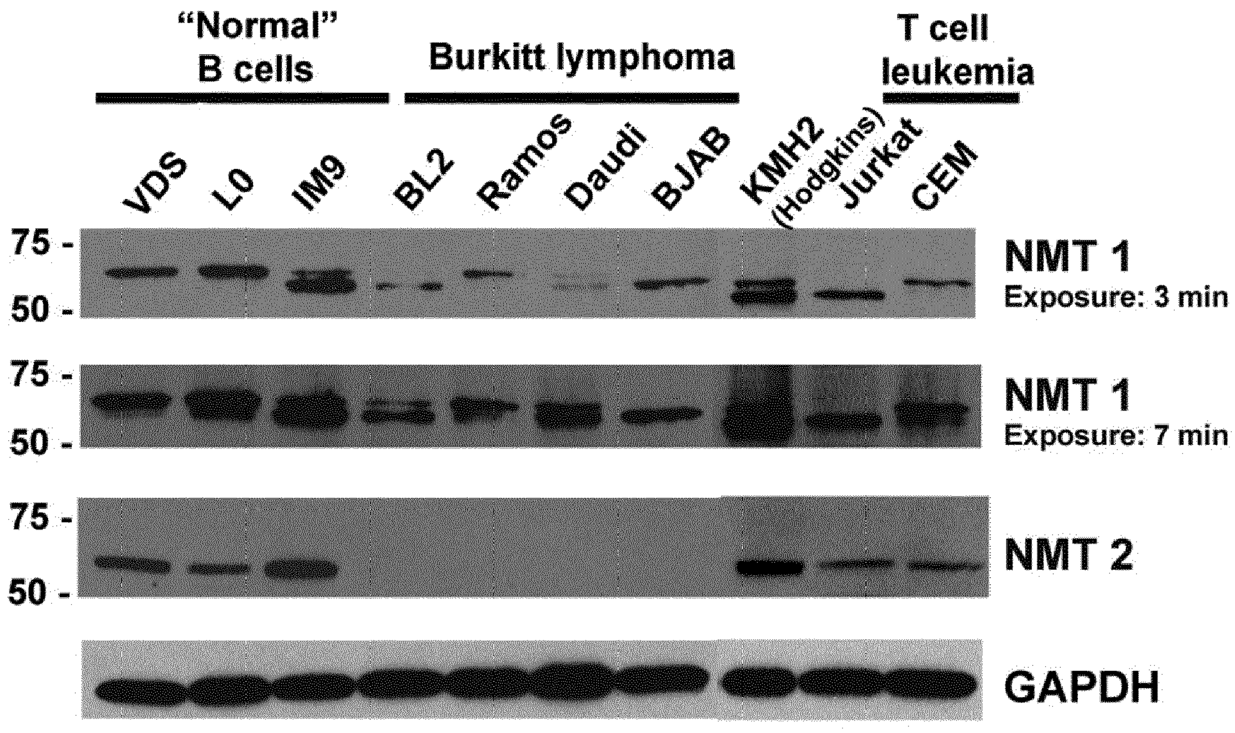
"Normal" B cells
Burkitt lymphoma
T cell leukemia
VDS
L0
IM9
BL2
Ramos
Daudi
BJAB
KMH2 (Hodgkins)
Jurkat
CEM
75 -
50 -
NMT 1
Exposure: 3 min
75 -
50 -
NMT 1
Exposure: 7 min
75 -
50 -
NMT 2
GAPDH

Figure 26

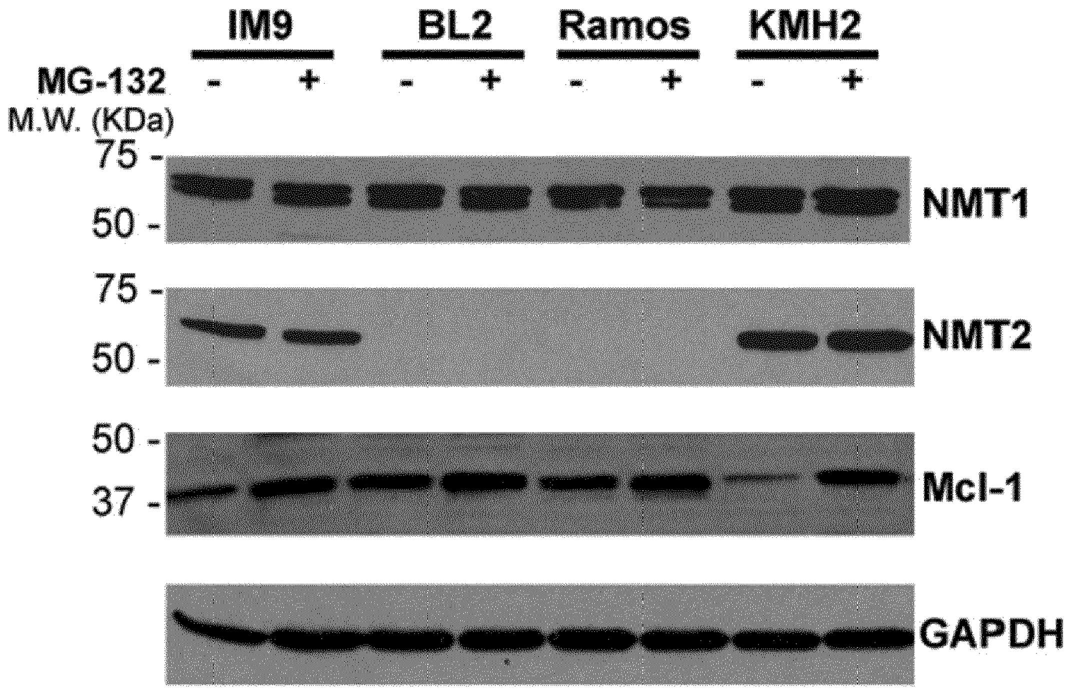
IM9
BL2
Ramos
KMH2
MG-132
- + - + - + - +
M.W. (KDa)
75 -
50 -
NMT1
75 -
50 -
NMT2
50 -
37 -
Mcl-1
GAPDH

Figure 27

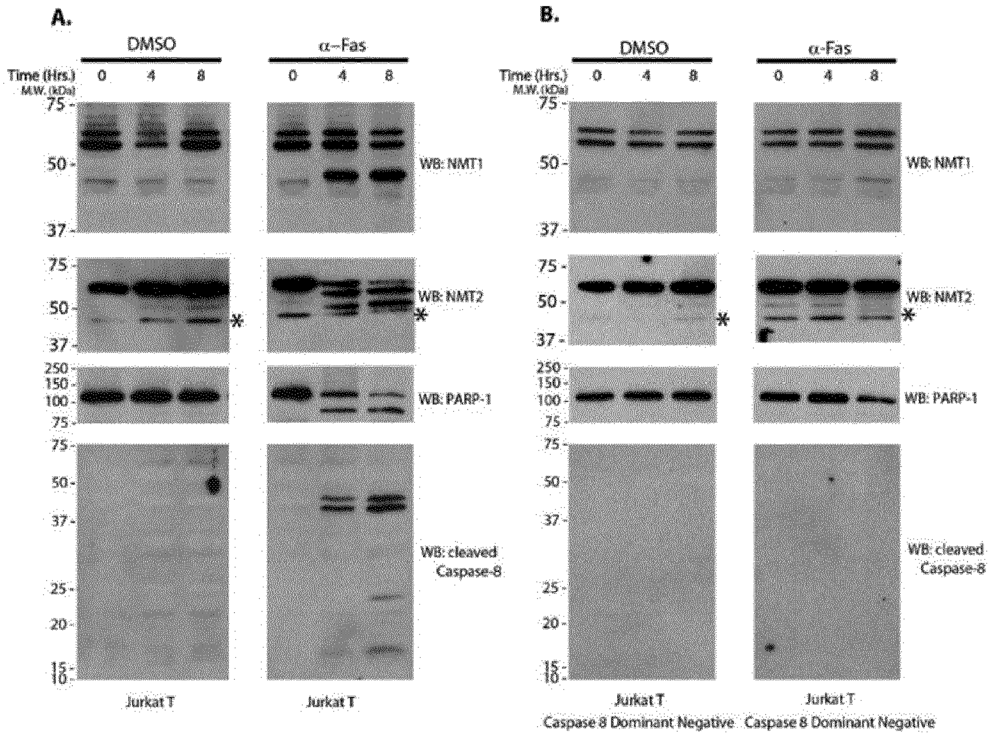
A.
DMSO
α–Fas
Time (Hrs.) 0 4 8 0 4 8
M.W. (kDa)
75
50
37
WB: NMT1
WB: NMT2
WB: PARP-1
250
150
100
WB: cleaved Caspase-8
25
20
15
10
Jurkat T
Jurkat T
B.
DMSO
α-Fas
Jurkat T
Caspase 8 Dominant Negative
Jurkat T
Caspase 8 Dominant Negative

Figure 28

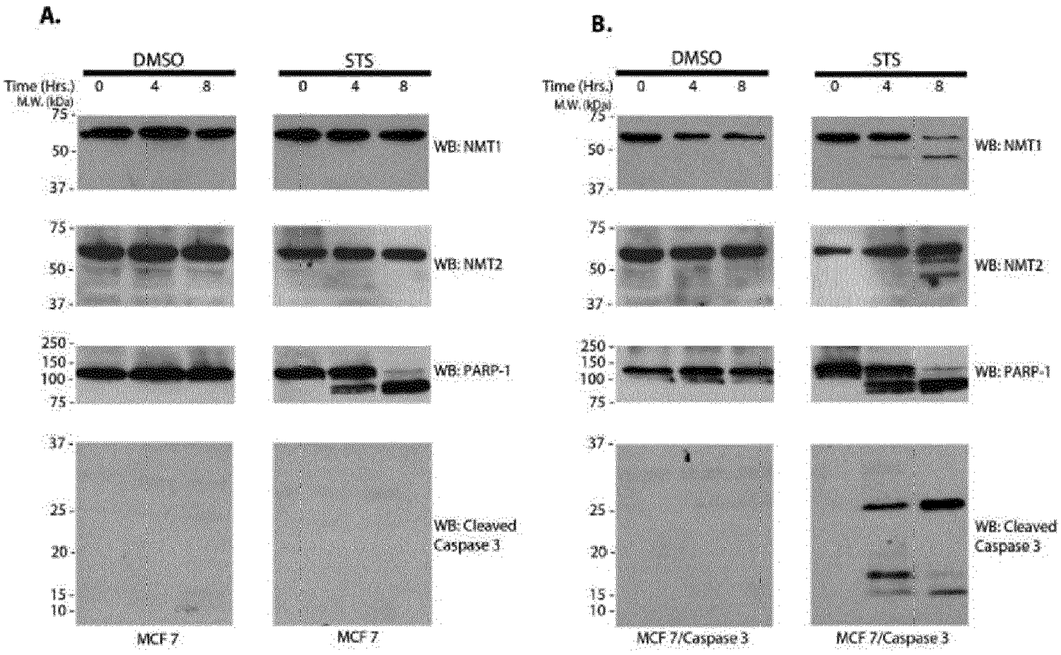
A.
DMSO
STS
Time (Hrs.)
0 4 8
0 4 8
M.W. (kDa)
75
50
37
WB: NMT1
75
50
37
WB: NMT2
250
150
100
75
WB: PARP-1
37
25
20
15
10
WB: Cleaved Caspase 3
MCF 7
MCF 7
B.
DMSO
STS
Time (Hrs.)
0 4 8
0 4 8
M.W. (kDa)
WB: NMT1
WB: NMT2
WB: PARP-1
WB: Cleaved Caspase 3
MCF 7/Caspase 3
MCF 7/Caspase 3

Figure 29

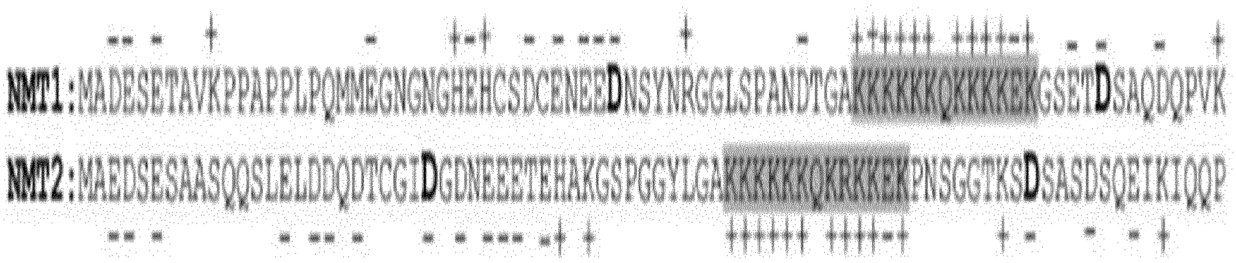
NMT1:MADESETAVKPPAPPLPQMMEGNGNGHEHCSDCENEEDNSYNRGGLSPANDTGAKKKKKKQKKKKEKGSETDSAQDQPVK
NMT2:MAEDSESAASQQSLELDDQDTCGIDGDNEEETEHAKGSPGGYLGAKKKKKKQKRKKEKPNSGGTKSDSASDSQEIKIQQP

Figure 30

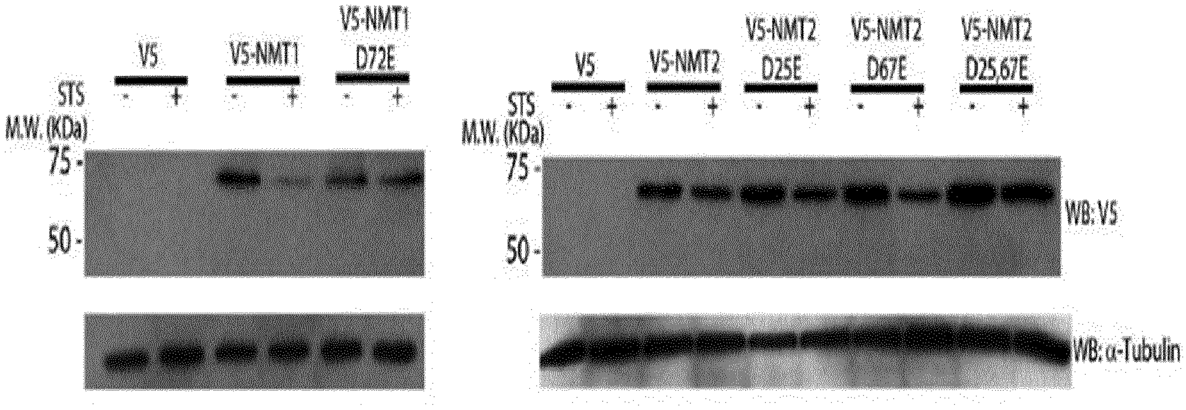
V5
V5-NMT1
V5-NMT1
D72E
STS
- + - + - +
M.W. (KDa)
75 -
50 -
V5
V5-NMT2
V5-NMT2
D25E
V5-NMT2
D67E
V5-NMT2
D25,67E
STS
- + - + - + - + - +
M.W. (KDa)
75 -
50 -
WB: V5
WB: α-Tubulin

Figure 31

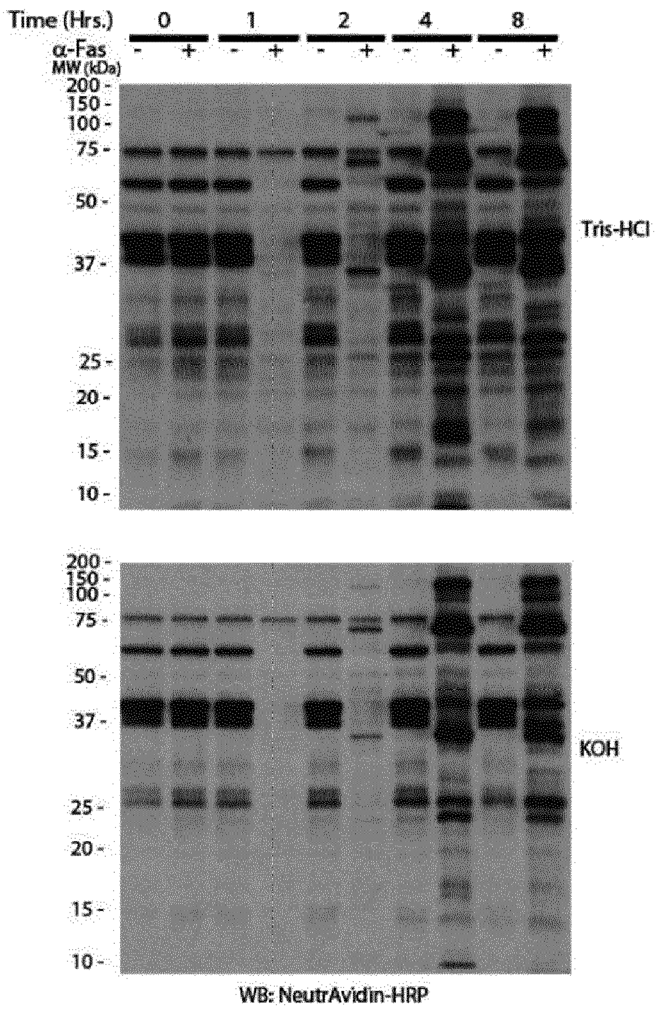
Time (Hrs.)
0
1
2
4
8
α-Fas
- + - + - + - + - +
MW (kDa)
200 -
150 -
100 -
75 -
50 -
37 -
25 -
20 -
15 -
10 -
Tris-HCl
KOH
WB: NeutrAvidin-HRP

Figure 32

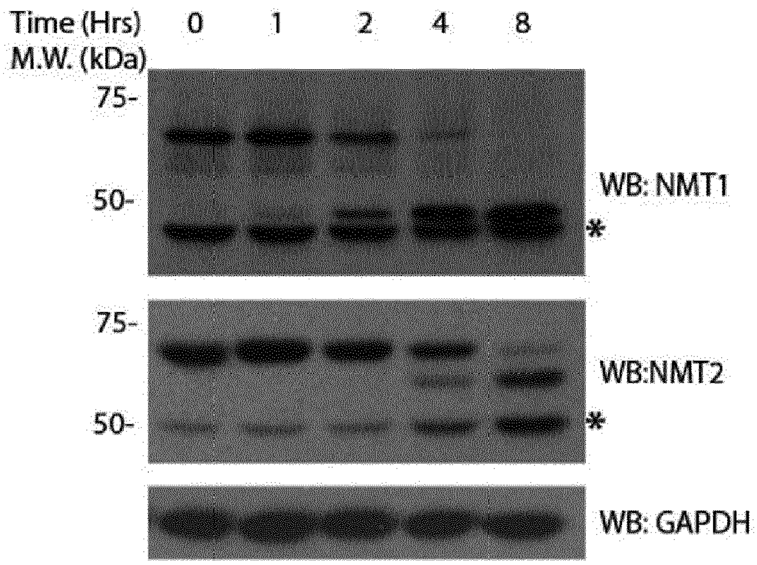

Figure 33

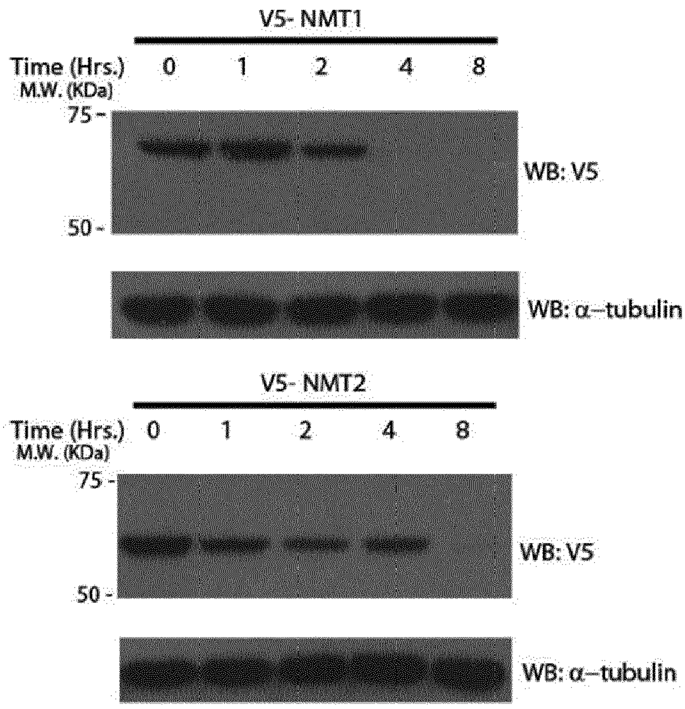
V5- NMT1
Time (Hrs.) 0 1 2 4 8
M.W. (KDa)
75 -
50 -
WB: V5
WB: α–tubulin
V5- NMT2
Time (Hrs.) 0 1 2 4 8
M.W. (KDa)
75 -
50 -
WB: V5
WB: α–tubulin

Figure 34

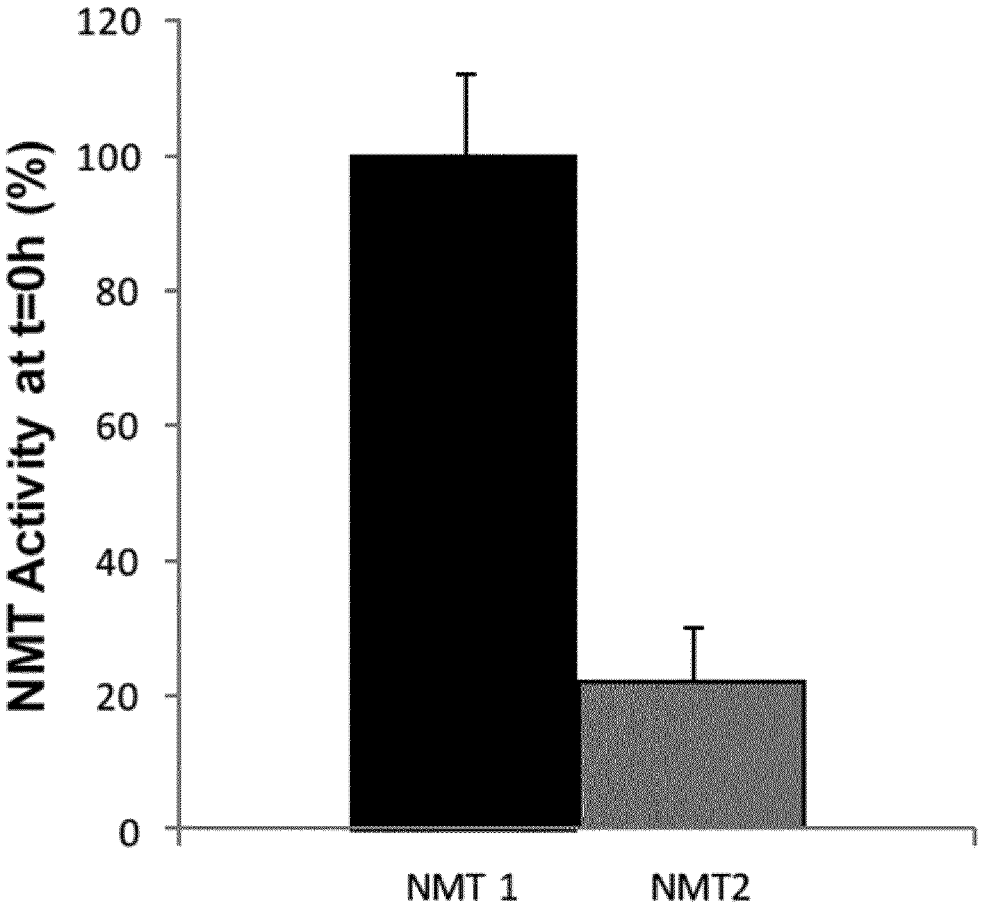

Figure 35

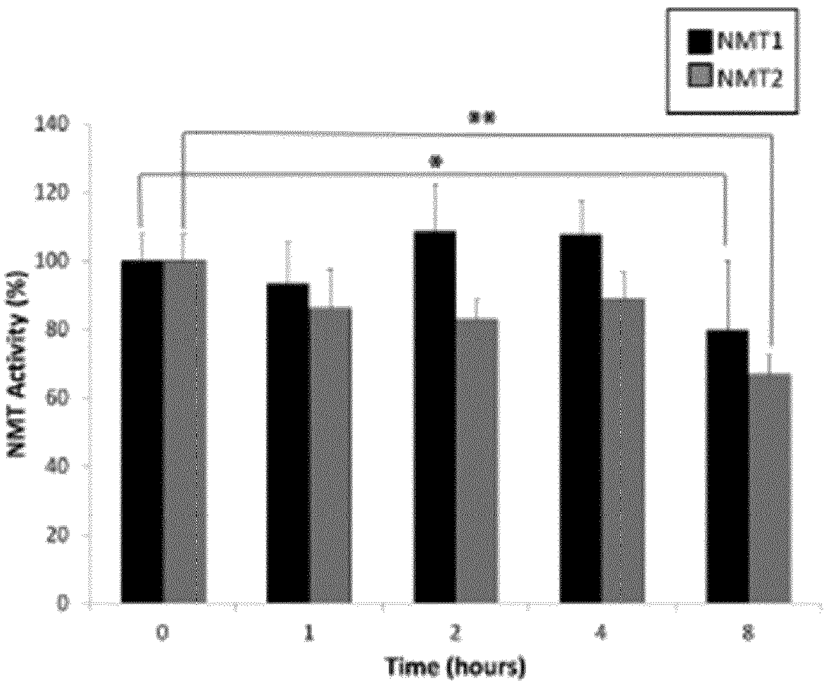
NMT1
NMT2
140
120
100
80
60
40
20
0
NMT Activity (%)
**
*
0
1
2
4
8
Time (hours)

Figure 36

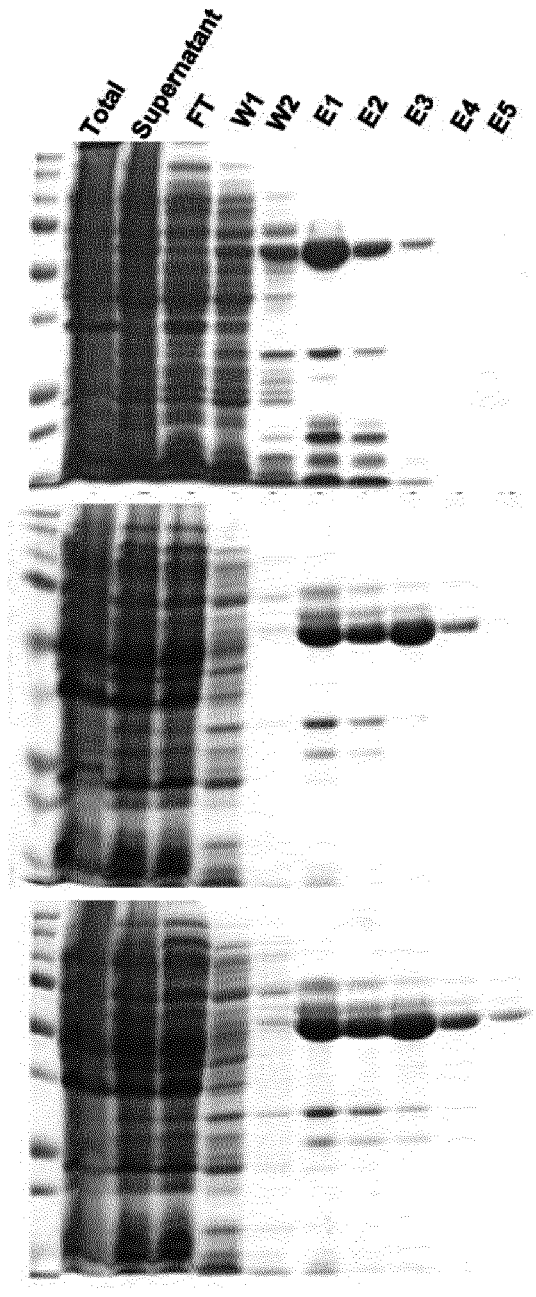

Figure 37

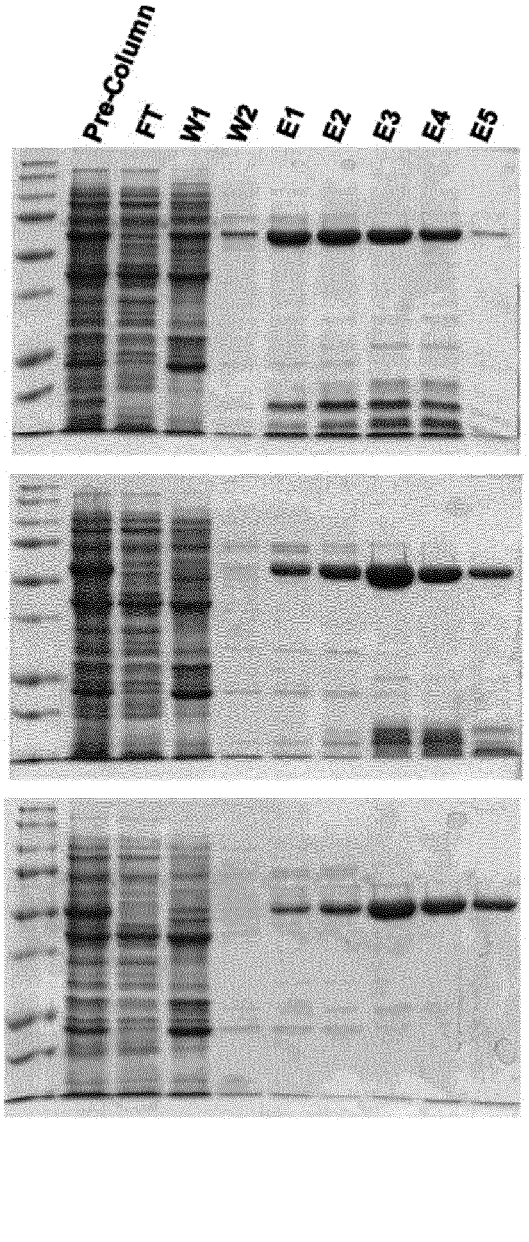

Figure 38

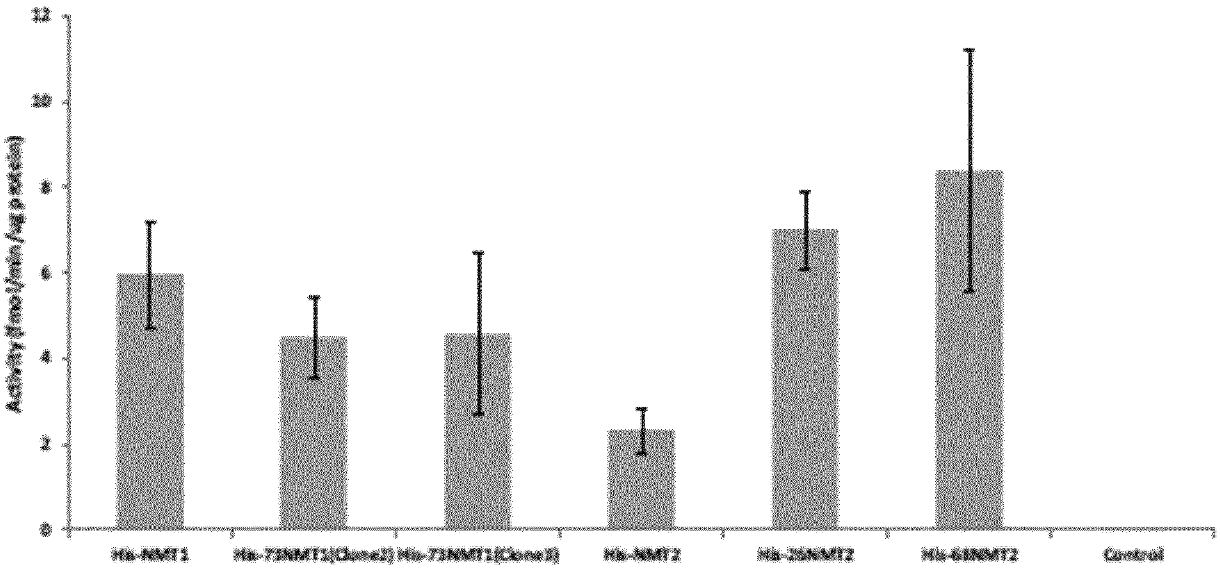
12
10
8
6
4
2
0
Activity (fmol/min/ug protein)
His-NMT1
His-73NMT1(Clone2)
His-73NMT1(Clone3)
His-NMT2
His-26NMT2
His-68NMT2
Control

Figure 39

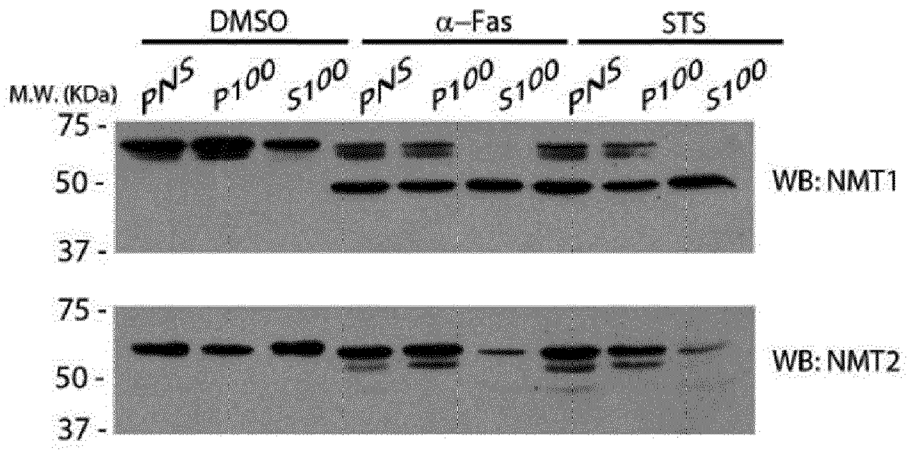
DMSO
α–Fas
STS
M.W. (KDa)
PNS
P100
S100
PNS
P100
S100
PNS
P100
S100
75 -
50 -
37 -
WB: NMT1
75 -
50 -
37 -
WB: NMT2

Figure 40

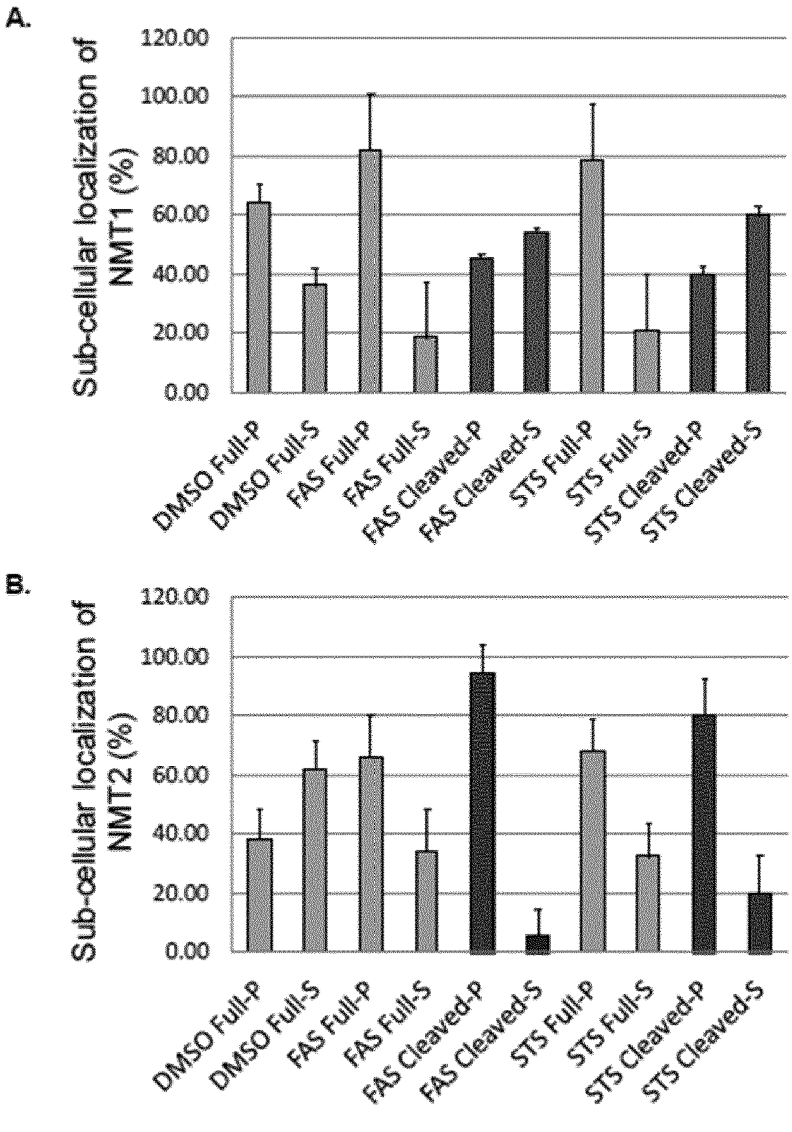
A.
Sub-cellular localization of NMT1 (%)
120.00
100.00
80.00
60.00
40.00
20.00
0.00
DMSO Full-P
DMSO Full-S
FAS Full-P
FAS Full-S
FAS Cleaved-P
FAS Cleaved-S
STS Full-P
STS Full-S
STS Cleaved-P
STS Cleaved-S
B.
Sub-cellular localization of NMT2 (%)
120.00
100.00
80.00
60.00
40.00
20.00
0.00
DMSO Full-P
DMSO Full-S
FAS Full-P
FAS Full-S
FAS Cleaved-P
FAS Cleaved-S
STS Full-P
STS Full-S
STS Cleaved-P
STS Cleaved-S

Figure 41

A.

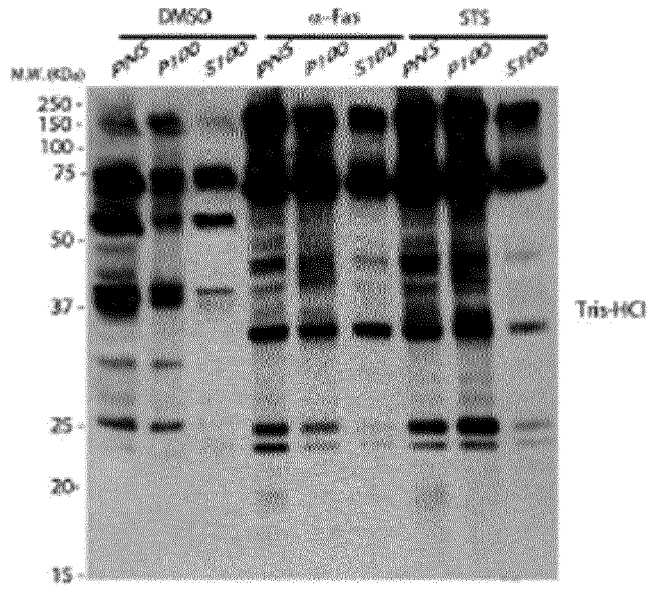
DMSO
α-Fas
STS
PNS
P100
S100
PNS
P100
S100
PNS
P100
S100
M.W.(KDa)
250
150
100
75
50
37
25
20
15
Tris-HCl

B.

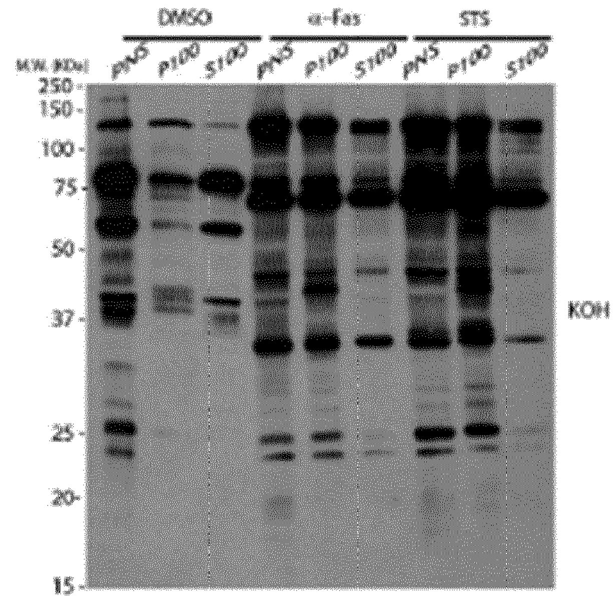
DMSO
α-Fas
STS
PNS
P100
S100
PNS
P100
S100
PNS
P100
S100
M.W.(KDa)
250
150
100
75
50
37
25
20
15
KOH

Figure 42

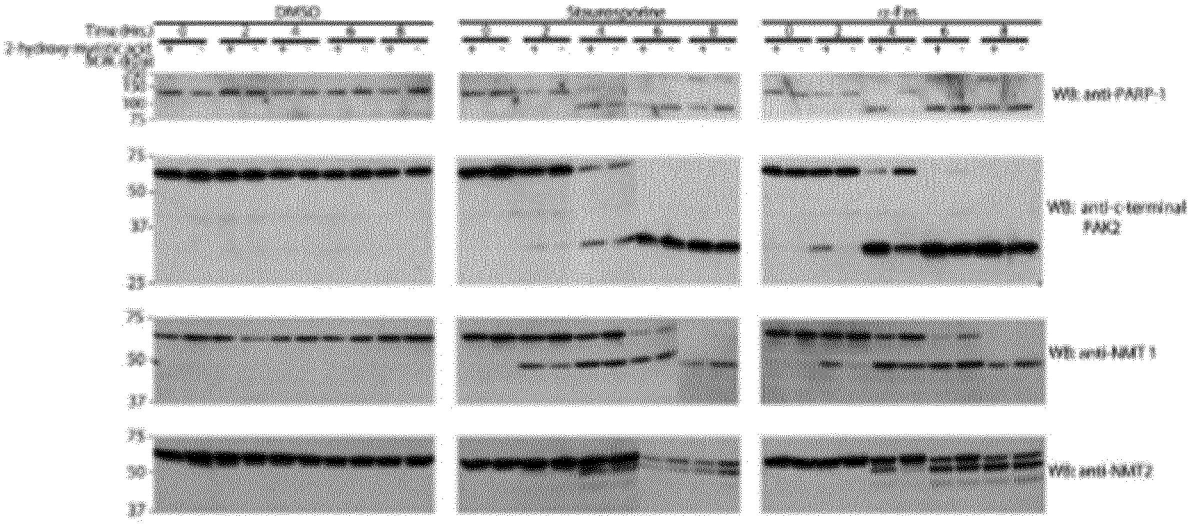
DMSO
Staurosporine
α-Fas
WB: anti-PARP-1
WB: anti-c-terminal PAK2
WB: anti-NMT1
WB: anti-NMT2

Europäisches Patentamt
European Patent Office
Office européen des brevets

# EUROPEAN SEARCH REPORT

Application Number
EP 20 21 3908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P<br><br><br>Y,P | WO 2013/013302 A1 (PACYLEX PHARMACEUTICALS INC [CA]; BERTHIAUME LUC G [CA]; BEAUCHAMP ERW) 31 January 2013 (2013-01-31)<br>* paragraphs [0105], [0111] - [0113], [0152]; claims 1-6 * | 1-15<br><br><br>1-15 | INV.<br>A61K31/4439<br>A61K31/12<br>A61K31/20<br>A61K39/395<br>A61P35/00<br>C12Q1/68<br>G01N33/574<br>A61K31/28<br>A61K31/496<br>A61K31/7105<br>A61K31/713<br>C07K16/40 |
| A | WO 2008/076965 A1 (ARBISER JACK [US]) 26 June 2008 (2008-06-26)<br>* examples 5,6 * | 1-15 | |
| A | S. S. BHANDARKAR ET AL: "Tris (Dibenzylideneacetone) Dipalladium, a N-Myristoyltransferase-1 Inhibitor, Is Effective against Melanoma Growth In vitro and In vivo",<br>CLINICAL CANCER RESEARCH,<br>vol. 14, no. 18,<br>15 September 2008 (2008-09-15), pages 5743-5748, XP055142988,<br>ISSN: 1078-0432, DOI: 10.1158/1078-0432.CCR-08-0405<br>* abstract * | 1-15 | TECHNICAL FIELDS SEARCHED (IPC) |
| A | Neil Kay ET AL: "Paper: TRIS (DIBENZYLIDENEACETONE) Dipalladium a Small-Molecule Palladium Complex Is Effective in the Induction of Apoptosis for B-Chronic Lymphocytic Leukemia B-Cells",<br>,<br>11 December 2011 (2011-12-11), XP055250861,<br>Retrieved from the Internet:<br>URL:https://ash.confex.com/ash/2011/webprogram/Paper37660.html<br>[retrieved on 2016-02-17]<br>* abstract * | 1-15 | A61K<br>G01N |

-/--

The present search report has been drawn up for all claims

1

| Place of search | Date of completion of the search | Examiner |
|---|---|---|
| The Hague | 1 June 2021 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 21 3908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/086043 A2 (STC UNM [US]; WILLIAM CHERYL L [US]; BEDRICK EDWARD [US]; KANG HUINING) 17 August 2006 (2006-08-17)<br>* abstract *<br>----- | 1-15 | |
| Y | WO 2010/026365 A1 (UNIV DUNDEE [GB]; BRAND STEPHEN [GB]; WYATT PAUL [GB]) 11 March 2010 (2010-03-11)<br>* claims 49-54 *<br>* page 26, paragraph 5 *<br>* page 39, paragraph 5 *<br>* page 160; table 2 *<br>----- | 1-15 | |
| A | SELVAKUMAR ET AL: "Potential role of N-myristoyltransferase in cancer", PROGRESS IN LIPID RESEARCH, PERGAMON PRESS, PARIS, FR, vol. 46, no. 1, 22 November 2006 (2006-11-22), pages 1-36, XP005776871, ISSN: 0163-7827, DOI: 10.1016/J.PLIPRES.2006.05.002<br>* page 21, paragraph 4 - page 27, paragraph 2 *<br>----- | 1-15 | TECHNICAL FIELDS SEARCHED (IPC) |

The present search report has been drawn up for all claims

| Place of search | Date of completion of the search | Examiner |
|---|---|---|
| The Hague | 1 June 2021 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 20 21 3908

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2021

| Patent document cited in search report | Publication date | Patent family member(s) | Publication date |
|---|---|---|---|
| WO 2013013302 A1 | 31-01-2013 | AU 2012286542 A1 | 06-02-2014 |
| | | AU 2018202839 A1 | 10-05-2018 |
| | | BR 112014001430 A2 | 21-02-2017 |
| | | CA 2842443 A1 | 31-01-2013 |
| | | CN 103826623 A | 28-05-2014 |
| | | CN 110115768 A | 13-08-2019 |
| | | EP 2734199 A1 | 28-05-2014 |
| | | JP 6270719 B2 | 31-01-2018 |
| | | JP 2014523440 A | 11-09-2014 |
| | | KR 20140072026 A | 12-06-2014 |
| | | KR 20200093065 A | 04-08-2020 |
| | | MX 368261 B | 25-09-2019 |
| | | RU 2014101787 A | 27-08-2015 |
| | | SG 10201605814Q A | 29-09-2016 |
| | | US 2014227284 A1 | 14-08-2014 |
| | | US 2019000838 A1 | 03-01-2019 |
| | | WO 2013013302 A1 | 31-01-2013 |
| WO 2008076965 A1 | 26-06-2008 | CA 2670837 A1 | 26-06-2008 |
| | | CN 101631466 A | 20-01-2010 |
| | | EP 2099305 A1 | 16-09-2009 |
| | | HK 1137622 A1 | 06-08-2010 |
| | | IL 198942 A | 31-08-2015 |
| | | US 2010076076 A1 | 25-03-2010 |
| | | WO 2008076965 A1 | 26-06-2008 |
| WO 2006086043 A2 | 17-08-2006 | US 2006141504 A1 | 29-06-2006 |
| | | WO 2006086043 A2 | 17-08-2006 |
| WO 2010026365 A1 | 11-03-2010 | EP 2323987 A1 | 25-05-2011 |
| | | EP 2980076 A1 | 03-02-2016 |
| | | ES 2546865 T3 | 29-09-2015 |
| | | ES 2631227 T3 | 29-08-2017 |
| | | US 2011312921 A1 | 22-12-2011 |
| | | US 2016060224 A1 | 03-03-2016 |
| | | WO 2010026365 A1 | 11-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61720218 B
- US 61870418 B
- US 61870435 B


### Non-patent literature cited in the description


- **J.A.FREARSON et al.** *Nature,* 2010, vol. 464, 728-723
- **RAJU, R. V. ; SHARMA, R. K.** Preparation and assay of myristoyl-CoA:protein N-myristoyltransferase. *Methods Mol Biol,* 1999, vol. 116, 193-211
- **TOWLER, D. ; GLASER, L.** Protein fatty acid acylation: enzymatic synthesis of an N-myristoylglycyl peptide. *Proc Natl Acad Sci U S A,* 1986, vol. 83, 2812-2816
- **KING et al.** *Anal Biochem.,* 1991
- **KING et al.** *Anal Biochem. Experiments in duplicate,* 1999
- **BARNEDA-ZAHONERO, B. ; M. PARRA.** Histone deacetylases and cancer. *Mol Oncol.,* 2012, vol. 6, 579-589